# FASCICULE DE BREVET EUROPEEN

(11) **EP 0 968 168 B1**
(45) Date de publication et mention de la délivrance du brevet: **29.08.2001**
(21) Numéro de dépôt: 97950241.6
(22) Date de dépôt: 05.12.1997
(51) Int. Cl.: C07C 63/66, A61K 31/19, A61K 31/34, A61K 31/41, A61K 31/66, A61K 7/00, C07C 63/74, C07C 233/65, C07D 307/79, C07D 257/04, C07F 9/38

(54) **COMPOSES TETRACYCLIQUES AROMATIQUES DE TYPE RETINOIDE, LEUR PROCEDE DE PREPARATION ET LEUR UTILISATION**
TETRACYCLISCHE AROMATISCHE RETINOIDE VERBINDUNGEN, IHRE HERSTELLUNG UND VERWENDUNG
AROMATIC TETRACYCLIC COMPOUNDS OF THE RETINOID TYPE, METHOD FOR PREPARING AND USE

(30) Priorité: 16.01.1997 FR 9700421
(43) Date de publication de la demande: 05.01.2000
(73) Titulaire: Centre Europeen de Bioprospective -CEB, 76131 Mont-Saint-Aignan Cédex (FR)
(72) Inventeur: LEBLOND, Bertrand, F-76000 Rouen (FR); DEYINE, Abdallah, F-76000 Rouen (FR); SCHOOFS, Alain-René, F-92400 Courbevoie (FR); GERMAIN, Pierre, F-34280 La Grande Motte (FR); POURRIAS, Bernard, F-02860 Bièvres (FR)
(74) Mandataire: Breese, Pierre
(86) Numéro de dépôt international: FR9702223
(87) Numéro de publication internationale: WO9831654

(56) Documents cités:
- US-A- 5 514 825
- M. I. DAWSON: "Effect of structural modification in the C7-C11 region of the retinoid skeleton on biological activity in a series of aromatic retinoids" JOURNAL OF MEDICINAL CHEMISTRY, vol. 32, no. 7, juillet 1989, WASHINGTON US, pages 1504-1517, XP000563865 cité dans la demande
- LING JONG: "Conformational effects on retinoid receptor selectivity. I. Effect of a 9-double bond geometry on retinoid X receptor activity" JOURNAL OF MEDICINAL CHEMISTRY, vol. 36, no. 18, 3 septembre 1993, WASHINGTON US, pages 2605-2613, XP000563864 cité dans la demande
- M. F. BOEHM: "Synthesis and structure-activity relationships of novel retinoid X receptor-selective retinoids" JOURNAL OF MEDICINAL CHEMISTRY, vol. 37, no. 18, 2 septembre 1994, WASHINGTON US, pages 2930-2941, XP000615432 cité dans la demande
- H. KAGECHIKA: "Retinobenzoic acids. Structure-activity relationships of aromatic amides with retinoidal activity" JOURNAL OF MEDICINAL CHEMISTRY, vol. 31, no. 11, novembre 1988, WASHINGTON US, pages 2182-2192, XP000608417 cité dans la demande

## Description

La présente invention concerne des composés tétracycliques aromatiques de type rétinoïdes de formule générale : 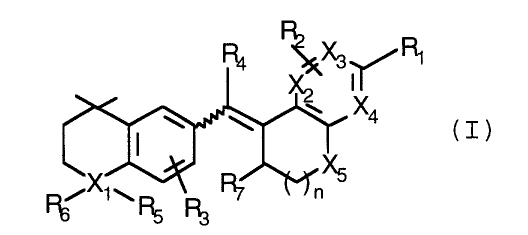 dans laquelle :
- R₁ est choisi parmi un atome d'hydrogène, un radical alkyle de 1 à 6 atomes de carbone linéaires ou ramifiés, un groupe de formule -PO₃H₂, -CH₂OH, -OH, -COR₈,-CH₂OCOR₉, -SH, -S-alkyl, -NH₂, NHCOOR₁₀, p-hydroxyphénylaminocarbonyl, tétrazol-5-yl-aminocarbonyl, tétrazol-5-yl, 5-trifluorométhyl-tétrazoyl et quand cela est possible leurs sels avec des acides tolérés physiologiquement, où R₁₀ est un groupe alkyle de 1 à 6 atomes de carbone linéaires ou ramifiés ou aralkyle, et R₈ et R9 sont choisis parmi :
   . un atome d'hydrogène, un groupe -OH, un radical alkyle de 1 à 6 atomes de carbone linéaires ou ramifiés ou un groupe de formule -OR₁₁, où R₁₁ représente un radical alkyle ramifié ou non ayant de 1 à 20 atomes de carbone, un radical alkényle ramifié ou non ayant de 2 à 20 atomes de carbone, un radical aryle ou aralkyle, ou
   . un groupe aminé de formule : 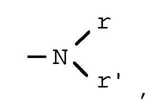 dans laquelle r et r', identiques ou différents, représentent un atome d'hydrogène, un radical alkyle de 1 à 6 atomes de carbone linéaires ou ramifiés, un radical aryle ou aralkyle, un reste d'α-aminoacide, un reste de sucre ou un hétérocycle dans lequel r et r' pris ensemble forment un hétérocycle.
- R₂ est choisi parmi un atome d'hydrogène, un atome d'halogène et plus particulièrement un atome de fluor, un radical alkyle de 1 à 6 atomes de carbone linéaires ou ramifiés, un groupe de formule -COOH, -OR₁₁, -SR₁₁,-(CF₂)ₙCF₃ où n est un nombre entier compris entre 0 et 10, ou un groupe -OCOR₁₂, et quand cela est possible leurs sels avec des acides tolérés physiologiquement, ou un groupe aminé de formule : 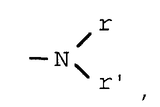 dans laquelle r et r' ont la même signification que précédemment, et R₁₂ représente un atome d'hydrogène, un radical alkyle de 1 à 6 atomes de carbone linéaires ou ramifiés, un radical fluoroalkyle ayant de 1 à 6 atomes de carbone et de 3 à 7 atomes de fluor, un radical aryle ou un radical aralkyle.
- R₃ est choisi parmi un atome d'hydrogène, un radical alkyle de 1 à 6 atomes de carbone linéaires ou ramifiés, un atome d'halogène, un radical fluoroalkyle ayant de 1 à 6 atomes de carbone et de 3 à 7 atomes de fluor, ou un groupe de formule -OR₁₃ où R₁₃ représente un atome d'hydrogène, un radical alkyle de 1 à 6 atomes de carbone linéaires ou ramifiés, un radical aryle ou un radical aralkyle ou un groupement trifluorométhyle.
- X₁ est choisi parmi un atome de carbone, un atome d'oxygène ou un atome de soufre, et alors,
- R₅ et R₆ sont :
   . des radicaux méthyles ou éthyles, dans le cas où X₁ est un atome de carbone,
   . rien dans le cas où X₁ est un atome de soufre ou un atome d'oxygène,
   . un ou deux atomes d'oxygène dans le cas où X₁ est un atome de soufre (cas d'un sulfoxyde -SO- ou d'une sulfone -SO₂-).
- R₄ est choisi parmi un atome d'hydrogène, un atome d'halogène et plus particulièrement un atome de fluor, un radical trifluorométhyle, un radical aryle, un radical aralkyle, ou un radical alkyle de 1 à 6 atomes de carbone linéaires ou ramifiés, éventuellement substitué par un hydroxyle, un ou plusieurs atomes de fluor, un alcoxy de 1 à 6 atomes de carbone linéaires ou ramifiés ou par un groupe de formule -(C=O)R₁₄ dans laquelle R₁₄ représente un atome d'hydrogène, un radical de 1 à 6 atomes de carbone linéaires ou ramifiés, un radical hydroxyle, un radical alcoxy de 1 à 6 atomes de carbone linéaires ou ramifiés ou un groupe aminé de formule: 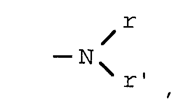 dans laquelle r et r' ont la même signification que précédemment.
- X₂ et X₃, identiques ou différents, représentent un atome de carbone ou un atome d'azote, ou encore X₂-X₃ sont un seul atome de soufre, d'oxygène, ou d'azote. Ainsi le noyau porteur de X₂ et X₃ peut être benzénique, pyridinique, thiophénique, furanique, pyrrolique.
- R7 est choisi parmi un atome d'hydrogène, un radical trifluorométhyle, un radical alkyle de 1 à 6 atomes de carbone linéaires ou ramifiés éventuellement substitué par un ou plusieurs atomes de fluor, un groupe -OR₁₅ où R₁₅ représente un atome d'hydrogène ou un radical alkyle de 1 à 6 atomes de carbone linéaires ou ramifiés
- X4 représente un atome de carbone ou d'azote.
- X₅ est choisi parmi un atome de carbone, d'oxygène, de soufre ou d'azote, un sulfure de formule -S-, un sulfoxyde de formule -SO-, une sulfone de formule -SO₂-, une amine de formule -NR16- où R₁₆ représente un atome d'hydrogène ou un radical alkyle de 1 à 6 atomes de carbone linéaires ou ramifiés, un groupe de formule -COR₁₇- ou-CO₂R₁₇- où R₁₇ est un radical alkyl de 1 à 6 atomes de carbone linéaires ou ramifiés ou benzyle.
- n est 0 ou 1.

A titre de sels pharmaceutiquement acceptables des composés précédents, on peut citer de manière non limitative : les sels d'acides acétique, chlorhydrique, cinnamique, citrique, formique, hydrobromique, hydroiodique, hydrofluorique, malonique, méthanesulfonique, oxalique, picrique, maléique, lactique, nicotinique, phénylacétique, phosphorique, succinique, sulfurique, tartrique, les sels d'ammonium, de pipérazine, de diéthylamine, de nicotinamide, d'urée, de sodium, de potassium, de calcium, de magnésium, de zinc, de lithium, de méthylamino, de diméthylamino, de triméthylamino, de tris(hydroxyméthyl)aminométhane.

Le terme alkyl ou alcoxy inférieur désigne des groupes de 1 à 6 atomes de carbone linéaires ou ramifiés tels que les radicaux méthyle, éthyle, propyle, isopropyle, butyle, isobutyle, butyle secondaire, méthoxy, éthoxy, propoxy, isopropoxy, butyloxy, isobutyloxy, butyloxy secondaire.

L'acide tout-*trans* rétinoïque, métabolite de la vitamine A présente un grand nombre de propriétés biologiques. Plusieurs molécules construites à partir de modifications chimiques de cet acide ont été synthétisées et se sont révélées biologiquement actives. Ces analogues synthétiques et leurs dérivés sont appelés rétinoïdes, selon la définition de Sporn, M. B. et Roberts, A. B., *Ciba*. *Found. Symp*., 113, 1-5, 1985. Parmi ces composés, on peut citer ceux décrits dans les demandes de brevet européen publiées sous les numéros 350 846, 303 186, 253 302, dans la demande de brevet internationale PCT publiée sous le numéro WO 93/11755, ou encore dans les brevets américains 5 300 522, 5 420 273, 4 578 498 et les brevets allemands 3602473 et 3715955, ainsi que dans les articles de Marcia I. Dawson et al. (*J*. *Med*. *Chem*., 1989, 32, 1504-1517 ; *J. Med*. *Chem. ,* 1993, 36, 2605-2613).

Les composés qui présentent une activité de type rétinoïde sont utiles pour le traitement des mammifères et plus particulièrement de l'homme par chimioprévention et par chimiothérapie, notamment dans le traitement des symptômes de nombreuses maladies telles que les dermatoses, l'acné, la maladie de Darier, le psoriasis, l'icthyose, l'eczéma. Ces composés sont aussi utilisés pour le traitement et la prévention de maladies cancéreuses et de nombreuses maladies hyperproliferatives malignes telles que les cancers du sein, de la prostate, du poumon, de la tête et du cou, ainsi que de certains types de cancers d'origine épithéliale et des leucémies myélocytaires. Les composés présentant une activité de type rétinoïde sont aussi utiles pour le traitement et la prévention de l'athérosclérose, de la resténose issue d'hyperprolifération néointimale, des pathologies hyperprolifératives bénignes telles que l'hyperplasie endométriale, l'hypertrophie bégnine de la prostate, la rétinopathie proliférative, pour le traitement des maladies auto-immunes et des désordres immunologiques tels que le lupus érythémateux, pour le traitement et la prévention de maladies associées au métabolisme des lipides et pour le traitement des effets du soleil sur la peau.

Toutefois, ces composés de type rétinoïde présentent des effets secondaires importants, notamment une forte irritation au niveau de la peau et des muqueuses, une perturbation du bilan lipidique, et sont même tératogènes, ce qui rend leur usage délicat en clinique (Kistler, A. et al. Arch Toxicol, 64 : 616-622 ; "Retinoids in Oncology", Edited by Waun Ki Hong & Reuben Lotan, The University of Texas M. D. Anderson Cancer Center, Houston, Texas, USA, Marcel Dekker Inc., pages 127-146 ; "Retinoids in Clinical Practice", Edited by Gideon Koren The Motherisk Program, The Hospital for Sick Children and The University of Toronto, Toronto, Ontario, Canada, Marcel Dekker Inc.).

Les effets néfastes rapportés ci-dessus ont conduit la Demanderesse à rechercher des composés polycycliques aromatiques de type rétinoïde actifs notamment dans le traitement et la prévention des maladies précédentes mais ne présentant pas d'effets secondaires.

La Demanderesse a conçu puis étudié les propriétés des nouveaux composés de type rétinoïde répondant à la formule (I), notamment par rapport à l'arotinoïde de référence : l'acide (E)-4-[2-(5,6,7,8-tétrahydro-5,5,8,8-tétraméthyl-naphtalényl)-1-propényl] benzoïque (TTNPB).

Les composés de formule (I) de l'invention comporte une double liaison entre les groupements aromatiques et peuvent donc exister sous les configurations cis (Z) ou trans (E) ou encore se présenter sous la forme d'un mélange d'isomères cis/trans.

Afin de faciliter la compréhension de l'isomérie ci-dessus, on utilisera généralement dans ce qui suit, la figure (Ia) pour représenter la configuration trans dans laquelle les groupements aromatiques sont situés de part et d'autre de la double liaison, et la figure (Ib) pour représenter la configuration cis dans laquelle les groupements aromatiques sont situés du même côté de la double liaison; l'invention concerne bien entendu le mélange des deux isomères aussi bien que chacun d'entre-eux pris séparément.

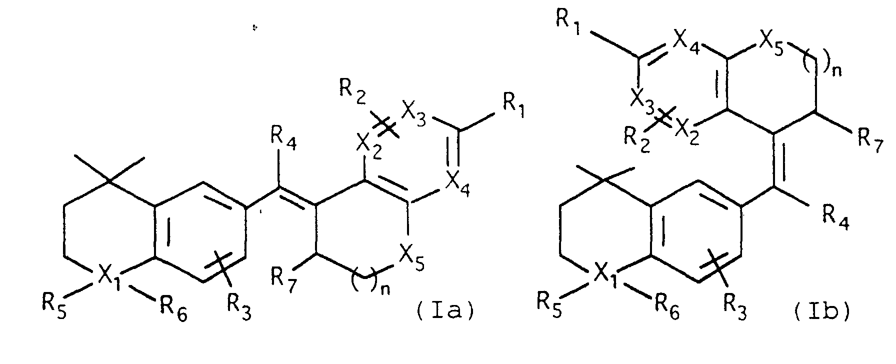

Selon la nature des radicaux R₅, R₆ et/ou R₇, les composés de formule (I) peuvent contenir un ou plusieurs carbones asymétriques. L'invention concerne donc tant le mélange racémique que chacun des énantiomères.

L'art antérieur et notamment les brevets et demandes de brevet sus-mentionnés indiquent logiquement l'existence d'isomères cis et trans, mais s'intéressent spécifiquement aux composés de configuration trans car cette configuration est celle rencontrée dans l'arotinoïde de référence cité ci-dessus.

Les travaux de recherche réalisés par la Demanderesse sur les composés de formule générale (I) l'ont conduit à démontrer que ces composés présentent une activité intrinsèque leur permettant de moduler la prolifération et la différenciation cellulaire, et autorisant leur application dans le traitement et la chimioprévention de maladies telles que le cancer du sein, le cancer de la prostate, le cancer des poumons, les cancers cutanés et les leucémies promyélocytaires dans des compositions non tératogéniques, et dans le traitement des symptômes de maladies comme l'acné, la maladie de Darier, le psoriasis, l'icthyose, l'eczéma .

La présente invention a donc pour objet les composés polycycliques aromatiques de formule générale (I) définie précédemment, leur procédé de préparation ainsi que leur utilisation en médecine humaine et vétérinaire, en dermatologie et en cosmétique.

Parmi les composés de formule (I), une série préférée est celle dans laquelle R₂ représente un atome d'hydrogène et R₁ est choisi parmi un groupe tétrazoyle, un groupe -COOH, un groupe -PO₃H₂, un groupe -CONH₂.

L'invention envisage à titre spécifique les composés de formule I suivants :

L'acide (E) 3-[2-(5,6,7,8-tétrahydro-5,5,8,8-tétraméthyl)naphtalénylméthylène]-dihydro-benzo[b]furane-5-carboxylique, désigné CB93128 et répondant à la formule suivante :

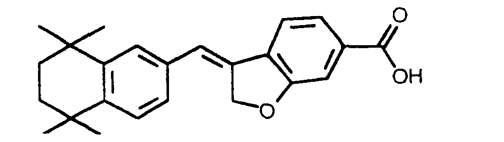

L'acide (Z) 1-[2-(5,6,7,8-tétrahydro-5,5,8,8-tétraméthyl)naphtalénylméthylène]-1,2,3,4-tétrahydro-naphtalène-6-carboxylique, désigné CB80830 et répondant à la formule suivante :

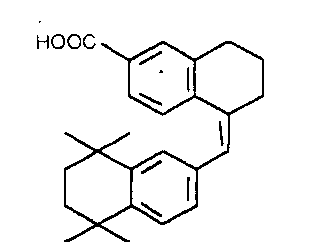

L'acide (E) 1-[2-(5,6,7,8-tétrahydro-5,5,8,8-tétraméthyl)naphtalénylméthylène]-1,2,3,4-tétrahydronaphtalène-6-carboxylique, désigné CB66049 et répondant à la formule suivante :

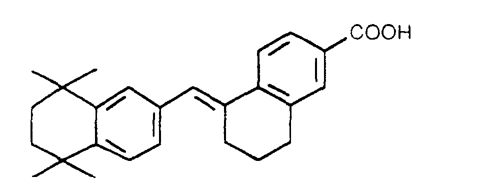

Le (E) 5-[1-[2-(5,6,7,8-tétrahydro-5,5,8,8-tétraméthyl)-2-naphtalénylméthylène]-1,2,3,4-tétrahydronaphtalènyl]-1*H*-tétrazole, désigné CB44858 et répondant à la formule suivante :

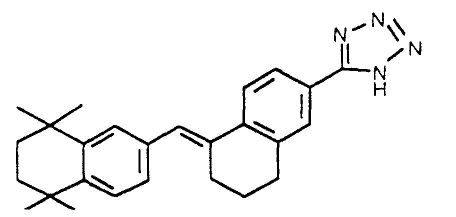

L'acide (Z) 1-[2-(5,6,7,8-tétrahydro-3,5,5,8,8-pentaméthyl)naphtalénylméthylène]-1,2,3,4-tétrahydronaphtalène-6-carboxylique, désigné CB53261 et répondant à la formule suivante :

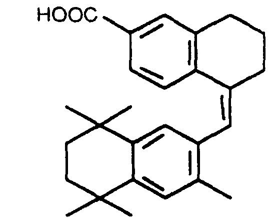

L'acide (E) 1-[2-(5,6,7,8-tétrahydro-3,5,5,8,8-pentaméthyl)naphtalénylméthylène]-1,2,3,4-tétrahydronaphtalène-6-carboxylique, désigné CB95970 et répondant à la formule suivante :

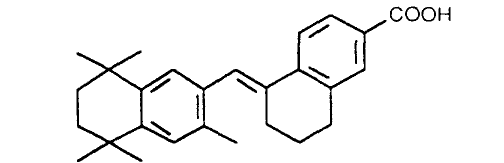

Le (Z) 5-[1-[2-(5,6,7,8-tétrahydro-3,5,5,8,8-pentaméthyl)naphtalénylméthylène]-1,2,3,4-tétrahydronaphtalènyl]-*1H*-tétrazole, désigné CB02305 et répondant à la formule suivante :

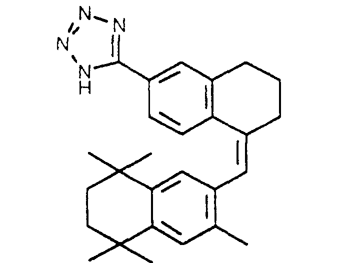

Le (E) 5-[1-[2-(5,6,7,8-tétrahydro-3,5,5,8,8-pentaméthyl)naphtalénylméthylène)-1,2,3,4-tétrahydronaphtalènyl]-1*H*-tétrazole, désigné CB58248 et répondant à la formule suivante :

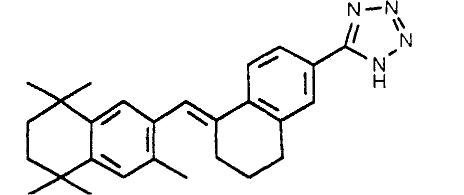

L'acide (E) 1-[2-(5,6,7,8-tétrahydro-5,5,8,8-tétraméthyl)naphtalénylméthylène]-2,3-dihydro-1*H*-indène-5-carboxylique, désigné CB78937 et répondant à la formule suivante :

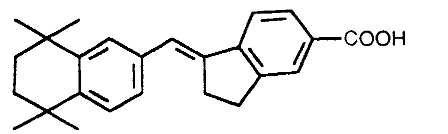

Le (E) 5-[1-[2-(5,6,7,8-tétrahydro-5,5,8,8-tétraméthyl)naphtalénylméthylène]-2,3-dihydro-1*H*-indényl]-1*H*-tétrazole, désigné CB99811 et répondant à la formule suivante :

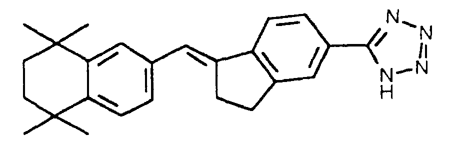

L'acide (Z) 1-[2-(5,6,7,8-tétrahydro-5,5,8,8-tétraméthyl)naphtalénylméthylène)-2,3-dihydro-1*H*-indène-5-carboxylique, désigné CB27871 et répondant à la formule suivante :

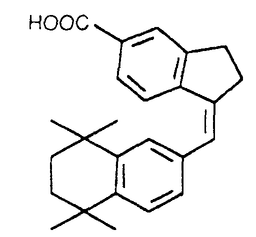

Le (Z) 5- [1- [2-(5,6,7,8-tétrahydro-5,5,8,8-tétraméthyl)naphtalénylméthylène]-2,3-dihydro-1*H*-indényl]-1*H*-tétrazole, désigné CB94083 et répondant à la formule suivante :

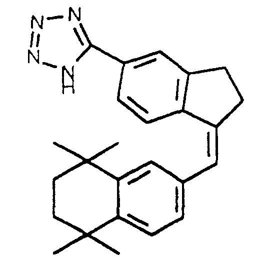

L'acide (Z) 1-[2-(5,6,7,8-tétrahydro-3,5,5,8,8-pentaméthyl)naphtalénylméthylène]-2,3-dihydro-1*H*-indène-5-carboxylique, désigné CB75403 et répondant à la formule suivante :

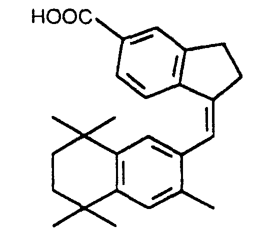

Le (Z) 5-[1-[2-(5,6,7,8-tétrahydro-3,5,5,8,8-pentaméthyl)naphtalénylméthylène]-2,3-dihydro-1*H*-indényl]-1*H*-tétrazole, désigné CB02981 et répondant à la formule suivante :

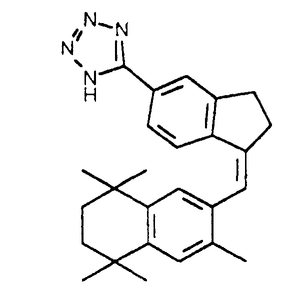

L'acide (E) 1-[2-(5,6,7,8-tétrahydro-3,5,5,8,8-pentaméthyl)naphtalénylméthylène]-2,3-dihydro-1*H*-indène-5-carboxylique, désigné CB40341 et répondant à la formule suivante :

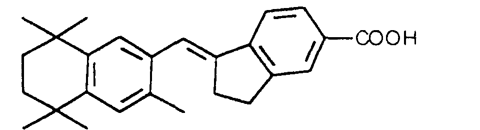

Le (E) 5-[1-[2-(5,6,7,8-tétrahydro-3,5,5,8,8-pentaméthyl)naphtalénylméthylène]-2,3-dihydro-1*H*-indényl]-1*H*-tétrazole, désigné CB23804 et répondant à la formule suivante :

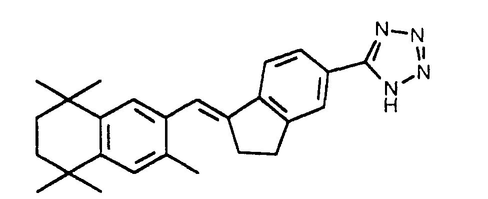

L'acide (E) 1-[2-(5,6,7,8-tétrahydro-3,5,5,8,8-pentaméthyl)naphtalénylméthylène]-2,3-dihydro-1*H*-indényl-5-phosphonique, désigné CB69179 et répondant à la formule suivante :

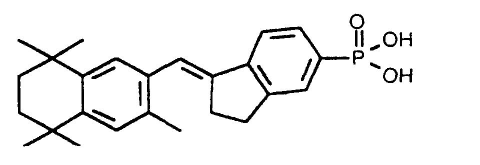

L'acide (E) 1-[2-(5,6,7,8-tétrahydro-5,5,8,8-tétraméthyl)naphtalénylméthylène]-2,3-dihydro-2-méthyl-1*H*-indène-5-carboxylique, désigné CB52809 et répondant à la formule suivante :

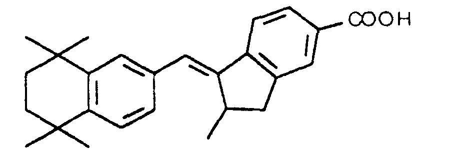

Le (Z) 1-[2-(5,6,7,8-tétrahydro-5,5,8,8-tétraméthyl)naphtalénylméthylène]-2,3-dihydro-2-méthyl-1*H*-indène-5-amide, désigné CB96711 et répondant à la formule suivante :

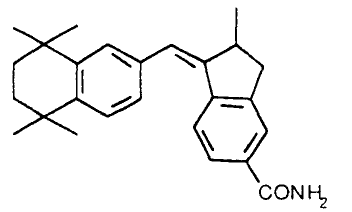

L'acide (Z) 1-[2-(5,6,7,8-tétrahydro-5,5,8,8-tétraméthyl)naphtalénylméthylène]-2,3-dihydro-2-méthyl-1*H*-indène-5-carboxylique, désigné CB91261 et répondant à la formule suivante :

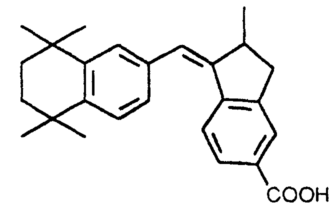

Le (E) 5-[1-[2-(5,6,7,8-tétrahydro-5,5,8,8-tétraméthyl)naphtalénylméthylène)-2,3-dihydro-2-méthyl-1*H*-indényl]-1*H*-tétrazole, désigné CB69831 et répondant à la formule suivante :

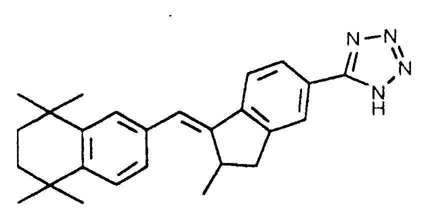

L'invention concerne aussi la préparation des composés de formule (I) et les compositions pharmaceutiques ou cosmétiques contenant comme principe actif au moins un desdits composés.

Les composés de l'invention peuvent être préparés par réaction de wittig entre un sel de phosphonium et une cétone selon le schéma suivant :

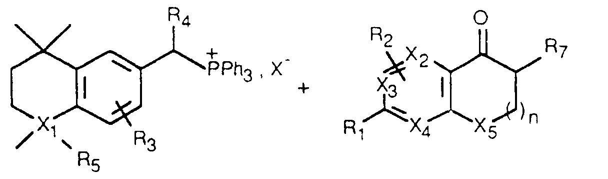

Ils peuvent aussi étre préparés par réaction de Wittig consistant à condenser un composé carbonylé (aldéhyde ou cétone) avec un sel de phosphonium selon le schéma suivant :

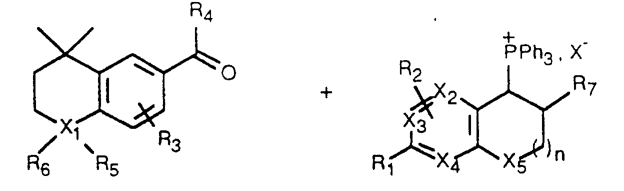

Les composé de l'invention peuvent être encore préparés par réaction de Horner-Emmons consistant à condenser un composé carbonylé (aldéhyde ou cétone) avec un phosphonate selon le même principe que les schémas ci-dessus avec un phosphonate remplaçant un sel de phosphonium. Ils peuvent aussi être obtenus par déshydratation d'alcools tertiaires répondant à l'une des formules suivantes :

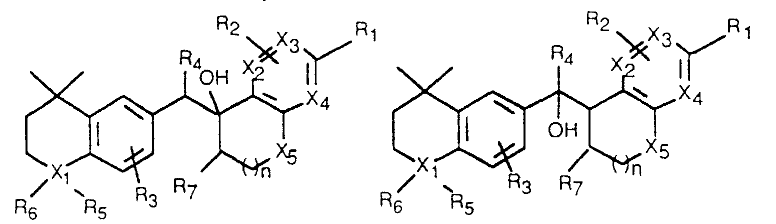

Les composés de l'invention peuvent être également préparés par réaction de couplage de type Suzuki (A.R. de Lera et al., Synthesis, 285, 1995) selon le schéma suivant :

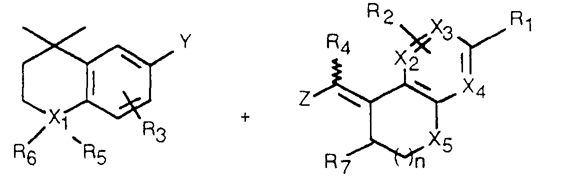

Dans les formules ci-dessus Y = B(OH)₂ et Z = OTf, I, Br; ou Y = OTf, I, Br et Z = B(OH)₂ et X₁, X₂, X₃, X₄, X₅, R₁, R₂, R₃, R₄, R₅, R₆ et R₇ ont les mêmes significations que dans la formule (I).

Les méthodes ci-dessus sont données à titre non limitatif, et tous autres procédés permettant de créer des doubles liaisons ou des liaisons carbone - carbone peuvent être utilisés pour préparer des composés de l'invention.

Les formes isomériques cis et trans des dérivés obtenus par ces différentes méthodes peuvent être séparées et purifiées soit au cours du processus de synthèse par changement de solvant ou addition de sel (March, J., Modern Organic Synthesis, 3rd Edition, Wiley Interscience, p. 845-854), soit au stade final, selon des techniques connues, telles que, par exemple, la recristallisation, l'HPLC préparative ou la chromatographie.

En outre, il est possible à partir des dérivés de formule (I) et de leurs isomères cis et trans obtenus selon les méthodes précédentes, de préparer d'autres dérivés par des réactions classiques au niveau d'un ou plusieurs des radicaux X₁, X₂, X₃, X₄, X₅, R₁, R₂, R₃, R₄, R₅, R₆ et R₇ . On peut citer par exemple les réactions suivantes :
- Un ester d'acide carboxylique de formule (I) dans laquelle R₁ est un groupe -COOR₁₁, peut être saponifié par des méthodes connues, par exemple par traitement avec des solutions alcalines, plus spécialement par traitement avec une solution hydro-alcoolique d'hydroxyde de sodium ou de potassium à des températures comprises entre l'ambiante au point d'ébullition du mélange réactionnel. L'acide carboxylique ainsi obtenu peut être transformé en amide via passage par un halogénure d'acide ou transformé en amide directement en utilisant une réaction de couplage peptidique, par exemple en utilisant comme agent de couplage du carbonyldiimidazole (CDI) et comme amine le 5-aminotétrazole en solution dans du THF à température ambiante (Paul R., Anderson G. W., J. Am. Chem. Soc., 82, 4596, 1960).
- Un acide carboxylique de formule (I) dans laquelle R₁ est un groupe -COOH, peut être converti par une méthode connue, par exemple par traitement avec du chlorure de thionyle, dans du toluène ou de la pyridine, ou par du trichlorure ou pentachlorure de phosphore dans du toluène, en chlorure d'acide. Ce chlorure d'acide peut être converti, en ester par réaction avec un alcool, ou en amide correspondant par réaction avec une amine.
- Un acide carboxylique ou un ester d'acide carboxylique de formule (I) dans laquelle R₁ est un groupe -COOH ou un groupe -COOR₁₁, peut être réduit par des méthodes connues de manière à donner l'alcool correspondant où R₁ est un groupe -CH₂OH.
- Un sulfure de formule (I) dans laquelle X₁ est un atome de soufre (-S-), peut être oxydé en sulfoxyde (-SO-) ou en sulfone (-SO₂-), R₆ et R₇ sont alors identiques ou différents et représentent soit rien (cas d'un sulfure : -S-), soit un oxygène (cas d'un sulfoxyde :-SO-) ou deux oxygènes (cas d'une sulfone : -SO₂-). L'oxydation d'un groupement sulfure peut être réalisée en utilisant des agents d'oxydation tels que des périodates (par exemple le périodate de sodium) ou en utilisant des peracides organiques tel que l'acide m-chloroperbenzoïque (mCPBA). Quand l'oxydation est réalisée en utilisant un peracide organique, environ 1 équivalent permet d'obtenir un sulfoxyde alors que l'utilisation de 2 équivalents de peracide conduisent à la sulfone.
- Un amide de formule (I) dans laquelle R₁ est un groupe -CONrr', peut être réduit par des méthodes connues de manière à donner un aldéhyde (R₁ = -CHO), par exemple par l'hydrure de diisobutylaluminium en solution dans le toluène, préférablement en utilisant du THF comme solvant réactionnel à des températures comprises entre -78°C et l'ambiante.
- Un aldéhyde de formule (I) dans laquelle R₁ est un groupe -CHO, peut être oxydé par des méthodes connues de manière à donner un acide carboxylique (R₁ = -COOH) ou un ester d'acide carboxylique (R₁ = -COOR₁₁), par exemple par la méthode de Corey (Corey E. J. et al., J. Am. Chem. Soc., 90, 5616, 1968) mettant en jeu le dioxyde de manganèse, du cyanure de sodium, de l'acide acétique et du méthanol à température ambiante.
- Un dérivé nitrile de formule (I) dans laquelle R₁ est un groupe -CN, peut être hydrolysé en acide carboxylique (R₁=-COOH) correspondant par des méthodes connues, par exemple par traitement avec des bases alcalines, plus spécialement par traitement avec une solution hydro-alcoolique d'hydroxyde de sodium ou de potassium à des températures comprises entre l'ambiante et le point d'ébullition du mélange réactionnel.
- Un dérivé nitrile de formule (I) dans laquelle R₁ est un groupe -CN, peut être transformé en 1*H*-tétrazole par des méthodes connues, par exemple par traitement avec de l'azoture de triméthylsilane N₃SiMe₃ en présence ou non de catalyseur tel que de l'oxyde de dibutylétain (Bu)₂SnO dans des solvants aromatiques préférablement du toluène ou du benzène à des températures comprises entre l'ambiante et le point d'ébullition du mélange réactionnel.
- Un dérivé aromatique bromé, iodé ou chloré, de formule (I) dans laquelle R₁ est un atome de brome, d'iode ou de chlore, peut être transformé par des méthodes connues en dérivé nitrile (R₁ = -CN), par exemple par la réaction de Rosenmund-von Braun utilisant du cyanure cuivreux dans un solvant, préférablement du diméthylformamide ou de la quinoléine à des températures comprises entre l'ambiante et le point d'ébullition du mélange réactionnel.
- Un dérivé aromatique bromé, iodé ou chloré, de formule (I) dans laquelle R₁ est un atome de brome, d'iode ou de chlore, peut être transformé par des méthodes connues en acide carboxylique (R₁ = -COOH), par exemple par échange halogène-métal en utilisant du butyllithium (primaire, secondaire ou tertiaire) en solution dans du THF à froid (-78°C à 0°C) et condensation de dioxyde de carbone puis remontée à l'ambiante.
- Un dérivé aromatique bromé, iodé ou chloré, de formule (I) dans laquelle R₁ est un atome de brome, d'iode ou de chlore, peut être transformé par des méthodes connues en ester d'acide carboxylique (R₁ = -COOR₁₁), par exemple par échange halogène-métal en utilisant du butyllithium (primaire, secondaire ou tertiaire) en solution dans du THF à froid (-78°C à 0°C) et condensation sur un chloroformiate d'alkyle.
- Les dérivés de formule (I) dans laquelle R₁ est un groupe -COOH ou -tétrazoyl, peuvent être transformés par des méthodes connues en sels par des bases physiologiquement acceptables, non toxiques, inorganiques ou organiques, par exemple en sels de métaux alcalins ou en sels de métaux alcalino-terreux, par exemple du sodium, du potassium, du magnésium ou des sels de calcium, aussi bien que des sels avec de l'ammoniaque ou des amines non toxiques.
- Un acide carboxylique de formule (I) dans laquelle R₁ est un groupe -COOH, peut être converti par une méthode connue, par exemple par un réarrangement de type Curtius, avec de l'azoture de diphénylphosphorane en présence de triéthylamine dans du toluène à 80°C, suivi d'une addition d'un alcool de type R₁₀OH, préférentiellement l'alcool méthylique (R₁₀ = Me) ou benzylique (R₁₀ = benzyl), en carbamate -NHCOOR₁₀, qui par traitement par de la soude aqueuse à 10% (R₁₀ = Me) ou par hydrogénation (R₁₀ = benzyl) conduit à une aniline de formule (I) où R₁ est un groupe -NH₂.
- Une aniline de formule (I) dans laquelle R₁ est un groupe -NH₂, peut être convertie par des méthodes connues de trifluoroacétylation, par exemple en utilisant la 2-(trifluoroacétyloxy)pyridine (TFAP) dans l'éther à des températures comprises entre 0°C au point d'ébullition du mélange réactionnel (T. Keumi et al., Bull. Chem. Soc. Jpn., 63, 2252, 1990), en l'amide trifluorométhylé de formule (I) où R₁ est un groupe -NH(C=O)CF₃. L'amide trifluorométhylé de formule (I) peut être converti par des méthodes connues en imine chlorée, préférentiellement en utilisant de la triphénylphosphine et du tétrachlorure de carbone (K. Tamura et al. J. Org. Chem., 58, 32, 1993), pour conduire au composé de formule (I) où R₁ est un groupe -N=C(CF₃)(Cl).
- Un chlorure de trifluoroacétimidoyle de formule (I) où R₁ est un groupe -N=C(CF₃)(Cl) peut être cyclisé par des méthodes connues, préférentiellement par de l'azoture de sodium dans l'acide acétique à 70°C (D. Armour et al., Bioorg. & Med. Chem. Lett., 6, 1015, 1996), pour conduire à un tétrazole de formule (I) où R₁ est un groupe de formule suivante : 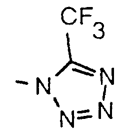
- Un composé halogéné de formule (I) où R₁ est un brome, un chlore ou un iode peut être converti par réaction d'Arbuzov, préférentiellement par traitement du diéthylphosphite dans du toluène au point d'ébullition du mélange réactionnel, en phosphonate de diéthyle de formule (I) où R₁ est un groupe -P(O)(OEt)₂.
- Un phosphonate de formule (I) où R₁ est un groupe -P(O)(OEt)₂ peut être hydrolysé par des méthodes connues, par exemple en présence d'iodure de triméthylsilyle, pour conduire à un acide phosphonique de formule (I) où R₁ est un groupe -PO₃H₂.

Les cétones ayant servi à la préparation des composés de l'invention ont été préparées comme suit :
- La 6-bromo-3-coumarone est préparée en quatre étapes à partir du 3-bromophénol commercial. La première étape consiste en une O-alkylation du 3-bromophénol par le bromoacétate de méthyle. La saponification du 3-bromophénoxyacétate de méthyle permet d'obtenir l'acide 3-bromophénoxyacétique, qui est transformé en chlorure d'acide par action du chlorure de thionyle, celui-ci subit une acylation de Friedel et Craft intramoléculaire pour conduire à la 6-bromo-3-coumarone.
- La préparation de la 6-cyano-1-tétralone a été décrite dans l'art antérieur (C. Almansa et al. Synthetic Commun., 23, 2965, 1993). La 6-méthoxy-1-tétralone en présence d'acide bromhydrique aqueux conduit à la 6-hydroxy-1-tétralone qui, en présence d'anhydride trifluorométhanesulfonique dans la pyridine permet d'obtenir la 6-trifluorométhanesulfonyl-1-tétralone. L'action du cyanure de potassium sur le triflate obtenu précédemment en présence de nickel(0) permet l'obtention de la 6-cyano-1-tétralone.

Les composés de l'invention présentent des propriétés très intéressantes sur la différenciation et la prolifération cellulaire, permettant d'envisager leur utilisation à des fins thérapeutiques, dermatologiques et cosmétiques. Parmi les utilisations thérapeutiques, on peut citer le traitement et la prévention des cancers du type tumeurs solides, tels que les cancers du sein, des poumons, de la prostate ou du foie, ainsi qué le traitement et la prévention de maladies de la peau, tels que le psoriasis et l'acné.

En outre, les travaux de biologie moléculaire, rapportés ci-après, ont permis de définir les profils d'activité des composés de l'invention sur les récepteurs rétinoïques et certains facteurs transcriptionnels.

Or, il a été récemment proposé d'utiliser des composés RXR agonistes dans le traitement du diabète non-insulino-dépendant, des maladies inflammatoires et immunitaires. En conséquence, les composés de l'invention sont utiles pour le traitement du diabéte non insulino-dépendant, des maladies inflammatoires et immunitaires.

L'invention concerne donc aussi l'utilisation des composés de formule (I) pour la fabrication de compositions pharmaceutiques utiles dans le traitement ou la prévention des cancers ainsi que pour le traitement du diabéte non insulino-dépendant, des maladies inflammatoires et immunitaires. Ils peuvent aussi être utilisés pour la fabrication de compositions cosmétiques utiles dans le traitement ou la prévention de maladies de la peau.

En tant que médicament, les composés de l'invention sont administrés sous forme d'une composition pharmaceutique comprenant au moins desdits dérivés, sous forme libre ou sous forme d'un sel pharmaceutiquement acceptable, en association avec un véhicule ou diluant traditionnel. Ces composés peuvent aussi être administrés sous forme d'esters hydrolysables pharmaceutiquement acceptables. Par pharmaceutiquement acceptable, on entend des esters tels que les esters de benzyle, ou benzyle substitués, et plus particulièrement substitués par des radicaux alkyles de 1 à 6 atomes de carbone linéaires ou ramifiés.

De telles compositions, qui font également partie de l'invention, peuvent se présenter pour l'administration par voie entérale, par exemple sous forme de comprimés, ou pour l'administration par voie parentérale, par exemple sous forme de solutions ou de suspensions injectables par voie intraveineuse ou musculaire, ou encore pour l'administration sous forme de pulvérisation nasale.

En tant que cosmétique, les composés de l'invention sont administrés sous forme d'une composition cosmétique comprenant au moins un desdits dérivés, sous forme libre ou sous forme d'un sel pharmaceutiquement acceptable, en association avec un véhicule ou diluant traditionnel. De telles compositions, qui font également partie de l'invention, peuvent se présenter pour l'administration par voie entérale, par exemple sous forme de comprimés, ou pour l'administration par voie parentérale, par exemple sous forme de solutions ou de suspensions injectables par voie intraveineuse ou musculaire, ou encore pour l'administration sous forme de pulvérisation nasale, ou avantageusement pour une application topique, sous forme de crèmes, pommades, de laits, de poudres ou de gels.

Les véhicules et diluants susceptibles d'être utilisés en association avec les composés de l'invention sont ceux généralement utilisés dans ce type d'indications.

Pour les indications précédentes, la dose dépend du mode d'administration et du traitement désiré. On obtient des résultats satisfaisants lorsque le dérivé est administré à une dose quotidienne comprise entre 0,1 mg/kg et environ 100 mg/kg. Chez l'homme, l'administration est effectuée par exemple par voie intraveineuse, en une dose unique par jour ou en doses fractionnées jusqu'à plusieurs fois par jour, sous forme de doses unitaires contenant une concentration de 0,001 % à environ 0,01 % de substance active.

### ACTIVITÉS DES COMPOSÉS DE L'INVENTION SUR LES RÉCEPTEURS RÉTINOÏOUES ET CERTAINS FACTEURS TRANSCRIPTIONNELS.

### I) Modèles et molécules de références.

L'acide rétinoïque tout trans (ttRA) et ses stéréoisomères 9-cis, 11-cis et 13-cis se lient et activent, plus ou moins sélectivement, des récepteurs intranucléaires dits récepteurs rétinoïques. Une avancée importante dans le mécanisme d'action moléculaire du signal de tranaduction de l'acide rétinoïque a été établie notamment gräce aux travaux pionniers de R. M. Evans et al. (Sciences, 1988, 240, 889-895).

Les componés de l'invention du type rétinoïde ou arotinoïde possèdent des profils de sélectivité différents vis à vis des sous-types de récepteurs de l'acide rétinoïque (RARs) et des récepteurs X rétinoïdes (RXRs).

Un grand nombre de résultats cliniques récents ont montré que l'acide rétinoïque, certains de ses isomères et des dérivés formant la classe des rétinoïdes, sont utilisés pour le traitement de maladies telles que l'acné, le psoriasis et certains cancers (U. Reichert et al, Pharmacology of Retinoids in the Skin, Karger AG Eds, Basel, 1989 ; M.S. Tallman et al, Retinoids in Cancer Treatment, *J.* Clin. Pharmacol., 1992, 32, 868-888 ; Warrell et al, N, Engl. *J.* Med., 1991, 324, 1385-1393).

Ces rétinoïdes sont aussi évalués dans d'autres domaines thérapeutiques tels que, à titre indicatif : l'arthrite (Vinienti M.P. et al, Using Inhibitors of Metalloproteinases to treat Arthritis, Arthritis Rheumatoidism, 1994, 37, 1115-1126) ; la dyslipidémie (Rottman et al, A RARE Element in the Apolipoprotein AI Gene Distinguishes Between Two Different Retinoic Acid Response Pathways, Mol. Cell. Biol., 1991, 3814-3820) ; la prévention de la lymphopénie VIH induite (Yang Y. et al, 9-cis RA Inhibits Activation-Driven T-Cell Apoptosis: Implications for Retinoid X Receptor Involvement in Thymocyte Development, Proc.Natl. Acad. Sci. USA, 1993, 90, 6170-6174). Ces effets thérapeutiques résultent de la capacité de l'acide rétinoïque et de certains rétinoïdes à contrôler des situations cellulaires anormales par la modulation de la croissance cellulaire, de la différenciation cellulaire et/ou de l'apoptose ou mort cellulaire programmée (The Retinoids, Biology, Chemistry and Medecine, M.B. Sporn, A.B. Roberts et D.S Goodman, Raven Press Eds, 2nd ed, New York 1994). Ces régulations ont été attribuées pour large partie à la formation de complexes ligand(s)-récepteurs. Ces protéines appartiennent à la superfamille des récepteurs nucléaires, et fonctionnent comme facteurs de transcriptions ligands dépendants. Ce sont ces interactions qui sont responsables de l'activation transcriptionnelle et des effets physiologiques associés.

A l'aide de ligands endogènes et synthétiques, cette famille a été classée en deux séries dénommées RAR et RXR, composées chacune en trois sous-types de récepteurs appelés α, β et γ. De plus, ces rétinoïdes se sont montrés capables de réguler l'expression d'autres gènes par un effet inhibiteur de facteurs transcriptionnels comme le complexe AP-1 composé des protéines oncogènes c-Fos et c-Jun. Toutes ces protéines récepteurs modulent l'expression de certains gènes par liaison sélective, sous forme de dimère, à des régions spécifiques de l'ADN appelées RARE's (pour Retinoic Acid Response Element's, M.B. Sporn et al, p.319-349, D.J. Mangelsdorf et al., Proc. Natl. Acad. Sci. USA., 1991, 88, 3559-3563).

Les récepteurs RXR fonctionnent comme homodimères ou peuvent s'hétérodimériser avec les récepteurs RAR ainsi qu'avec les autres membres de la superfamille des récepteurs intracellulaires.

L'acide rétinoïque tout trans (ttRA) est le ligand naturel des récepteurs RARs, alors que son isomère 9-cis (9-cis RA) est à la fois ligand des récepteurs RXRs et RARs sous forme d'homodimères et d'hétérodimères (M. B. Sporn, page 5-178, X-K. Zhang et al, Homodimer formation of Retinoid X receptor induced by 9-cis RA, Nature, 1992, 358, 587-591, Heyman R.A. et al , 9-cis RA is a high affinity ligand for the Retinoic Receptor X, Cell, 1992, 68, 397-406, Levin A. A. et al, 9-cis RA stereoisomer binds and activates the nuclear receptor RXRα, Nature, 1992, 355, 359-361).

Il a été montré que ces récepteurs sont significativement différents : les structures primaires des domaines de liaisons (composition en acides aminés) sont différentes à plus de 80%. De même, une distribution différente de ces sous-types de récepteurs est fonction de la nature des tissus. Par exemple, les RARs ne sont pas exprimés dans les viscères, par contre les RXRα mRNA sont les plus abondants dans le foie, les reins, les poumons, l'intestin et les muscles.

Les voies hormono-dépendantes des RARs peuvent être activées par des ligands RAR spécifiques qui se lient à la partie RAR des RAR-RXR hétérodimères, tandis que des ligands specifiques RXR se révèlent incapables d'activer ces mêmes voies en se fixant sur la partie RXR. Des ligands RXR présentent une synergie d'activation des gènes répondant au ttRA quand ils sont utilisés en association avec des ligands spécifiques RARs (Roy B. et al, Mol. Cell Biol., 1995, 15, 6481-6487). Les RXRs forment des homodimères, en présence de ligands RXR et régulent la transcription de gènes qui sont distincts de ceux contrôlés par les hétérodimères RAR-RXR (Zhang X.-K. et al. cité ci-dessus).

Ainsi des rétinoïdes qui sont sélectifs pour des sous-types de récepteurs seront utiles à un contrôle sélectif voir indépendant des voies physiologiques médiées par ces mêmes sous-types. Par comparaison un panagoniste sera utile pour contrôler les voies physiologiques médiées par plusieurs de ces sous-types. Il apparaît que des rétinoïdes agissant sélectivement sur ces sous types pourront augmenter l'efficacité thérapeutique et réduire le profil des effets secondaires. Un panagoniste est défini comme un agent qui se lie et active au moins un des récepteurs de la sous-famille RAR et de la sous famille RXR. Un véritable panagoniste active tous les membres des sous familles RAR et RXR.

L'acide rétinoique tout trans (ttRA), comme son isomère 13-cis, a lors de tout traitement chronique un puissant effet d'hypervitaminose, de toxicité mucocutanée et de tératogénécité. De plus, ttRA est inducteur de son propre métabolisme ce qui a pour effet direct de diminuer rapidement son efficacité thérapeutique.

C'est pourquoi, la présente invention vise à fournir de nouveaux composés ayant une plus grande stabilité chimique et métabolique, et des profils d'activité différents vis à vis de ces sous-types de récepteurs reliés à des activités anti-tumorales et anti-prolifératives sélectives bien établies. Une telle stratégie a conduit à la formation de molécules :
- panagonistes RAR-RXR,
- sélectives RAR ou RXR,
- dissociantes anti-AP-1.
   Par leur propriété propre de co-activation des protéines RAR, des rétinoides RXR-sélectifs constituent un nouveau plus thérapeutique. A des doses où ils sont inactifs par eux-mêmes, ils peuvent augmenter l'activité de rétinoïdes RAR-sélectifs, et notamment RARα, alors utiles dans le traitement (régression ou rémission) de cancers de type leucémies, de tumeurs solides, plus particulièrement du sein, de la tête et du cou, mais aussi de manière plus classique dans les épisodes d'acné, d'acné sévère et de peau endommagée par le soleil. L'administration de rétinoïdes utilisés en combinaison, peut être concommitante ou simultanée. Dans ce cas, l'écart d'administration entre les rétinoïdes ne doit pas dépasser quelques heures, de telle sorte que les rétinoides RXR et RAR seraient dans des concentrations sanguines telles que la potentialisation soit effective.

### 1) Expression des récepteurs RAR, RXR et RE en fonction des lignées cellulaires.

Les cellules MCF-7 et HeLa sont cultivées dans du DMEM avec rouge de phénol additionné de sérum de veau foetal 5%. Les cellules T47-D sont cultivées dans du RPMI additionné de sérum de veau foetal 10%. Les essais expérimentaux sont réalisés dans du DMEM sans rouge de phénol additionné de sérum de veau foetal traîté au charbon dextran à 3 %. Les lignées cellulaires transfectées de façon stable, issues des lignées MCF-7 et HeLa, sont établies selon le protocole décrit par D. Gagne et al. (J. Biolumin. Chemilumin., 1994, 9, 201-209). Les expériences utilisant les différents rétinoïdes sont réalisées à l'abri de la lumière afin d'éviter toute isomérisation.

Le tableau 1 ci-dessous rapporte la différence d'expression des récepteurs de l'acide rétinoïque et du récepteur aux estrogènes RE par les cellules HeLa et MCF-7 (Titcomb M. W. et al., Mol. Endocrinol., 1994, 8, 870-877). Les résultats du tableau 1 sont exprimés en fentomoles de récepteur par mg de protéines.

**Tableau 1**

| Type de récepteur | HeLa | MCF-7 |
|---|---|---|
| RE | non détecté | exprimé |
| RARα | 28 | 80 |
| RARβ | 9 | non détecté |
| RARγ | 16 | 34 |
| RXRα | 50 | 12 |
| RXRβ | 28 | non détecté |
| RXRγ | 9 | non détecté |

### 2) Spécificité des molécules de référence dans des modèles de transfections transitoires.

### a) Récepteurs chimériques Gal4-RAR.

Les études de spécificité transactivatrice des rétinoïdes ont été réalisées par transfection transitoire de cellules HeLa. Deux types de récepteurs chimériques peuvent alors être exprimés par les cellules. Les plasmides Gal-RARα, Gal-RARβ et Gal-RARγ (J.Y. Chen et al., EMBO J., 1995, 14, 1187-1197) codent pour des récepteurs chimériques Gal4-RAR dans lesquels le domaine de liaison à l'ADN de la protéine Gal4 de levure est fusionné avec les régions E et F (régions contenant le domaine de liaison au ligand et la fonction d'activation AF-2) des récepteurs de l'acide rétinoïque. La région C (domaine de liaison à l'ADN) et les régions A et B (domaine d'activation AF-1) sont supprimées.

Ces récepteurs chimériques activés par un agoniste stimulent spécifiquement la transcription du gène de la luciférase présent dans un plasmide cotransfecté ((17M)₅-βG-Luc) où 17M est l'élément de réponse de Gal4. La coopérativité transcriptionnelle entre AF-1 et AF-2 n'existe pas avec ce type de récepteur.

L'utilisation des molécules de référence a permis de vérifier la validité du modèle GAL-RAR pour déterminer la spécificité de molécules agonistes : ce modèle GAL-RAR traduit l'affinité d'un composé pour le domaine de liaison à l'hormone de RAR. L'arotinoïde TTNPB, à la concentration 10⁻⁸ M, est utilisé comme référence de transactivation maximale (100%) obtenue avec un agoniste synthétique. Ainsi TTNPB et ttRA sont de bons agonistes RAR tandis que Am580 se comporte bien comme un RARα spécifique à la concentration de 10 nM. Les composés décrits comme RXR spécifiques (LGD1069 et LGD-CB14499) ne permettent pas d'observer une bonne transactivation médiée par RAR. Le tableau 2 ci-dessous rapporte ces résulats, où l'activité des composés est exprimée en pourcentage de l'activité mesurée pour le TTNPB 10⁻⁸ M.

Le composé désigné LGD1069 (Boehm M.F. et al., *J. Med.Chem.,* 37, 2930-2941, 1999) répond à la formule suivante :

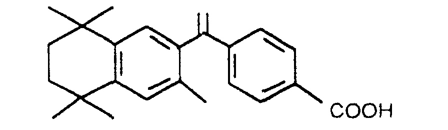

Le composé désigné LGD-CB14499 (Boehm M.F. et al., *J. Med*.*Chem*., 37, 2930-2941, 1994) répond à la formule suivante :

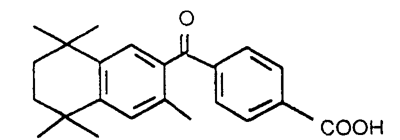

Le composé désigné Am580 est l'acide 4-[(5,6,7,8-tétrahydro-5,5,8,8-tetraméthyl-2-naphtalényl) carboxamido]benzoïque (Shudo K. et al., J. Med. Chem., 1988, 31, 2182-2192).

**Tableau 2**

| GAL4-RAR | | | | |
|---|---|---|---|---|
| Produit | Concentration (Log M) | RARα (%) | RARβ (%) | RARγ (%) |
| | -8 | 82 | 104 | 106 |
| ttRA | -7 | 102 | 93 | 104 |
| | -6 | 108 | 92 | 111 |
| | -8 | 110 | 22 | 12 |
| Am580 | -7 | 102 | 77 | 64 |
| | -6 | 113 | 111 | 96 |
| | -8 | 100 | 100 | 100 |
| TTNPB | -7 | 101 | 116 | 94 |
| | -6 | 105 | 109 | 112 |
| | -8 | 0 | 1 | 1 |
| LGD1069 | -7 | 7 | 5 | 8 |
| | -6 | 21 | 22 | 29 |
| LGD- | -8 | 1 | 0 | 0 |
| CB14499 | -7 | 4 | 1 | 0 |
| | -6 | 17 | 9 | 13 |

b) Récepteurs chimériques ERcassettes. Les récepteurs chimériques RAR-ERcassettes ont été décrits par Petkovitch et al. (Nature, 1987, 330, 444-450). Les cellules HeLa sont cotransfectées par un plasmide qui code pour un récepteur rétinoïque dans lequel le domaine C de liaison à l'ADN est substitué par celui du récepteur aux estrogènes RE, et un plasmide qui place l'expression de la luciférase sous le contrôle d'un ERE (Elément de Réponse aux estrogènes). Les récepteurs chimériques exprimés sont désignés comme RARα-ERcassette, RARβ-ERcassette, RARγ-ERcassette et RXRα-ERcassette. Les régions A, B, D, E et F du récepteur naturel sont conservées ainsi que la coopérativité transcriptionnelle entre les domaines d'activation AF-1 et AF-2. Ces expériences de transfections transitoires suivent la méthode de coprécipitation au phosphate de calcium. Les cellules HeLa sont cotransfectées par 0,25 mg de plasmide codant pour le récepteur chimérique, 1 mg de plasmide rapporteur et 0,5 mg de vecteur d'expression CMV-β-galactosidase utilisé comme contrôle interne de transfection. 24 heures après la transfection, les cellules sont incubées pendant 16 heures avec les différents effecteurs.

Dans ce type de récepteur chimérique, le domaine C de liaison à l'ADN de RAR est substitué par celui du récepteur aux estrogènes RE. La protéine obtenue est proche du récepteur RAR naturel et conserve les propriétés transcriptionnelles des domaines AF-1 et AF-2. Elle module la transcription par l'intermédiaire d'un ERE. Ce modèle RAR-ERcassette permet d'observer une réponse transcriptionnelle à un ligand plus physiologique.

L'effet des hormones naturelles (ttRA et 9-cis RA) est remarquable. Ces ligands induisent une surexpression de luciférase en comparaison à l'induction provoquée par un composé synthétique (TTNPB). Ce phénomène est également observé sur d'autres réponses (cf notamment résultats de transactivation sur les modèles HRLN). Cela montre que les constructions RAR-ERcassettes reflètent un contexte physiologique. La spécificité RARα de Am580 est à nouveau constatée pour les concentrations inférieures à 10 nM. Le profil défini en RAR-ERcassette pour les agonistes RXR (LGD1069 et LGD-CB14499) diffère de leur profil GAL-RAR. En comparaison au TTNPB, leur pouvoir transcriptionnel apparaît plus élevé notamment par l'intermédiaire de RARβ-ERcassette. Ce résultat peut s'expliquer par une hétérodimérisation de RAR-ERcassette avec les récepteurs RXR endogènes exprimés par les cellules HeLa transfectées. LGD1069 1 µM permet une coactivation de RAR et de RXR, entraînant une suractivation de l'hétérodimère par rapport à l'activation RAR spécifique du TTNPB. Le modèle RAR-ERcassette traduit donc une activité RAR d'un composé et aussi une activité RXR. Ainsi, toute molécule panagoniste entraîne une transcription maximale supérieure à celle du TTNPB. Cette observation est confirmée par le fait que si une molécule RAR spécifique est associée à une molécule RXR spécifique, une surexpression de luciférase intervient. L'utilisation de la construction RAR-ERcassette permet ainsi de visualiser et mettre en évidence une activité RXR comme rapporté dans le tableau 3 ci-dessous.

**Tableau 3**

| RAR-ERcassettes | | | | |
|---|---|---|---|---|
| Produit | Concentration (LogM) | RARα (%) | RARβ (%) | RARγ (%) |
| | -9 | 65 | 93 | 86 |
| ttRA | -8 | 127 | 128 | 114 |
| | -7 | 249 | 270 | 122 |
| | -6 | 443 | 475 | 189 |
| | -9 | 7 | 16 | 11 |
| 9-cis RA | -8 | 60 | 94 | 56 |
| | -7 | 187 | 212 | 108 |
| | -6 | 348 | 369 | 157 |
| | -9 | 88 | 6 | 9 |
| Am580 | -8 | 113 | 70 | 45 |
| | -7 | 115 | 99 | 93 |
| | -6 | 106 | 89 | 100 |
| | -9 | 52 | 93 | 66 |
| TTNPB | -8 | 100 | 100 | 100 |
| | -7 | 119 | 104 | 97 |
| | -6 | 123 | 116 | 112 |
| | -8 | 14 | 78 | 19 |
| LGD1069 | -7 | 36 | 194 | 41 |
| | -6 | 70 | 237 | 62 |
| LGD- | -8 | 4 | 10 | 13 |
| CB14499 | -7 | 12 | 58 | 31 |
| | -6 | 42 | 130 | 66 |

### 3) Effet des rétinoïdes de référence sur la prolifération estrogéno-induite - Lignées cellulaires MCF-7 et T-47D.

Afin de tester l'effet antiprolifératif des rétinoïdes, des expériences de croissances en conditions estrogéniques sur des cellules MCF-7 et T-47D ont été réalisées. Ce sont des cellules cancéreuses mammaires humaines estrogéno-dépendantes qui expriment le récepteur aux estrogènes RE. L'effet des molécules est évalué après 7 jours de culture en conditions estrogéniques (estradiol 10⁻⁹ M) par dosage de l'ADN cellulaire. Les cellules sont distribuées dans des plaques 24 puits à une densité de 2.10⁴ cellules par puits. Les essais avec les différents rétinoïdes sont réalisés en triple et le milieu de culture est changé après 4 jours de croissance. L'ADN cellulaire est mesuré par la méthode du 4,6-diamidino-2-phenylindole (C.F. Brunck et al., Anal Biochem., 1979, 92, 497-500). L'activité des composés est exprimée en pourcentage, 100% représentant la quantité d'ADN mesurée avec l'estradiol 10⁻⁹ M.

Le tableau 4 ci-dessous présente les concentrations de rétinoïde nécessaires pour inhiber de 50% la croissance des cellules MCF-7 et T-47D, ou le pourcentage d'inhibition de croissance à la concentration de 1 µM. Les molécules RAR spécifiques (TTNPB et Am580) exercent un effet inhibiteur plus fort que les ligands naturels (ttRA et 9-cis RA) et que LGD1069 (RXR agoniste). Ces résultats confirment ceux rapportés par Dawson et al. , Cancer Res., 1995, 55, 446-451, qui ont montré que des RARα agonistes sont des inhibiteurs efficaces de la croissance des cellules MCF-7, et que l'affinité des rétinoïdes pour RARα est étroitement corrélée à leur activité anti-proliférative. A la concentration de 10⁻⁸ M, LGD1069 est RXRα spécifique et n'exerce aucun effet sur la croissance en condition estrogénique des cellules MCF-7 et T-47D.

**Tableau 4**

| EFFET ANTIPROLIFERATIF | | |
|---|---|---|
| Produit | T-47D IC₅₀ (nM) | MCF-7 IC₅₀ (nM) |
| ttRA | 39,1 | 14,1 +/- 10,3 |
| 9-cis RA | 25,1 +/ -1,4 | Non déterminé |
| Am580 | 74% | 67% |
| TTNPB | 3,3 +/- 1,1 | 0,35 +/- 0,07 |
| LGD1069 | 20% | 33% |

Certaines propriétés des rétinoïdes ont été déterminées gràce à des modèles cellulaires plus élaborés. Ces modèles consistent en des lignées cellulaires transfectées de façon stable par des plasmides recombinants qui placent l'expression du gène de la luciférase sous le contrôle de différents éléments de réponse nucléaire. Les effets observés correspondent alors à des régulations physiologiques et à une activité des récepteurs endogènes. Les essais réalisés en double sont décrits ci-dessous pour chacun de ces modèles.

### 4) Activité transactivatrice des rétinoïdes de référence médiée par les récepteurs de l'acide rétinoïque - Lignées cellulaires HRLN et HRL+N transfectées de facon stable.

Les lignées cellulaires HRLN et HRL⁺N permettent d'étudier l'activation d'un RARE par des récepteurs endogènes en utilisant des ligands à concentrations physiologiques. Ces lignées dérivent de cellules HeLa transfectées de façon stable par un gène rapporteur qui place l'expression du gène de la luciférase sous le contrôle d'un élément de réponse nucléaire RARE (RAPE₃-tk-Luc). Les cellules HeLa expriment tous les récepteurs de l'acide rétinoïque connus (RARα,β,γ et RXRα,β,γ) avec une prédominance de RARα et RXRα. L'élément de réponse RARE utilisé pour les cellules HRLN correspond à la séquence du gène naturel du récepteur RARβ (GGTTCAnnnnnAGTTCA). Les cellules HRL⁺N comportent la séquence GAGTGAnnnnnCGGTGA.

### a) Lignée HRLN.

Cette lignée comporte l'élément de réponse RARE du gène naturel du récepteur RARβ qui contrôle l'expression du gène de la luciférase. ttRA et 9-cis RA induisent une activation dose-dépendante. Une suractivation par rapport au TTNPB est observée à forte concentration (1 µM), comparable à celle observée avec les constructions ERcassettes. Une coactivation de RAR et de RXR au niveau de l'hétérodimère est certainement impliquée.

Les résultats concernant TTNPB et Am580 indiquent l'activation induite spécifiquement par les récepteurs RAR. Les EC₅₀ de ces deux molécules sont similaires. Am580 induit une transactivation médiée par RARα et TTNPB par RARα,γ comme le montre l'utilisation de l'antagoniste RARα Ro 41-5253 (Apfel, C. et al., PNAS, USA, 1992, 89, 7129-7133) qui abolit totalement la réponse de Am580 et partiellement celle du TTNPB. Cependant, RARα apparaît comme le récepteur prédominant pour la transactivation dans les cellules HeLa. Le ligand RXRα spécifique LGD1069 transactive avec une EC₅₀ de ~ 10 nM, ce qui correspond à son activité RXR. La lignée HRLN permet de mettre clairement en évidence une activité RAR physiologique des composés, mais une faible activité RXR est observée. Le tableau 5 ci-dessous rapporte ces résultats où le 100% d'expression correspond à l'induction provoquée par TTNPB 10⁻⁸ M. Les EC₅₀ sont déterminées à partir des résultats obtenus avec une gamme de concentrations allant de 1 nM à 1 µM.

**Tableau 5**

| TRANSACTIVATION HRLN | | |
|---|---|---|
| Produit | E max % | EC₅₀ (nM) |
| ttRA | 208 | 2,5 +/- 4 |
| 9-cis RA | 196 | non déterminé |
| Am580 | 104 | 0,10 +/- 0,06 |
| TTNPB | 100 | 0,55 +/- 0,72 |
| LGD1069 | 73 | 9,4 +/- 6,3 |
| LGD-CB14499 | 30 | non déterminé |

### b) Lignée HRL+N.

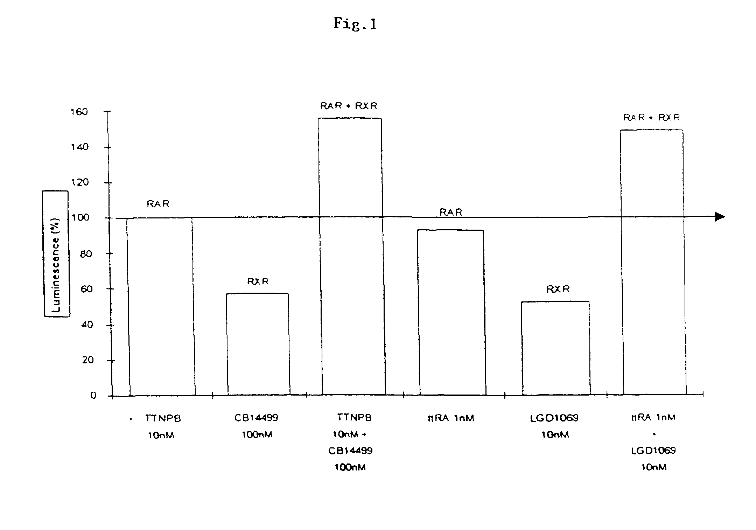

Les résultats de transactivation obtenus avec la lignée HRL⁺N sont comparables à ceux obtenus avec la lignée HRLN pour les molécules RAR agonistes (TTNPB et Am580) et les ligands naturels (ttRA et 9-cis RA). Les agonistes RXR (LGD1069 et LGD-CB14499) induisent une transactivation plus forte avec les cellules HRL⁺N et LGD1069 1 µM est plus efficace que les molécules RAR spécifiques. De plus, l'association d'un agoniste RAR et d'un agoniste RXR (par exemple TTNPB + LGD-CB14499, figure 1 en annexe) permet une meilleure transactivation que celle provoquée par chaque molécule utilisée séparément. La lignée HRL⁺N permet de visualiser une coactivation des récepteurs RAR et RXR au niveau de l'hétérodimère RAR-RXR. Ainsi, LGD1069 1 µM ayant une activité RAR à cette concentration se comporte comme un panagoniste. Ce résultat est à corréler à la suractivation induite par les molécules RXR agonistes avec les modèles RAR-ERcassettes. La lignée HRL⁺N permet clairement de mettre en évidence une activité RAR et RXR des molécules. Ces résultats sont rapportés dans le tableau 6 ci-dessous où l'activité transcriptionnelle des produits est exprimée en pourcentage, 100% correspondant au niveau d'activité mesuré en présence de TTNPB 10⁻⁸ M.

**Tableau 6**

| TRANSACTIVATION HRL+N | |
|---|---|
| Produit (10⁻⁶ M) | Emax (%) |
| ttRA | 225 |
| Am580 | 99 |
| TTNPB (10⁻⁸ M) | 100 |
| LGD1069 | 131 |
| LGD-CB14499 | 94 |

La figure 1 en annexe montre la suractivation induite par une molécule RXR sélective en présence d'un agoniste RAR spécifique sur le modèle HRL+N.

### 5) Effet anti-AP-1 des rétinoïdes de référence sur des cellules estrogéno-dépendantes activées par le TPA - Lignée cellulaire MTLN.

L'effet transrepresseur anti-facteur AP-1 des rétinoïdes est déterminé à l'aide de la lignée MTLN, issue de cellules MCF-7 transfectées de façon stable par un vecteur p(TRE)₃-tk-Luc qui place l'expression du gène de la luciférase sous le contrôle du TPA (12-O-tétradécanoyl-phorbol-13-acétate). Le TPA active le complexe AP-1 formé des protéines de la famille des protooncogènes nucléaires (c-Jun et c-Fos). Cette lignée MTLN permet d'étudier la relation existant entre les voies estrogéniques et AP-1 (M. E. Astruc et al., Endocrinology, 1995, 136, 824-832), et de montrer la dissociation entre l'activité transactivatrice et l'effet anti-AP-1 de rétinoïdes de synthèses (J.Y. Chen et al., EMBO J., 1995, 14, 1187-1197). Les expériences se font avec des cellules MTLN activées par du TPA 10⁻⁷ M.

Les résultats rapportés dans le tableau 7 ci-dessous montrent que les 3 types de récepteurs de l'acide rétinoïque exprimés par les cellules MCF-7 (RARα,γ et RXRα) médient un effet inhibiteur de la voie AP-1. L'utilisation de l'antagoniste RARα sélectif Ro 41-5253 ne permet pas de lever totalement l'imhibition induite par le TTNPB, ce qui indique que l'activation de RARγ médie un effet anti-AP-1. L'activation par un agoniste RARα (Am580 10 nM) ou RXRα (LGD1069 et LGD-CB14499) entraîne également une inhibition de la voie AP-1. L'association d'une molécule RAR spécifique et d'une molécule RXR spécifique provoque un fort effet inhibiteur anti-AP-1, il y a effet additif entre les deux voies d'inhibition. De part son profil panagoniste à 1 µM, LGD1069 apparaît comme le composé le plus efficace testé dans ce modèle.

**Tableau 7**

| EFFET ANTI-AP-1 | | |
|---|---|---|
| Produit | Concentration (Log M) | Inhibition (%) |
| ttRA | -8 | 22 - 33 |
| | -7 | 28 - 44 |
| 9cisRA | -8 | 30 - 37 |
| Am580 | -8 | 40 - 46 |
| | -7 | 41 - 48 |
| TTNPB | -8 | 26 - 53 |
| | -7 | 29 - 54 |
| LGD1069 | -8 | 18 - 29 |
| | -7 | 48 - 62 |
| LGD-CB14499 | -7 | 17 - 30 |

L'effet des composés sur des cellules MTLN activées par le TPA 10⁻⁷ M est exprimé en pourcentage. 100% représente le niveau d'activité maximale mesuré avec le TPA qui est 5 à 6 fois supérieur à l'activité basale des cellules. Les propriétés inhibitrices des produits sont calculées après déduction de l'activité basale des cellules. Les pourcentages rapportés correspondent à une fourchette d'inhibitions déterminées à partir de plusieurs expérimentations.

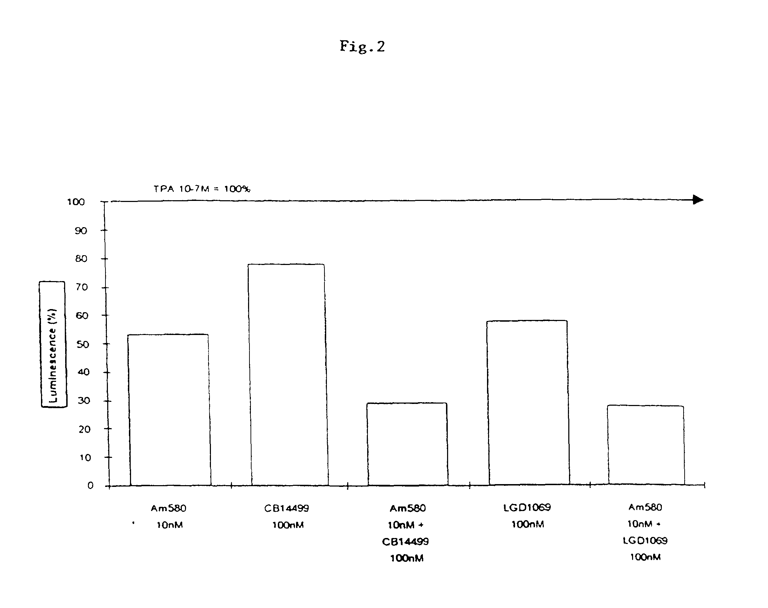

La figure 2 en annexe montre l'additivité de l'effet inhibiteur anti-AP-1 sur la lignée MTLN d'un agoniste RXR et d'un agoniste RAR sélectif.

### 6) Effet anti-estrogénique des rétinoïdes de référence sur des cellules estrogéno-dépendantes activées par l'estradiol - Lignée MELN.

Les cellules MCF-7 expriment RARα,γ et RXRα. La transfection de cellules MCF-7 par un gène estrogéno-dépendant (ERE-βGlob-Luc) a permis d'établir la lignée cellulaire MELN, utilisée pour déterminer l'activité anti-estrogénique des rétinoïdes. Ces cellules contiennent le gène de la luciférase sous le contrôle transcriptionnel du promoteur de la βGlobine et de l'élément de réponse ERE isolé du gène de la vitélogénine A2 de poulet. Les expériences sont réalisées avec des cellules MELN activées par de l'estradiol 10⁻⁹ M. Le niveau d'activité basale des cellules MELN est obtenu avec l'antiestrogène 4-OH-tamoxifène (hydroxytamoxifène) 1 µM. Ce niveau est toujours de 8 à 10 fois plus bas que l'activité maximale de référence mesurée en présence d'estradiol 10⁻⁹ M qui représente 100%. ttRA réduit la croissance cellulaire ainsi que l'expression de gènes estrogéno-dépendants (E. Demirpence et al., Cancer Res., 1994, 54, 1458-1464), ce que cette lignée MELN permet de bien vérifier.

Les molécules RAR sélectives (Am580 et TTNPB) induisent une inhibition de l'ordre de 40%. Am580 à une concentration RARα spécifique (10 nM) permet une inhibition maximale, ce qui indique que RARα médie l'effet antiestrogénique dans les cellules MCF-7. L'utilisation de l'antagoniste RARα Ro 41-5253 lève l'effet inhibiteur de Am580 mais aussi du TTNPB qui, dans ces conditions, conserve une activité RARγ et perd son activité RARα. Les composés RXR sélectifs (LGD1069 et LGD-CB14499) sont inactifs. L'activation de RXR n'est donc pas impliquée dans l'inhibition de la voie estrogénique par les rétinoïdes dans les cellules MCF-7.

Ces résultats sont rapportés dans le tableau 8 ci-dessous, ils sont parfaitement corrélés aux expériences de prolifération cellulaire estrogéno-induites. L'association d'un agoniste RAR spécifique et d'un agoniste RXR spécifique ne permet pas d'observer un effet inhibiteur supérieur à celui exercé par l'agoniste RAR seul.

**Tableau 8**

| EFFET ANTI- ESTROGENIQUE | | |
|---|---|---|
| Produit | Concentration (Log M) | Inhibition (%) |
| ttRA | -7 | 35 |
| 9-cis RA | -7 | 35 |
| Am580 | -8 | 36-51 |
| TTNPB | -8 | 30-41 |
| LGD1069 | -8 -7 | 0 0 |
| LGD-CB14499 | -7 -6 | 0 0 |

L'effet des produits testés sur des cellules activées par l'estradiol 10⁻⁹ M est exprimé en pourcentage, le niveau d'activité basale étant retranché.

### 6) Conclusion.

Les molécules de référence ont permis de montrer l'efficacité et la complémentarité des différents modèles utilisés et conduisent aux conclusions suivantes :
- les constructions chimériques GAL-RAR et RAR-ERcassette permettent de déterminer le profil RAR agoniste de molécules, mais aussi une activité RXR agoniste avec RAR-ERcassette,
- la lignée HRLN traduit principalement l'activité d'un composé médiée par RARα endogène,
- la lignée HRL+N met également en évidence une activité RXR agoniste des rétinoïdes,
- l'effet anti-estrogénique des rétinoïdes (prolifération cellulaire estrogéno-induite et lignée MELN) est médiée par RARα,
- l'effet anti-AP-1 est médié dans les cellules MCF-7 (lignée MTLN) par RARα, RARγ et RXRα, et un effet additif existe entre les voies RAR et RXR.

### II - Activité des composés de l'invention.

### 1) Spécificité des composés de l'invention.

L'étude de la sélectivité des composés a été réalisée avec le modèle de récepteurs chimériques RAR-ERcassette. Tous les composés testés sont inactifs ou faiblement transactivateurs avec RARα. Les produits comportant un tétrazole sont inactifs (CB02981) ou faiblement actifs (CB23804, CB99811 et CB94083) sur les trois types de récepteurs RAR et ne sont donc pas RAR agonistes. Le même résultat est observé pour les molécules dont le cycle comporte 6 atomes de carbone (CB66049 et CB80830).

Le tableau 9 ci-dessous rapporte ces résultats, où 100% représente la transactivation mesurée pour chaque type de récepteur avec TTNPB 10⁻⁸ M. Les composés ont été testés à la concentration de 1 µM. Pour une même ligne du tableau, le composé cis (Z) représente la structure isomère du composé trans (E).

**Tableau 9**

| RAR-ERcassettes | | | | | | | |
|---|---|---|---|---|---|---|---|
| Composé trans (E) | RARα (%) | RARβ (%) | RARγ (%) | Composé cis (Z) | RARα (%) | RARβ (%) | RARγ (%) |
| CB23804 | 4 | 28 | 19 | CB02981 | 4 | 7 | 0 |
| CB78937 | 18 | 65 | 93 | CB27871 | 29 | 36 | 67 |
| CB40341 | 14 | 75 | 79 | CB75403 | 12 | 65 | 68 |
| CB66049 | 0 | 0 | 8 | CB80830 | 2 | 0 | 1 |
| CB99811 | 3 | 11 | 14 | CB94083 | 4 | 14 | 18 |
| CB52809 | 5 | 0 | 13 | -- | | | |
| CB93128 | 16 | 148 | 110 | -- | | | |

Comme l'indique le tableau 9 et le tableau 10 ci-dessous, les composés ayant un cycle de 5 atomes de carbone et un carboxyle présentent un intérêt certain. CB78937, CB40341 et CB75403 induisent une transactivation médiée par RARβ et par RARγ significative à la concentration de 1 µM et sont inactifs sur RARα. Ces molécules comportent une spécificité RARβ,γ. De plus, CB75403 induit le même niveau de transactivation par l'intermédiaire de RARβ à 0,1 µM et à 1 µM, mais aussi à 3µM, ce qui indique que cette molécule présente une bonne affinité pour RARβ mais reste un agoniste partiel. CB93128 est également un agoniste RARβ,γ sélectif. Ce composé est capable d'activer complètement RARγ à 1 µM et suractive RARβ en comparaison à l'activation provoquée par le TTNPB. Cette suractivation traduit une activité RXR.

**Tableau 10**

| RAR-ERcassettes | | | | |
|---|---|---|---|---|
| Produit | Concentration (Log M) | RARα (%) | RARβ (%) | RARγ (%) |
| TTNPB | -8 | 100 | 100 | 100 |
| | -9 | 88 | 6 | 9 |
| Am580 | -8 | 113 | 70 | 45 |
| | -7 | 115 | 99 | 93 |
| | -6 | 106 | 89 | 100 |
| CB75403 | -7 | 3 | 68 | 40 |
| | -6 | 12 | 65 | 68 |
| CB93128 | -7 | 8 | 80 | 65 |
| | -6 | 16 | 148 | 110 |

### 2) Effet des composés de l'invention sur la prolifération estrogéno-induite - Lignées cellulaires MCF-7 et T-47D.

L'effet des rétinoïdes sur la croissance des cellules MCF-7 et T-47D est évaluée aprés 7 jours de culture en présence d'estradiol 10⁻⁹ M par dosage de l'ADN cellulaire. La molécule CB02981, qui est inactive en transactivation RAR, n'exerce aucun effet sur la prolifération estrogéno-induite des cellules MCF-7 et T-47D.

### 3) Activité transactivatrice des composés de l'invention médiée par les récepteurs de l'acide rétinoïque - Lignées cellulaires HRLN et HRL+N.

### a) Lignée HRLN,

Les résultats de transactivation obtenus avec la lignée HRLN sont corrélés aux résultats de spécificité (modèle RAR-ERcassette). Aucune molécule à 1 µM ne permet une transactivation comparable à celle induite par la référence TTNPB. Les composés comportant un tétrazole ou un cycle à 6 atomes de carbone sont peu (CB23804) ou pas actifs (CB02981, CB66049, CB80830), ce qui est en accord avec leur inactivité en modèle RAR-ERcassette. Les produits carboxylés (CB78937, CB40341, CB27871 et CB93128) induisent une transactivation partielle qui correspond à leur activité RARβ,γ. Le tableau 11 rapporte les résultats obtenus où 100% correspond à la transactivation induite par le TTNPB 10⁻⁸ M.

**Tableau 11**

| TRANSACTIVATION HRLN | | | |
|---|---|---|---|
| Produit trans (E) | Transactivation à 1 µM (%) | Produit cis (Z) | Transactivation à 1 µM (%) |
| CB23804 | 15 | CB02981 | 0 |
| CB78937 | 25 | CB27871 | 27 |
| CB40341 | 30 | CB75403 | non déterminé |
| CB66049 | 0 | CB80830 | 4 |
| CB99811 | non déterminé | CB94083 | non déterminé |
| CB52809 | 0 | -- | |
| CB93128 | 51 | -- | |

### 2) Lignée HRL+N.

L'utilisation du modèle HRL+N confirme les résultats obtenus avec les cellules HRLN. Comme indiqué dans le tableau 12 ci-dessous, CB02981 ne provoque pas de transactivation médiée par RAR et RXR. En association à un agoniste RAR sélectif, les composés testés n'induisent pas de suractivation à l'exception de CB93128. Seul ce produit semble bien présenter une activité RXR (suractivation observée avec RARβ-ERcassette).

**Tableau 12**

| TRANSACTIVATION HRL+N | |
|---|---|
| Produit | Transactivation à 1 µM (%) |
| TTNPB | 100 |
| CB02981 | 0 |
| CB75403 | 18 |
| CB94083 | 13 |

### 4) Effet anti-AP-1 des composés de l'invention sur des cellules estrogéno-dépendantes activées par TPA-Lignée cellulaire MTLN.

L'influence des composés CB02981 et CB75403 à la concentration de 1 µM sur la voie AP-1 dans les cellules MCF-7 a été déterminée avec le modèle MTLN. L'utilisation des molécules de référence a montré que tous les récepteurs de l'acide rétinoïque exprimés par les cellules MCF-7 (RARα,γ et RXRα) médient un effet anti-AP-1. CB75403 provoque une inhibition de l'ordre de 30%, qui correspond à l'activité RARγ constatée pour cette molécule. CB02981 présente une activité très intéressante. Ce composé, incapable d'induire une transactivation médiée par RAR, exerce un effet inhibiteur anti-AP-1 de l'ordre de 20% sur les cellules MTLN et présente un profil dissociant. De plus, en association à un agoniste RXR, une additivité des effets inhibiteurs des deux molécules est observée.

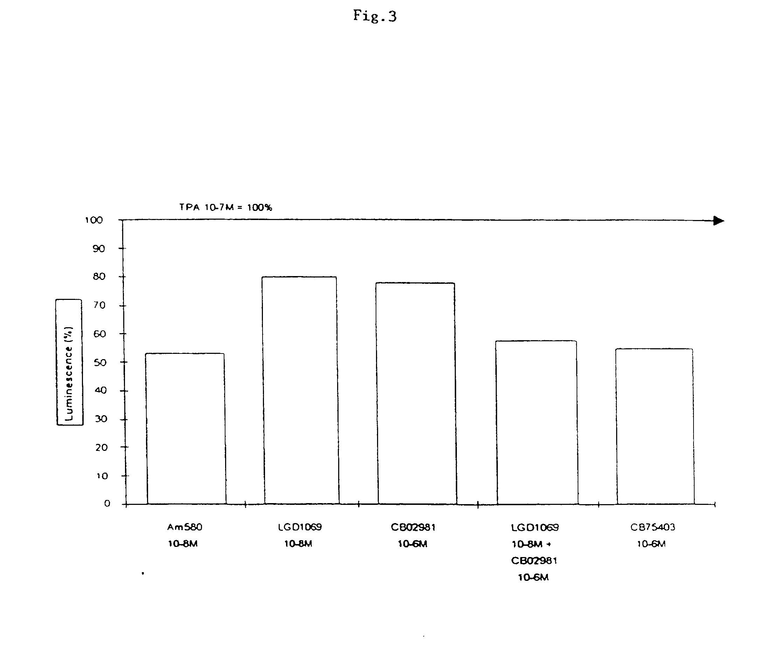

La figure 3 représente l'effet des composés CB02981 et CB75403 sur la lignée MTLN, et additivité des effets de CB02981 et d'un agoniste RXR sélectif.

### 5) Effet anti-estrogénique des composés de l'invention sur des cellules estrogéno-dépendantes activées par l'estradiol - Lignée MELN.

Comme rapporté dans le tableau 13 ci-dessous, l'influence des composés à 1 µM sur l'expression d'un gène contrôlé par un ERE a été déterminée avec le modèle MELN. Aucun composé testé n'exerce d'effet transrepresseur, ce qui correspond à leur incapacité à médier une transactivation par l'intermédiaire de RARα.

**Tableau 13**

| EFFET ANTIESTROGENIQUE | | | |
|---|---|---|---|
| Produit trans (E) (10⁻⁶ M) | Inhibition (%) | Produit cis (Z) (10⁻⁶ M) | Inhibition (%) |
| CB23804 | 0 | CB02981 | 0 |
| CB78937 | 4 | CB27871 | non déterminé |
| CB40341 | non déterminé | CB75403 | 0 |
| CB66049 | non déterminé | CB80830 | 2 |
| CB52809 | 0 | -- | |

### 6) Conclusion.

Les travaux rapportés ci-dessus montrent les propriétés interessantes des composés de l'invention. Parmi ceux-ci, les molécules comportant un carboxyle et un cycle à 5 atomes de carbone sont RARβ,γ spécifiques. La contrainte conformationnelle présente dans ces structures semble défavorable à une activité RARα et oriente vers une sélectivité RARβ,γ (CB40341, CB75403, CB78937). La présence du cycle entraine une perte d'activité transcriptionnelle RXR. La substitution du carboxyle par un radical tétrazole provoque une perte d'activité RAR sur la transcription. Cependant, malgré une inactivité transcriptionnelle, CB02981 est un composé dissociant et exerce une inhibition de la voie AP-1. L'association de CB02981 et d'un agoniste RXR peut s'avérer efficace pour potentialiser un effet anti-AP-1. CB93128 présente un profil original en étant capable d'activer RARβ, RARγ et RXR.

D'autres caractéristiques et avantages de l'invention apparaîtront à la lecture des exemples qui suivent concernant la préparation et l'analyse de composés de référence et de dérivés de l'invention, étant entendu que ces exemples ne sauraient être interprétés comme tendant à réduire la portée des revendications.

### Exemple 1 : Préparation de l'acide (E) 3-[-2-(5,6,7,8-tétrahydro-5,5,8,8-tétraméthyl)naphtalényl méthylène]-dihydro-benzo[b]furane-5-carboxylique(CB93128) :

### 1) 3-Bromophénoxyacétate de méthyle.

A une solution d'hydrure de sodium 50 % en dispersion dans l'huile minérale (5 g, 125 mmol) dans 50 ml de THF anhydre, on ajoute à température ambiante une solution du 3-bromophénol (17,3 g, 100 mmol) dans 30 ml de THF. On laisse agiter à température ambiante pendant 30 min avant d'ajouter successivement une solution du bromoacétate de méthyle (10,4 ml, 110 mmol) dans 33 ml de THF anhydre et de l'iodure de sodium (3,75 g, 25 mmol). Le milieu réactionnel est agité 30 min à température ambiante puis traité par 30 ml d'eau à 0 °C. Le produit brut est purifié par chromatographie éclair sur silice (éluant éther de pétrole : éther = 100 : 10). On obtient 19,66 g du 3-bromophénoxyacétate de méthyle (Rdt = 80 %).

F(°C) = 39

RMN¹H 200MHz (CDCl₃) :3,75 (s, 3H, MeO); 4,57(s, 2H, CH₂-O) ; 6,75-6,85 (m, 1H, ArH); 7,00-7,25 (m, 3H, ArH).

### 2) Acide 3-bromophénoxyacétique.

A une solution du 3-bromophénoxyacétate de méthyle (19,66 g, 80 mmol) en solution dans 100 ml d'un mélange eau-THF (3 : 1), on ajoute a 0 °C de l'hydroxyde de lithium monohydraté (8,41 g, 0,2 mol). L'agitation est poursuivie 15 min à 0 °C puis on acidifie par une solution aqueuse d'acide chlorhydrique 3N. On extrait à l'éther, sèche sur MgSO₄, filtre et évapore. On obtient 18,21 g (Rdt = 98%) de l'acide 3-bromophénoxyacétique.

F(°C) = 108.

RMN¹H 200MHz (CDCl₃) : 4,70 (s, 2H, CH₂-O) ; 6,80-6,90 (1H, ArH); 7,05-7,10 (m, 1H, ArH); 7,15-7,20 (m, 2H, ArH); 9,40-9,05 (m, 1H, H mobile).

### 3) 6-Bromo-3-coumarone.

A de l'acide 3-bromophénoxyacétique (5,77 g, 25 mmol), on ajoute à température ambiante du chlorure de thionyle (5 ml, 70 mmol). Le milieu réactionnel est porté au reflux pendant 2 heures. Après retour à température ambiante on évapore et le chlorure d'acide est utilisé tel que. Le chlorure du 3-bromophénoxyacétyle obtenu précédemment mis en solution dans le dichlorométhane (150 ml) est ajouté à une solution du chlorure d'aluminium (6,7 g, 50 mmol) dans 50 ml du dichlorométhane. Le milieu réactionnel est agité à température ambiante pendant 30 min puis versé sur un mélange eau-glace volume à volume (400 ml). On extrait au dichlorométhane, sèche sur MgSO₄, filtre et évapore. Le produit brut est purifié par chromatographie éclair sur silice (éluant éther de pétrole : éther = 100 : 10). On obtient 0,96 g de 6-bromo-3-coumarone (Rdt = 18 %) de formule :

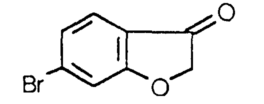

F (°C) = 124.

RMN¹H 200MHz (CDCl₃) : 4,65 (s, 2H, CH₂-O) ; 7,10-7,25 (m, 1H, ArH); 7,45-7,60 (m, 2H, ArH).

### 4) (E) 5-Bromo-[2-(5,6,7,8-tétrahydro-5,5,8,8-tétraméthyl)naphtalénylméthylènel-dihydrobenzo[b]furane.

A une solution de bromure de (5,6,7,8-tétrahydro-5,5,8,8-tétraméthyl-2-naphtalényl)-6-méthyltriphénylphosphonium (6 g, 11,06 mmol) dans 16 ml de THF, on ajoute à - 70 °C une solution de tBuOK 1 M dans le THF (12,2 ml, 12,2 mmol). On poursuit l'agitation à -70 °C pendant 1 h avant d'ajouter la 6-bromo-3-coumarone (1,18 g, 5,53 mmol) dans 10 ml du THF à cette température. Le milieu réactionnel est porté à température ambiante et on poursuit l'agitation pendant 2,5 h. On hydrolyse ensuite à 0 °C par une solution de HCl 3N (20 ml). Après retour à température ambiante, on extrait à l'éther, sèche sur MgSO₄, filtre et évapore les solvants. Le produit brut est purifié par chromatographie éclair sur silice (éluant : éther de pétrole). On obtient 180 mg du (E) 5-bromo-[2-(5,6,7,8-tétrahydro-5,5,8,8-tétraméthyl)naphtalényl méthylène]-dihydro-benzo[b]furane (Rdt global = 8 %) de formule :

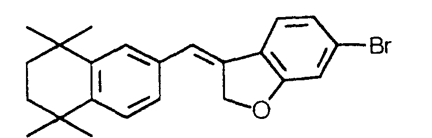

RMN¹H 200MHz (CDCl₃) : 1,25 (s, 6H); 1,30 (s, 6H); 1,65 (s,4H); 4,95 (s, 2H); 6,95-7,10 (m, 2H); 7,20 (s, 1H) ; 7,30 (s, 2H) ; 7,35 (s, 1H) ; 7,60 (s, 1H).

MS (m/z, % intensité) : 398 (36%); 397 (14); 384 (44); 383 (86); 381 (81); 211(97); 209(100).

### 5) (E) 5-Cyano-[2-(5,6,7,8-tétrahydro-5,5,8,8-tétraméthyl)naphtalénylméthylène]-dihydrobenzo[b]furane.

A une solution de (E) 5-bromo-[2-(5,6,7,8-tétrahydro-5,5,8,8-tétraméthyl)naphtalénylméthylène]-dihydro-benzo[b]furane (180 mg, 0,45 mmol) dans 1,7 ml de DMF anhydre, on ajoute à température ambiante du cyanure de cuivre (48 mg, 5,81 mmol). Le milieu réactionnel est porté au reflux pendant 24 h. Après retour à température ambiante, le milieu réactionnel est dilué avec de l'éther (50 ml) et filtré sur célite. On lave la phase organique avec une solution aqueuse saturée en NaHCO₃ (3 x 20 ml) puis sèche sur MgSO₄, filtre et évapore. Le produit brut est purifié par chromatographie éclair sur silice (éluant éther de pétrole : éther = 100 : 5). On obtient 50 mg du (E) 5-cyano-[2-(5,6,7,8-tétrahydro-5,5,8,8-tétraméthyl) naphtalénylméthylène]-dihydro-benzo[b]furane (Rdt = 32 %) de formule :

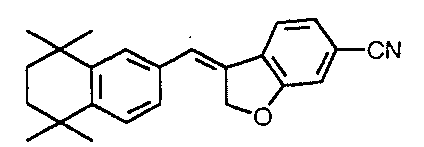

RMN¹H 200MHz (CDCl₃) : 1,20 et 1, 25 (2s, 12H, 5,5,8,8-Me); 1,65 (s, 4H, 6,7-CH₂) ; 3,95 (s, 2H, CH₂-O) ; 6,90-7,00 (m, 2H); 7,40-7,45 (m, 2H); 7,50 (s, 1H); 7,52 (s, 1H) ; 7,75 (s, 1H).

### 6) Acide (E) 3-[2-(5,6,7,8-tétrahydro-5,5,8,8-tétraméthyl)naphtalényl]méthylène]-dihydro-benzo[b]furane-5-carboxylique (CB93128).

Une solution du (E) 5-cyano-[2-(5,6,7,8-tétrahydro-5,5,8,8-tétraméthyl)naphtalénylméthylène]-dihydro-benzo[b]furane (50 mg, 0,14 mmol) dans la potasse (0,1 g 1,7 mmol) hydroéthanolique (H₂O 0,5 ml et EtOH 3 ml) est chauffée à reflux sous agitation magnétique pendant 15 h. On évapore l'éthanol à l'évaporateur rotatif, reprend à l'eau (10 ml), acidifie par HCl 3N, extrait à l'éther éthylique (3 x 50ml), sèche la phase éthérée sur MgSO₄, filtre et évapore. Le produit brut est purifié par HPLC préparative (éluant: MeOH : H₂O = 90 : 10 + 0,1% de TFA), on obtient 12,5 mg d'acide (E) 3-[2-(5,6,7,8-tétrahydro-5,5,8,8-tétraméthyl)naphtalényl-méthylène]-dihydro-benzo[b]furane-5-carboxylique (CB93128) (Rdt = 25 %) de formule :

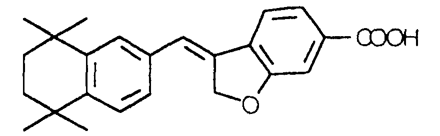

F (°C) = 178-181.

RMN¹H 200MHz (CDCl₃) : 1,03 et 1,24 (2s, 12H, 5,5,8,8-Me); 1,65 (s, 4H, 6,7-CH₂-) ; 3,97 (s, 2H); 6,99 (d, 1H, *J* 8 Hz) ; 7,19 (s, 2H); 7,45-7,55 (m, 2H); 7,96 (d, 1H, *J* 8 Hz); 8,21 (s, 1H); 8,16-10,2 (m, 1H, H mobile).

MS (m/z, % intensité) : 362 (56 %); 348 (53); 347 (94); 175 (100).

MSHR EI 70 eV : Mₜᵣ = 362,1881 pour C₂₄H₂₆O₃ Mₜₕ = 362,1882.

HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, acide (CB93128) tr = 3,81 min 99,6%.

### Exemple 2 : Préparation des acides (Z) et (E) 1-[2-(5,6,7,8-tétrahydro-5,5,8,8-tétraméthyl)naphtalényl-méthylène]-1,2,3,4-tétrahydronaphtalène-6-carboxyliques (CB80830 et CB66049).

### 1) (Z) et (E) 6-Cyano-1-[2-(5,6,7,8-tétrahydro-5,5,8,8-tétraméthyl)naphtalénylméthylène]-1,2,3,4-tétrahydronaphtalènes.

A une solution de bromure de (5,6,7,8-tétrahydro-5,5,8,8-tétraméthyl-2-naphtalényl)-6-méthyltriphénylphosphonium (3 g, 5,52 mmol) dans 8 ml de THF, on ajoute à - 70 °C une solution molaire de tBuOK dans le THF (6,1 ml, 6,1 mmol). On poursuit l'agitation à - 70 °C pendant 1 h avant d'ajouter la 6-cyano-1-tétralone (0,78 g, 4,6 mmol) dans 7 ml du THF à cette température. On remonte à température ambiante puis le milieu réactionnel est au reflux pendant 5 h. Après retour à temmpérature ambiante, le milieu réactionnel est versé sur 100 ml d'un mélange eau-glace volume à volume. On extrait à l'éther, sèche sur MgSO₄, filtre et évapore les solvants. Le produit brut est purifié par chromatographie éclair sur silice (éluant : éther de pétrole : éther éthylique = 100 : 5). On obtient 600 mg du mélange des (Z) et (E) 6-cyano-1-[2-(5,6,7,8-tétrahydro-5,5,8,8-tétraméthyl)naphtalénylméthylène]-1,2,3,4-tétrahydronaphtalènes (Pdt = 37%). La précipitation de ce mélange dans le pentane permet d'isoler 200 mg de l'isomère (E) pur et 330 mg du mélange (E) et (Z) enrichi en (Z).

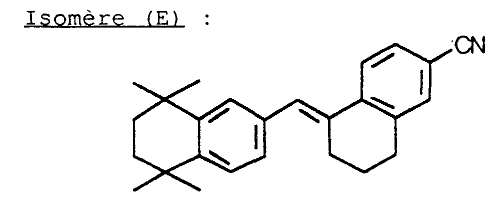

F (°C) = 144.

RMN¹H 200MHz (CDCl₃) : 1,29 (s, 12H, 5,5,8,8-Me) ; 1, 69 (s, 4H, 6,7-CH₂) ; 1,75-1,90 (m, 2H); 2,75-2,90 (m, 4H); 7,06 (s, 1H, H vinylique); 7,15 (dd, 1H, ArH, *J* 2Hz, *J* 8 Hz); 7,28 (s, 2H); 7,32-7,45 (m, 2H, ArH) ; 7,72 (d, 1H, ArH, *J* 8 Hz).

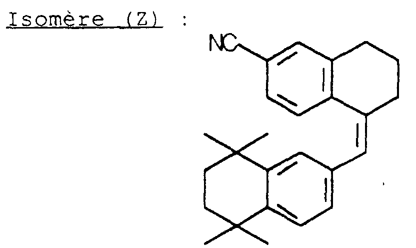

RMN¹H 200MHz (CDCl₃) : 1,08 et 1,24 (2s, 12H, 5,5,8,8-Me); 1,62 (s, 4H, 6,7-CH₂) ; 1,95-2,10 (m, 2H); 2,45-2,55 (m, 4H); 6,50 (s, 1H, H vinylique) ; 6,92 (d, 1H, ArH, *J* 8 Hz); 7,05-7,15 (m, 1H, ArH); 7,27 (m, 1H, ArH); 7,32 (dd, 1H, ArH, *J* 2Hz, *J* 8 Hz); 7,45 (m, 1H ArH).

### 2) Acides (Z) et (E) 1-[2-(5,6,7,8-tétrahydro-5,5,8,8-tétraméthyl)naphtalénylméthylène]-1,2,3,4-tétrahydronaphtalène-6-carboxyliques (CB80830 et CB66049)

Au mélange des (Z) et (E) 6-cyano-1-[2-(5,6,7,8-tétrahydro-5,5,8,8-tétraméthyl-naphtalénylméthylène]-1,2,3,4-tétrahydronaphtalènes (330 mg, 0,93 mmol) en solution dans 3 ml de THF, 1 ml d'eau et 2 ml d'éthanol, on ajoute de la potasse (1,56 g, 27,9 mmol) à température ambiante. Le milieu réactionnel est porté au reflux sous agitation magnétique pendant 36 h. Aprés retour à température ambiante on évapore, reprend à l'eau (10 ml), acidifie par HCl 3N, extrait à l'éther éthylique (3 x 50ml), sèche la phase éthérée sur MgSO₄, filtre et évapore. Le produit brut est purifié par HPLC préparative (éluant MeOH : H₂O = 90 : 10 + 0,1% de TFA) , on obtient 60 mg d'acide (Z) 1-[2-[(5,6,7,8-tétrahydro-5,5,8,8-tétraméthyl)naphtalénylméthylène]-1,2,3,4-tétrahydro-naphtalène-6-carboxylique (CB80830) (Rdt = 17 %) et 100 mg d'acide (E) 1-[2-(5,6,7,8-tétrahydro-5,5,8,8-tétraméthyl)naphtalénylméthylène)-1,2,3,4-tétrahydro-naphtalène-6-carboxylique (Rdt = 29 %) (CB66049).

### a) Acide (Z) 1-[2-(5,6,7,8-tétrahydro-5,5,8,8-tétraméthyl)naphtalénylméthylène]-1,2,3,4-tétrahydronaphtalène-6-carboxylique (CB80830) de formule :

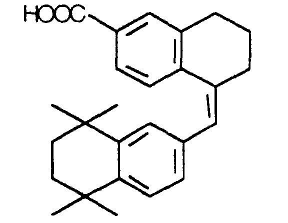

F (°C) = 218-220.

IR (cm⁻¹) :3100; 2954; 1686; 1604; 1430; 1290

RMN¹H 200MHz (CDCl₃) : 1,09 et 1,24 (2s, 12 H, 5,5,8,8-Me); 1,62 (s, 4H, 6,7-Me); 1,68-2,15 (m, 2H); 2,51(t, 2H, *J* 6,6 Hz); 2,91 (t, 2H, *J* 6,6 Hz); 6,49 (s, 1H); 6,93 (dd, 1H, *J* 1,3 Hz *J* 8 Hz); 7,10-7,16 (m, 2H); 7,34 (d, 1H, *J* 8Hz) ; 7,53 (d, 1H, *J* 8Hz); 7,84 (m, 1H).

MS EI, 70 eV (m/z, % intensité) : 374 (100, M⁺ %); 359 (67) ; 208 (35); 168 (39).

MSHR EI 70 eV : Mₜᵣ = 374,2249 pour C₂₆H₃₀O₂ Mₜₕ = 374,2246.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB80830) tr = 4,71 min 96,9%.

### b) Acide (E) 1-[2-(5,6,7,8-tétrahydro-5,5,8,8-tétraméthyl)naphtalénylméthylène]-1,2,3,4-tétrahydronaphtalène-6-carboxylique (CB66049) de formule:

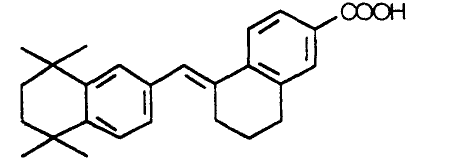

F (°C) = 234.

IR (cm⁻¹) : 3300; 2960; 1680; 1602;1430; 1434; 1294; 1186.

RMN¹H 200MHz (CDCl₃) : 1,28 et 1,29 (2s, 12 H, 5,5,8,8-Me); 1,69 (s, 4H, 6,7-Me); 1,81-1,88 (m, 2H); 2,82-2,90 (m, 4H); 7, 11 (s, 1H) ; 7,14-7,19 (m, 1H) ; 7,28-7,32 (m, 2H) ; 7,75 (d, 1H, *J,* 8Hz) ; 7,85-7,91 (m, 2H).

MS EI, 70 eV (m/z, % intensité) : 374 (100, M %) ; 359 (67) .

MSHR EI 70 eV : Mₜᵣ = 374,2257 pour C₂₆H₃₀O₂ Mₜₕ = 374,2246.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB66049) tr = 6,26 min 96,9%.

### Exemple 3 : Préparation du (E) 5-[1-[2-(5,6,7,8-tétrahydro-5,5,8,8-tétraméthyl)naphtalénylméthyléne]-1,2,3,4-tétrahydro-naphtalènyl]-1H-tétrazole (CB44858).

A une solution de (E) 6-cyano-1-[2-(5,6,7,8-tétrahydro-5,5,8,8-tétramèthyl)naphtalénylméthylène]-1,2,3,4-tétrahydronaphtalène (180 mg, 0,50 mmol) dans du toluène anhydre (1 ml), on additionne sucessivement de l'oxyde de dibutylétain (15 mg, 0,06 mmol) et de l'azoture de triméthylsilyle (0,133 ml, 1 mmol). Le milieu réactionnel est chauffé 18 h à reflux (110°C) sous atmosphère d'argon et agitation magnétique. Après retour à température ambiante, on évapore le toluène et purifie par chromatographie éclair sur silice (éluant MeOH : CH₂Cl₂ = 10 : 90) suivie d'une précipitation dans le chloroforme on obtient 34,8 mg d'une poudre blanche, le 5-[1-[2-(5,6,7,8-tétrahydro-5,5,8,8-tétraméthyl)naphtalénylméthylène]-1,2,3,4-tétrahydronaphtalènyl]-*1H*-tétrazole (CB44858) (Rdt = 17 %) de formule :

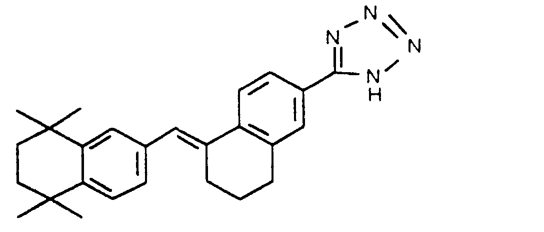

F (°C) =233-234.

IR (cm⁻¹) : 3480; 2926; 1606; 1490; 1450.

RMN¹H 200MHz (DMSO, D₆) : 1,24 et 1, 25 (2s, 12H, 5,5,8,8-Me); 1,64 (s, 4H, 6,7-CH₂) ; 1,74-1,80 (m, 2H) ; 2,77-2,89 (m ,4H); 7,16-7,21 (m, 2H); 7,30-7,35 (m, 2H); 7,82-7,86 (m, 2H); 7,97-8,00 (m, 1H).

MS IC (isobutane) (m/z, % intensité) : 399 (100, MH⁺).

MSHR IC (isobutane) : Mₜᵣ = 399,2592 pour C₂₆H₃₀N₄ Mₜₕ = 399,2549.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, tétrazole (CB44858) tr = 4,66 min 98,0%.

### Exemple 4 : préparation des acides (Z) et (E) 1-[2-(5,6,7,8-tétrahydro-3,5,5,8,8-pentaméthyl) naphtalénylméthylène]-1,2,3,4-tétrahydronaphtaléne-6-carboxyliques (CB53261 et CB95970)

### 1) (Z) et (E) 6-cyano-1-[2-(5,6,7,8-tétrahydro-3,5,5,8,8-pentaméthyl)naphtalénylméthylène]-1,2,3,4-tétrahydronaphtalènes.

A une solution de bromure de (5,6,7,8-tétrahydro-3,5,5,8,8-pentaméthyl-2-naphtalényl)-6-méthyltriphénylphosphonium (6,69 g, 12 mmol) dans 17,3 ml de THF, on ajoute à - 70 °C une solution de tBuOK 1M dans le THF (13,2 ml, 13,2 mmol). On poursuit l'agitation à-70 °C pendant 1 h avant d'ajouter la 6-cyano-1-tétralone (1,03 g, 6,0 mmol) dans 9 ml du THF à cette température. On remonte à température ambiante puis le milieu réactionnel est au reflux pendant 15 h. Après retour à temmpérature ambiante, le milieu réactionnel est versé sur 200 ml d'un mélange eau-glace volume à volume. On extrait à l'éther, sèche sur MgSO₄, filtre et évapore les solvants. Le produit brut est purifié par chromatographie éclair sur silice (éluant éther de pétrole : éther éthylique = 100 : 3). On obtient 0,91 g du mélange des (Z) et (E) 6-cyano-1-[2-(5,6,7,8-tétrahydro-3,5,5,8,8-pentaméthyl)naphtalénylméthylène]-1,2,3,4-tétrahydronaphtalènes (Rdt = 41 %) ((Z) : (E) = 1 : 2).

### a) (Z) 6-Cyano-1-[2-(5,6,7,8-tétrahydro-3,5,5,8,8-pentaméthyl)naphtalénylméthylène]-1,2,3,4-tétrahydronaphtalène de formule :

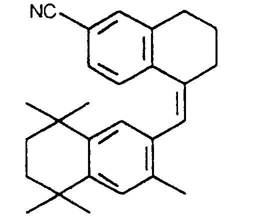

RMN¹H 200MHz (CDCl₃) : 0,97 et 1,25 (2s, 12H, 5,5,8,8-Me) ; 1,67 (s, 4H, 6,7-CH₂) ; 1,87-1,95 (m, 2H); 1,98 (s, 3H, ArMe); 2,52-2,58 (m, 2H); 2,62-2,69 (m, 2H); 6,53 (s, 1H, H vinylique); 7,00 (m, 2H, ArH); 7,45 (m, 1H, ArH);7,70 (d, 2H, ArH, *J* 8Hz).

### b) (E) 6-cyano-1-[2-(5,6,7,8-tétrahydro 3,5,5,8,8-pentaméthyl)naphtalénylméthylène]-1,2,3,4-tétrahydronaphtalène de formule :

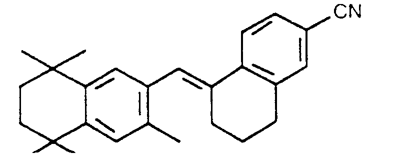

RMN¹H 200MHz (CDCl₃) : 1,26 et 1,28 (2s, 12H, 5,5,8,8-Me); 1,56 (s, 4H, 6,7-CH₂) ; 1,75-1,84 (m, 2H); 2,01 (s, 3H, ArMe) ; 2,82-2,92 (m, 4H) ; 6,86 (s, 1H, H vinylique); 7,01 (m, 1H, ArH); 7,06-7,14 (m, 2H, ArH); 7,37-7,39 (m, 2H, ArH).

### 2) Acides (Z) et (E) 1-[2-(5,6,7,8-tétrahydro-3,5,5,8,8-pentaméthyl)naphtalénylméthylène]-1,2,3,4-tétrahydronaphtalène-6-carboxyliques (CB53261 et CB95970).

Au mélange des (Z) et (E) 6-cyano-1-[2-(5,6,7,8-tétrahydro-3,5,5,8,8-pentaméthyl)naphtalénylméthylène]-1,2,3,4-tétrahydronaphtalènes (400 mg, 1,08 mmol) en solution dans 3 ml de THF, 1,2 ml d'eau et 2 ml d'éthanol, on ajoute de la potasse (1,81 g, 32,4 mmol) à température ambiante. Le milieu réactionnel est porté au reflux sous agitation magnétique pendant 12 h. Aprés retour à température ambiante on évapore, reprend à l'eau (10 ml), acidifie par HCl 3N, extrait à l'éther éthylique (3 x 50ml), sèche la phase éthérée sur MgSO₄, filtre et évapore. Le produit brut est purifié par HPLC préparative (éluant: MeOH : H₂O = 90 : 10 + 0,1% de TFA), on obtient 87,6 mg d'acide (Z) 1-[2-(5,6,7,8-tétrahydro-3,5,5,8,8-pentaméthyl)naphtalénylméthylène]-1,2,3,4-tétrahydronaphtalène-6-carboxylique (CB53261) (Rdt = 21 %) et 100 mg d'acide (E) 1-[2-(5,6,7,8-tétrahydro-3,5,5,8,8-pentaméthyl)naphtalénylméthylène)-1,2,3,4-tétrahydronaphtalène-6-carboxylique (CB95970) (Rdt = 30 %).

### a) Acide (Z) 1-[2-(5,6,7,8-tétrahydro-3,5,5,8,8-pentaméthyl)naphtalénylméthylène]-1,2,3,4-tétrahydronaphtalène-6-carboxylique (CB53261) de formule :

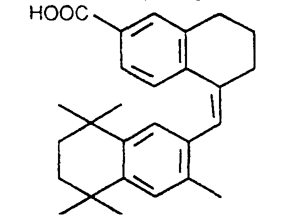

F (°C) = 207.

IR (cm⁻¹) : 2950; 1680; 1602; 1560; 1428; 1302.

RMN¹H 200MHz (CDCl₃) : 0,98 et 1,25 (2s, 12H, 5,5,8,8-Me); 1,59 (s, 4H, 6,7-CH₂) ; 1,99-2,05 (m, 2H); 2,18 (s, 3H, ArH); 2,57 (t, 2H, *J* 6Hz); 2,93 (t, 2H, *J* 6Hz); 6,51 (s, 1H); 6,92 (s, 1H); 7,00-7,05 (m, 2H) ; 7,43 (d, 1H, *J* 8 Hz); 7,81 (s, 1H).

MS EI, 70 eV (m/z, % intensité) : 388 (100, M⁺); 373 (78).

MSHR EI 70 eV : Mₜᵣ *=* 388,2416 pour C₂₇H₃₂O₂ Mₜₕ = 388,2402.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1 % de TFA, acide (CB53261) tr = 5,51 min 97,5%.

### b) Acide (E) 1-[2-(5,6,7,8-tétrahydro-3,5,5,8,8-pentaméthyl)naphtalénylméthylène]-1,2,3,4-tétrahydronaphtalène-6-carboxylique (CB95970) de formule:

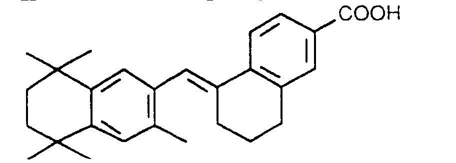

F (°C) = 226-229.

IR (cm⁻¹) : 3300; 2954; 1690; 1600; 1566; 1490; 1430; 1302; 1186.

RMN¹H 200MHz (CDCl₃) : 1,31 et 1,33 (2s, 12 H, 5,5,8,8-Me); 1,72 (s, 4H, 6,7-CH₂) ; 1,85-1,91 (m, 2H); 2,30 (s, 3H, ArMe); 2,73 (t, 2H, *J* 6Hz); 2,95 (t, 2H, *J* 6Hz); 7,17 (s, 2H); 7,21 (s, 1H); 7,80 (d, 1H, ArH, *J* 8Hz); 7,93-7,97 (m, 2H, ArH).

MS EI, 70 eV (m/z, % intensité) : 389 (35); 388 (100, M⁺) ; 373 (78).

MSHR EI 70 eV : Mₜᵣ = 388,2400 pour C₂₇H₃₂O₂ Mₜₕ = 388,2402.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB95970) tr = 6,45 min 98,7%.

### Exemple 5 : Préparation des (Z) et (E) 5-[1-[2-(5,6,7,8-tétrahydro-3,5,5,8,8-pentaméthyl)naphtalényl-méthylène]-1,2,3,4-tétrahydronaphtalènyl]-1H-tétrazoles (CB02305 et CB58248).

A une solution des (Z) et (E) 6-cyano-1-[2-(5,6,7,8-tétrahydro-3,5,5,8,8-pentaméthyl)naphtalényl-méthylène]-1,2,3,4-tétrahydronaphtalènes (Z / E = 1 / 2) (490 mg, 1,32 mmol) dans du toluène anhydre (2,6 ml), on additionne sucessivement de l'oxyde de dibutylétain (39 mg, 0,16 mmol) et de l'azoture de triméthylsilyle (0,350 ml, 2,64 mmol). Le milieu réactionnel est chauffé 15 h à reflux (110°C) sous atmosphère d'argon et agitation magnétique. Après retour à température ambiante on évapore le toluène et le produit brut est purifié par HPLC préparative (éluant MeOH : H₂O = 88 : 12 + 0,1% de TFA). On obtient 96 mg d'un solide, le (Z) 5-[1-[2-(5,6,7,8tétrahydro-3,5,5,8,8-pentaméthyl)naphtalénylméthylène]1,2,3,4-tétrahydronaphtalènyl)-*1H*-tétrazole (CB02305) (Rdt = 18 %) et 187 mg d'un solide, le (E) 5-[1-[2-(5,6,7,8tétrahydro-3,5,5,8,8-pentaméthyl)naphtalénylméthylène]1,2,3,4-tétrahydronaphtalène]-*1H*-tétrazole (CB58248) (Rdt = 34 %).

### 1) (Z) 5-[1-[2-(5,6,7,8-tétrahydro-3,5,5,8,8pentaméthyl)-2-naphtalénylméthylène]-1,2,3,4tétrahydronaphtalènyl]-1H-tétrazole (CB02305) de formule :

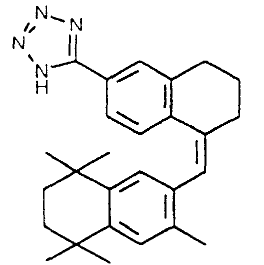

F (°C) = 148.

IR (cm⁻¹) : 2994; 1686; 1600; 1458; 1264.

RMN¹H 200MHz (CDCl₃) :0,94 et 1,22 (2s, 12H, 5,5,8,8-Me); 1,54 (m, 4H, 6,7-CH₂) ; 1,67-2,03 (m, 2H); 2,18 (s, 3H, ArMe); 2,53-2,59 (m, 2H); 2,89-2,95 (m, 2H); 6,50 (s, 1H); 6,94 (s, 1H); 7,09 (s, 1H); 7,13-7,17 (m, 2H); 7,42 (d, 1H, *J* 8, 7Hz).

MS EI, 70 eV (m/z, % intensité) : 412 (100, M⁺) ; 384 (83).

MSHR EI 70 eV : Mₜᵣ = 412,2637 pour C₂₇H₃₂N₄ Mₜₕ = 412,2627.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, tétrazole (CB02305) tr = 5,44 min 99,4%.

### 2) (E) 5-[1-[2-(5,6,7,8-tétrahydro-3,5,5,8,8-pentaméthyl)naphtalénylméthylène]-1,2,3,4-tétrahydronaphtalènyl]-1H-tétrazole (CB58248) de formule:

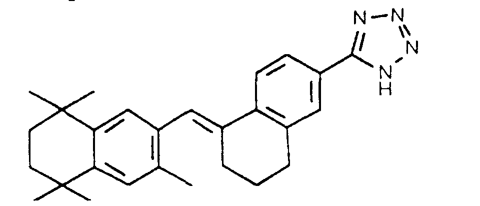

F (°C) = 237-239.

IR (cm⁻¹) : 3400; 2922; 1612; 1566; 1494; 1450; 1392; 1362.

RMN¹H 200MHz (DMSO D₆) : 1,07 (s, 12H, 5,5,8,8-Me); 1,22 (m, 4H, 6,7-CH₂) ; 1,73-1,75 (m, 2H); 2,20 (s, 3H, ArMe) ; 2,59-2,69 (m, 2H) ; 2,87-2,93 (m, 2H); 7,12-7,17 (m, 3H); 7,82-7,85 (m, 2H); 7, 99 (d, 1H, *J* 8,7Hz).

MS EI, 70 eV (m/z, % intensité) : 412 (100, M⁺); 384 (86); 369 (23).

MSHR EI 70 eV : Mₜᵣ = 412,2627 pour C₂₇H₃₂N₄ Mₜₕ = 412,2627.

HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, tétrazole (CB02305) tr = 6,31 min 99,4%.

### a) 2-Méthyl-5-bromo-1-indanone.

Une solution de 5-bromo-1-indanone (3,75 g, 17,76 mmol) dans 18 ml de THF anhydre est portée à -20°C sous atmosphère d'argon et agitation magnétique. On additionne à la seringue une solution de *t*-BuOK lM dans du THF (18 ml, 18 mmol) et poursuit l'agitation pendant 30 min en remontant à 0°C. On additionne alors MeI (1,32 ml, 21, 30 mmol) et poursuit l'agitation 3 h à température ambiante. On ajoute à 0°C 10 ml d'eau distillée, puis extrait à l'éther (4 x 75 ml), sèche sur MgSO₄ filtre et évapore pour obtenir un produit brut que l'on incorpore sur silice et purifie par chromatographie éclair sur silice (éluant éther : éther de pétrole = 2 : 98 jusqu'à 5 : 95). On obtient dans l'ordre d'élution: 1,85 g d'une huile jaunâtre, la 2,2-diméthyl-5-bromo-1-indanone (Rdt = 43%), 0,50 g d'un solide blanc, la 2-méthyl-5-bromo-1-indanone (Rdt = 13%) et 1,20 g d'un solide blanc (32%), la 5-bromo-1-indanone de départ.

RMN¹H 200MHz (CDCl₃) : 1,25 (d, 3H, Me *J* 7Hz); 2,55-2,75 (m, 2H, -CH₂-); 3,25-3,45 (m, 1H, -CHMe-); 7,40-7,65 (m, 3H, ArH).

### b) (E) et (Z) 1-[2-(5,6,7,8-tétrahydro-5,5,8,8-tétraméthyl)naphtalénylméthylène]2,3-dihydro-2-méthyl-5-bromo-1H-indènes.

A une solution de bromure de (5,6,7,8-tétrahydro-5,5,8,8-tétraméthyl-2-naphtalényl)-6-méthyltriphénylphosphonium (4,21 g, 8,44 mmol) dans 15 ml de THF anhydre, on ajoute à -70°C une solution de *t*-BuOK 1M dans le THF (8,44 ml, 8,44 mmol). On poursuit l'agitation à -70 °C pendant 1 h avant d'ajouter une solution de 2-méthyl-5-bromo-1-indanone (0,95 g, 4,22 mmol) dans 5 ml de THF anhydre. Le milieu réactionnel est porté à température ambiante et on agite pendant 16 h. On verse alors le milieu réactionnel sur un mélange eau-glace (150 ml) et HCl 1N (50 ml), puis extrait à l'éther (4 x 80 ml); sèche sur MgSO₄, filtre et évapore. Le produit brut est purifié par chromatographie éclair sur silice (éluant éther de pétrole pur). On obtient 1,56 g d'une huile jaunâtre, le mélange des (E) et (Z) 1-[2-(5,6,7,8-tétrahydro-5,5,8,8-tétraméthyl)naphtalénylméthyléne]2,3-dihydro-2-méthyl-5-bromo-1*H*-indène (Pdt = 90 %) en proportion déterminée par PMN¹H 200 MHz E : Z = 75 : 25, de formule suivante :

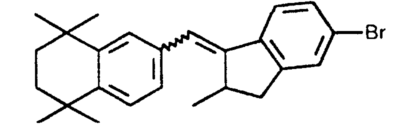

RMN¹H 200MHz (CDCl₃) : 1,15-1,40 (m, 15H, 5,5,8,8-Me et Me); 1,70 (s, 4H, 6,7-CH₂) ; 2,50-2,70 (m, 1H, -CH₂- indène) ; 3,05-3,35 (m, 1,25H, 0,75 H -CH₂- indène isomère E et 0,25 H -CH₂- indène isomère Z et 0,25H -CHMe-indène isomère Z); 3,55-3,75 (m, 0,75H, -CHMe- indène isomère E); 6,47 (d, 0,25H, H vinylique isomère Z); 6,83 (d, 0,75H, H vinylique isomère E *J* 1,5Hz); 6,95-7,50 (m, 6H, ArH).

### c) (E) et (Z) 1-[2-(5,6,7,8-tétrahydro-5,5,8,8-tétraméthyl)naphtalénylméthylène]-2,3-dihydro-2-méthyl-5-cyano-1H-indènes.

A une solution de mélange des (E) et (Z) 1-(2-(5,6,7,8-tétrahydro-5,5,8,8-tétraméthyl)naphtalényl-méthylène]2,3-dihydro-2-méthyl-5-bromo-1*H*-indène (1,56 g, 3,81 mmol) dans 15 ml de DMF anhydre, on ajoute à température ambiante du cyanure de cuivre (0,51 g, 5,71 mmol). Le milieu réactionnel est porté au reflux pendant 24 h sous atmosphère d'argon. Après retour à température ambiante, le milieu réactionnel est dilué avec de l'éther (100 ml) et filtré sur célite. On lave la phase organique avec une solution aqueuse saturée en NaHCO₃ (3 x 25 ml) puis sèche sur MgSO₄, filtre et évapore. On purifie le produit brut par chromatographie éclair sur silice (éluant éther : éther de pétrole 2 : 98). On obtient 0,21 g d'une huile jaunâtre, le 1-[(Z)-2-(5,6,7,8-tétrahydro-5,5,8,8-tétraméthyl)naphtalénylméthylène]-2,3-dihydro-2-méthyl-5-cyano-1*H*-indène (Pdt = 15,5 %) puis 0,60 g d'un solide jaunâtre (recristallisable dans l'hexane), le 1-[(E)-2-(5,6,7,8-tétrahydro-5,5,8,8-tétraméthyl)naphtalényl-méthylène]-2,3-dihydro-2-méthyl-5-cyano-1*H*-indène (Rdt = 44 %).

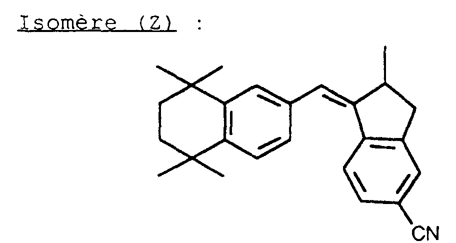

RMN¹H 200MHz (CDCl₃) : 1,23 et 1,30 (2s, 12H, 5,5,8,8-Me); 1,28 (d, 3H, Me indène *J* 7Hz); 1,70 (s, 4H, 6,7-CH₂); 2,62 (m, 1H, -CH₂- indène) ; 3, 14 (m, 1H, -CH₂-indène); 3,21 (t, 1H, -CHMe- indène *J* 7Hz); 6,61 (s, 1H, H vinylique) ; 7,06 (dd, 1H, ArH *J* 1, 1Hz et *J* 8Hz) ; 7, 15-7, 55 (m, 5H, ArH) .

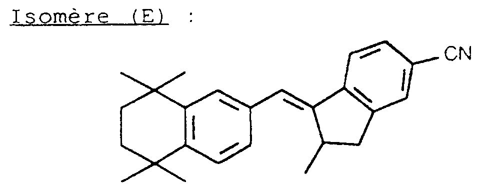

F (°C) = 104.

RMN¹H 200MHz (CDCl₃): 1,22 (d, 3H, Me indène *J* 7Hz) ; 1,29 (s, 6H, 2Me) ; 1,31 (s, 3H, Me) ; 1,33 (s, 1H, Me) ; 1,70 (s, 4H, 6,7-CH₂) ; 2,67 (d, 1H, -CH₂- indène *J* 16,5Hz); 3,27 (dd, 1H, -CH₂- indène *J* 16, 5Hz et *J* 7,8Hz); 3, 68 (q, 1H, -CHMe- indène *J* 16, 5Hz *J* 7,8Hz) ; 6,94 (d, 1H, H vinylique *J* 1, 4Hz); 7,25-7,65 (m, 6H, ArH).

### d) Acide (E) 1-[2-(5,6,7,8-tétrahydro-5,5,8,8-tétraméthyl)naphtalénylméthyléne]-2,3-dihydro-2-méthyl-1H-indène-5-carboxylique (CB52809).

A une suspension de (E) 1-[2-(5,6,7,8-tétrahydro-5,5,8,8-tétraméthyl)naphtalénylméthylène]-2,3-dihydro-2-méthyl-5-cyano-1*H*-indène (0,20 g, 0,56 mmol) en solution hydroéthanolique (H₂O 0,80 ml et EtOH 5,0 ml) et 1 ml de THF est ajouté de la potasse (0,64 g, 11,2 mmol). On chauffe à reflux sous agitation magnétique pendant 48 h. On reprend à l'eau (20 ml), acidifie par HCl 1N, extrait à l'éther (3 x 50 ml), sèche par MgSO₄, filtre et évapore. On obtient après un lavage au pentane, filtration puis séchage 0,19 g d'une poudre blanche, l'acide (E) 1-[2-(5,6,7,8-tétrahydro-5,5,8,8-tétraméthyl)naphtalényl-méthylène]-2,3-dihydro-2-méthyl-1*H*-indène-5-carboxylique (CB52809) (Rdt = 91%) de formule :

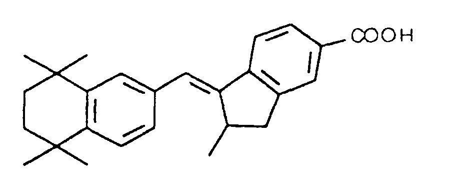

F (°C) = 206.

RMN¹H 200MHz (CDCl₃): 1,21 (d, 3H, Me indène *J* 6,9Hz); 1,29 (s, 6H, 2Me); 1,31 (s, 3H, Me); 1,32 (s, 3H, Me); 1,70 (s, 4H, 6,7-CH₂) ; 2,70 (d, 1H, -CH₂- indène *J* 16,4Hz); 3,30 (dd, 1H, -CH₂- indène *J* 16, 4Hz et *J* 7,5Hz); 3,69 (m, 1H, -CHMe- indène); 6,96 (s, 1H, H vinylique); 7,31 (s, 2H, ArH); 7,48 (s, 1H, ArH); 7,63 (d, 1H, ArH *J* 8,4Hz); 7,99 (d, 1H, ArH *J* = 8,4Hz); 8,01 (s, 1H, ArH).

MS EI 70 eV (m/z, % intensité) : 374 (M⁺, 100 %) ; 359 (M⁺-CH₃, 95), 143 (21).

MSHR EI 70 eV : Mₜᵣ = 374,2238 pour C₂₆H₃₀O₂ Mₜₕ = 354,2446.

HPLC : Colonne Waters HR C₁₈, 8 x 100 mm, 6 µ, détecteur UV Waters. 486 à 260 nm, débit 3 ml/min, éluant MeOH : H₂O = 90 : 10 + 0,1% de TFA, acide (CB52809) tr = 6,21 min 97,5% ; impureté tr = 7,37 min 1,4%.

### e) Acide (Z) 1-[2-(5,6,7,8-tétranydro-5,5,8,8-tétraméthyl)naphtalénylméthylène]-2,3-dihydro-2-méthyl-1H-indène-5-carboxylique (CB91261) et (Z) 1-[2-(5,6,7,8-tétrahydro-5,5,8,8-tétraméthyl)naphtalénylméthylène]-2,3-dihydro-2-méthyl-1H-indène-5-amide (CB96711).

A une suspension de (Z) 1-[2-(5,6,7,8-tétrahydro-5,5,8,8-tétraméthyl)naphtalénylméthylène]-2,3-dihydro-2-méthyl-5-cyano-1*H*-indène (0,20 g, 0,56 mmol) en solution hydroéthanolique (H₂O 0,80 ml et EtOH 5,0 ml) est ajoutée de la potasse (0,64 g, 11,2 mmol). On chauffe à reflux sous agitation magnétique pendant 24 h, reprend à l'eau (20 ml), acidifie par HCl 1N, extrait à l'éther (3 x 50 ml), sèche par MgSO₄, filtre et évapore. Le produit brut est purifié par HPLC préparative sur une colonne Waters HR C₁₈ (25 x 100 mm) avec comme éluant MeOH : H₂O = 90 : 10 + 0,1% de TFA. On obtient 0,04 g d'un solide blanc, le (Z) 1-[2-(5,6,7,8-tétrahydro-5,5,8,8-tètraméthyl)naphtalénylméthylène)2,3-dihydro-2-méthyl-1*H*-indène-5-amide (CB96711) (Rdt = 19%) et 0,12 g d'un solide blanc, l'acide (Z) 1-[2-(5,6,7,8-tétrahydro-5,5,8,8-tétraméthyl)naphtalénylméthylène)2,3-dihydro-2-méthyl-1*H*-indène-5-carboxylique (CB91261) (Rdt = 57%).

### (Z) 1-[-2-(5,6,7,8-Tétrahydro-5,5,8,8-tétraméthyl)naphtalénylméthylène]-2,3-dihydro-2-méthyl-1H-indène-5-amide (CB96711) :

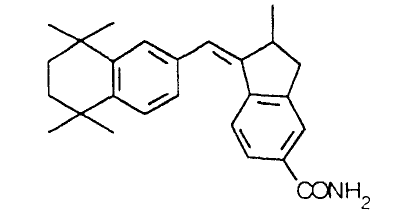

F (°C) = 187-188.

RMN¹H 200MHz (CDCl₃): 1,22 et 1,29 (2s, 12H, 5,5,8,8-Me); 1,27 (d, 3H, Me indène *J* 7Hz); 1,70 (s, 4H, 6,7-CH₂) ; 2,61 (m, 1H, -CH₂- indène) ; 3,00-3,30 (m, 2H, -CH₂- et -CHMe- indène); 6,54 (s, 1H, H vinylique); 7,08 (dd, 1H, ArH *J* 1,7Hz et *J* 8,1Hz); 7,23-7,45 (m, 4H, ArH); 7,67 (s, 1H, ArH).

MS EI 70 eV (m/z, % intensité) : 373 (M , 100 %); 358 (M⁺-CH₃, 60), 185 (12); 141 (11); 129 (12); 128 (25).

MSHR EI 70 eV : Mₜᵣ = 373,2406 pour C₂₆H₃₁NO Mₜₕ = 373,2406.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, amide (CB96711) tr = 3,57 min 99,5% ; impureté tr = 18,4 min 0,25%.

Acide (Z) 1-[2-(5,6,7,8-tétrahydro-5,5,8,8-tétraméthyl)naphtalénylméthylène]-2,3-dihydro-2-méthyl-1*H*-indène-5-carboxylique (CB91261) de formule :

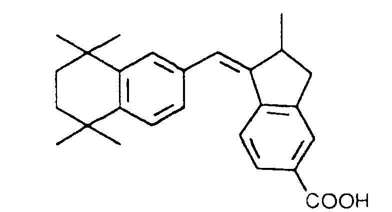

F (°C) = 196-197.

RMN¹H 200MHz (CDCl₃) : 1,22 et 1,29 (2s, 12H, 5,5,8,8-Me); 1,27 (d, 3H, Me indène *J* 7Hz) ; 1,70 (s, 4H, 6,7-CH₂) ; 2,63 (m, 1H, -CH₂- indène); 3,03-3,30 (m, 2H, -CH2- et -CHMe- indène); 6,57 (s, 1H, H vinylique) ; 7,07 (dd, 1H, ArH *J* 1,7Hz et *J* 8,1Hz) ; 7,25-7,45 (m, 3H, ArH); 7,67 (dd, 1H, ArH *J* 1,7Hz et *J* 8,1Hz) ; 7,97 (s, 1H, ArH).

MS EI 70 eV (m/z, % intensité) : 374 (M⁺, 100 %); 359 (M⁺-CH₃, 60), 143 (29); 128 (25).

MSHR EI 70 eV : Mₜᵣ = 374,2238 pour C₂₆H₃₀O₂ Mₜₕ = 374,2246.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB91261) tr = 6,17 min 97,9% ; impureté tr = 7,4 min 1,2%.

### f) (E) 5-[1-[2-(5,6,7,8-Tétrahydro-5,5,8,8-tétraméthyl)naphtalénylméthylène]-2,3-dihydro-2-méthyl-1H-indényl]-1H-tétrazole (CB69831).

A une solution de (E) 1-[2-(5,6,7,8-tétrahydro-5,5,8,8-tétraméthyl)naphtalényl-méthylène]-2,3-dihydro-2-méthyl-5-cyano-1*H*-indène (0,42 g, 1,18 mmol) dans du toluène anhydre (2,50 ml), on additionne successivement de l'oxyde de dibutyletain (30 mg) et de l'azoture de triméthylsilyle (0,31 ml, 2,36 mmol). Le milieu réactionnel est chauffé 16 h à reflux (110°C) sous atmosphère d'argon et agitation magnétique. Après refroidissement, on évapore le toluène, reprend au dichlorométhane et incorpore sur silice. On purifie par chromatographie éclair sur silice (éluant CH₂Cl₂ puis MeOH : CH₂Cl₂ = 5 : 95) pour obtenir après évaporation, lavage au pentane, filtration puis séchage à la pompe à palette, 0,10 g d'une poudre blanchâtre, le (E) 5-[1-[2-(5,6,7,8-tétrahydro-5,5,8,8-tétraméthyl)naphtalénylméthylène]-2,3-dihydro-2-méthyl-1*H*-indényl]-1*H*-tétrazole (CB69831) (Rdt = 21%) de formule suivante :

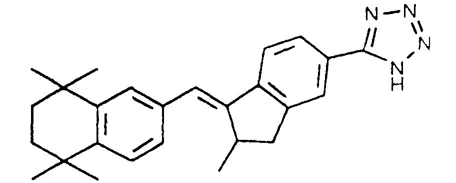

F (°C) = 149.

RMN¹H 200MHz (CDCl₃) : 1,22 (d, 3H, Me *J* 6,9Hz) ; 1,28 (s, 6H, 2 Me); 1,29 (s, 3H, Me); 1,30 (s, 3H, Me) ; 1,59 (s, 4H, 6,7-CH₂) ; 2,69 (d, 1H, -CH₂- indene *J* 16, 4Hz); 3,29 (dd, 1H, -CH₂- indène *J* 7, 5Hz *J* 16, 4Hz); 3,70 (m, 1H, -CHMe- indène); 6,92 (d, 1H, H vinylique *J* 1,3Hz) ; 7,29 (s, 1H, ArH); 7,46 (s, 1H, ArH); 7,69 (d, 1H, ArH *J* 8.0Hz); 7,94 (d, 1H, ArH *J* 8.0Hz); 7,99 (s, 1H, ArH).

MS EI 70 eV (m/z, % intensité) : 398 (M⁺, 62 %); 370 (100), 355 (39); 185 (12); 143 (14).

MSHR EI 70 eV : Mₜᵣ = 398,2466 pour C₂₆H₃₀N₄ Mₜₕ = 373,2470.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, tétrazole (CB69831) tr = 4,32 min 98,6% ; impureté tr = 5,77 min 0,30%.

### Exemple 6 : Préparation des acides (E) et (Z) 1-[2-(5,6,7,8-tétrahydro-5,5,8,8-tétraméthyl)naphtalényl-méthylène]-2,3-dihydro-1H-indène-5-carboxyliques (CB78937 et CB27871) et des (E) et (Z) 5-[1-[2-(5,6,7,8-tétrahydro-5,5,8,8-tétraméthyl)naphtalénylméthylènel-2,3-dihydro-1H-indényl]-1H-tétrazoles (CB99811 et CB94083).

### a) (E) et (Z) 1-[2-(5,6,7,8-Tétrahydro-5,5,8,8-tétraméthyl)naphtalénylméthylène]-2,3-dihydro-5-bromo-1H-indènes.

A du bromure de (5,6,7,8-tétrahydro-5,5,8,8-tétraméthyl-2-naphtalényl)-6-méthyltriphénylphosphonium (7,00 g, 12,56 mmol) dans 20 ml de THF anhydre, sous atmosphère d'argon et agitation magnétique, on ajoute à -70 °C une solution de *t*-BuOK 1M dans le THF (12,56 ml, 12,56 mmol). On poursuit l'agitation à -70 °C pendant 1 h avant d'ajouter une solution de 5-bromo-1-indanone (1,33 g, 6,28 mmol) dans 15 ml de THF anhydre. Le milieu réactionnel est porté à température ambiante et on agite pendant 16 h. On verse alors le milieu réactionnel sur un mélange eau-glace (150 ml), puis extrait à l'éther (4 x 100 ml), sèche sur MgSO₄, filtre et évapore. Le produit brut est purifié par chromatographie éclair sur silice (éluant éther de pétrole pur). On obtient 0,57 g d'une huile incolore, le (Z) 1-[2-(5,6,7,8-tétrahydro-5,5,8,8-tétraméthyl)naphtalénylméthylène]-2,3-dihydro-5-bromo-1*H*-indène (Rdt = 23%) puis 1,42 g d'un solide jaunâtre, le 1-[(E)-2-(5,6,7,8-tétrahydro-5,5,8,8-tétraméthyl)naphtalénylméthylène]-2,3-dihydro-5-bromo-1*H*-indène (Rdt = 57%).

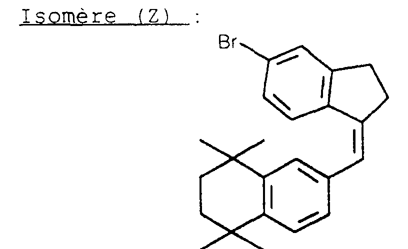

RMN¹H 200MHz (CDCl₃) : 1,23 et 1,29 (2s, 12H, 5,5,8,8-Me) ; 1,69 (s, 4H, 6,7-CH₂) ; 2,80-3,00 (m, 4H, -CH₂-indène); 6,56 (s, 1H, H vinylique); 7,00-7,10 (m, 2H, ArH); 7,15-7,40 (m, 4H,ArH).

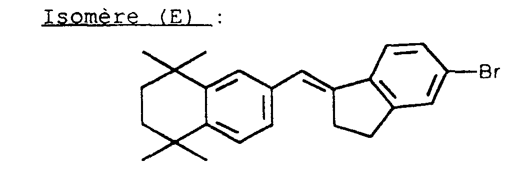

F (°C) = 141-142.

RMN¹H 200MHz (CDCl₃) : 1,28 et 1,30 (2s, 12H, 5,5,8,8-Me); 1,69 (s, 4H, 6,7-CH₂) ; 3,07 (s large, 4H, -CH₂- indène); 6,88 (s, 1H, H vinylique); 7,15-7,50 (m, 6H, ArH).

MS EI 70 eV (m/z, % intensité) : 396-394 (M⁺, 99%-98%); 381-379 (100-99).

### b) (E) 1-[2-(5,6,7,8-Tétrahydro-5,5,8,8-tétraméthyl)naphtalénylméthyléne]-2,3-dihydro-5-cyano-1H-indène.

A une solution de (E) 1-[2-(5,6,7,8-tétrahydro-5,5,8,8-tétraméthyl)naphtalénylméthylène]-2,3-dihydro-5-bromo-1*H*-indène (1,20 g, 3,03 mmol) dans 10 ml de DMF anhydre, on ajoute à température ambiante du cyanure de cuivre (0,35 g, 3,94 mmol). Le milieu réactionnel est porté au reflux pendant 70 h sous atmosphère d'argon. Après retour à température ambiante le milieu réactionnel est dilué avec de l'éther (100 ml) et filtré sur célite. On lave la phase organique avec une solution aqueuse saturée en NaHCO₃ (3 x 25 ml) puis sèche sur MgSO₄, filtre et évapore. On purifie le produit brut par chromatographie éclair sur silice (éluant éther : éther de pétrole 2 : 98 puis 4 : 96). On obtient 0,45 g d'un solide blanc, le (E) 1-[2-(5,6,7,8-tétrahydro-5,5,8,8-tétraméthyl)naphtalényl-méthylène]-2,3-dihydro-5-cyano-1*H*-indène (Rdt = 43 %).

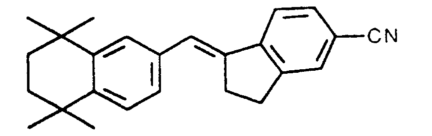

F (°C) = 160-161.

RMN¹H 200MHz (CDCl₃) : 1,29 et 1,31 (2s, 12H, 5,5,8,8-Me); 1,69 (s, 4H, 6,7-CH₂) ; 3,11 (s large, 4H, -CH₂- indène); 7,01 (s, 1H, H vinylique); 7,23-7,55 (m, 5H, ArH); 7,62 (d, 1H, ArH *J* 8Hz).

MS EI 70 eV (m/z, % intensité) : 341 (M⁺, 67%); 326 (100).

### c) Acide (E) 1-[2-(5,6,7,8-tétrahydro-5,5,8,8-tétraméthyl)naphtalénylméthylène]-2,3-dihydro-1H-indène-5-carboxylique (CB78937).

A une suspension du (E) 1-[2-(5,6,7,8-tétrahydro-5,5,8,8-tétraméthyl)naphtalénylméthylène]-2,3-dihydro-5-cyano-1*H*-indène (0,25 g, 0,73 mmol) dans une solution hydroéthanolique (H₂O 0,50 ml et EtOH 4,5 ml) est ajoutée de la potasse (0,41 g, 7,30 mmol). On chauffe à reflux sous agitation magnétique pendant 7 h. Après refroidissement du milieu réactionnel, on reprend à l'eau distillée (20 ml), acidifie par HCl 1N (15 ml), extrait à l'éther (3 x 40 ml), sèche par MgSO₄, filtre et évapore. On obtient après un lavage à l'hexane, filtration puis séchage 0,20 g d'un solide blanc, l'acide (E) 1-[2-(5,6,7,8-tétrahydro-5,5,8,8-tétraméthyl)naphtalényl-méthylène]-2,3-dihydro-2-1*H*-indène-5-carboxylique (CB78937) (Rdt = 76%).

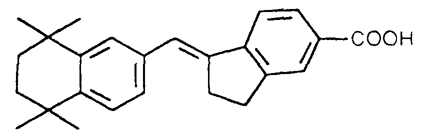

F (°C) = 294.

RMN¹H 200MHz (CDCl₃) : 1,29 et 1,32 (2s, 12H, 5,5,8,8-Me); 1,70 (s, 4H, 6,7-CH₂) ; 3,14 (s, 4H, -CH₂-indène); 7,05 (s, 1H, H vinylique); 7,25-7,35 (m, 2H, ArH); 7,42 (s, 1H, ArH); 7,65 (d, 1H, ArH *J* 8,1Hz) ; 7,98 (d, 1H, ArH *J* = 8,1Hz); 8,01 (s, 1H, ArH).

MS EI 70 eV (m/z, % intensité) : 360 (M⁺, 72 %); 345 (M⁺-CH₃, 100), 143 (22); 129 (55).

MSHR EI 70 eV : Mₜᵣ = 360,2103 pour C₂₅H₂₈O₂ Mₜₕ = 360,2090.

HPLC : Colonne Waters HR C₁₈, 8 x 100 mm, 6 µ, détecteur UV Waters 486 à 260 nm, débit 3 ml/min, éluant MeOH : H₂O = 93 : 7 + 0,1% de TFA, acide (CB78937) tr = 4,31 min 99,8%.

### d) (E) 5-[1-[2-(5,6,7,8-Tétrahydro-5,5,8,8-tétraméthyl)naphtalénylméthylènel-2,3-dihydro-1H-indényl]-1H-tétrazole (CB99811).

A une solution du (E) 1-[2-(5,6,7,8-tétrahydro-5,5,8,8-tétraméthyl)naphtalénylméthylène)-2,3-dihydro-5-cyano-1*H*-indène (0,20 g, 0,59 mmol) dans 1,25 ml de toluène anhydre, on additionne successivement de l'oxyde de dibutylétain (10 mg) et de l'azoture de triméthylsilyle (0,16 ml, 1,22 mmol). Le milieu réactionnel est chauffé 16 h à reflux (110°C) sous atmosphère d'argon et agitation magnétique. Après refroidissement, on évapore le toluène, reprend au dichlorométhane et incorpore sur silice. On purifie par chromatographie éclair sur silice (éluant CH₂Cl₂ puis MeOH : CH₂Cl₂ = 5 : 95) pour obtenir après évaporation, puis séchage à la pompe à palette, 0,14 g d'un solide blanc, le (E) 5-[1-[2-(5,6,7,8-tétrahydro-5,5,8,8-tétraméthyl)naphtalénylméthylène)-2,3-dihydro-1*H*-indényl]-1*H*-tétrazole (CB99811) (Rdt = 60%) de formule :

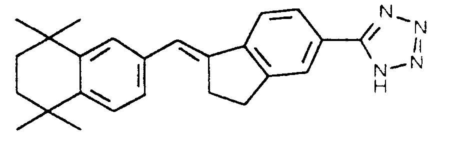

F (°C) = 248.

RMN¹H 200MHz (CDCl₃) : 1,28 et 1,32 (2s, 12H, 5,5,8,8-Me); 1,71 (s, 4H, 6,7-CH₂) ; 3,19 (s, 4H, -CH₂-indène); 7,16 (s, 1H, H vinylique); 7,25-7,40 (m, 2H, ArH); 7,52 (s, 1H, ArH); 7,84 (d, 1H, ArH J 8,1Hz) ; 8,00 (d, 1H, ArH *J* = 8,1Hz) ; 8,04 (s, 1H, ArH).

MS EI 70 eV (m/z, % intensité) : 384 (M⁺, 24%); 356 (62), 341 (32); 61 (100).

MSHR EI 70 eV : Mₜᵣ = 384,2291 pour C₂₅H₂₈N₄ = 384,2314.

HPLC : Colonne Waters HR C₁₈, 8 x 100 mm, 6 µ, détecteur UV waters 486 à 260 nm, débit 3 ml/min, éluant MeOH : H₂O = 90 : 10 + 0,1% de TFA, tétrazole (CB99811) tr = 4,37 min 95,2%.

### e) (Z) 1-[2-(5,6,7,8-Tétrahydro-5,5,8,8-tétraméthyl)naphtalénylméthylène]-2,3-dihydro-5-cyano-1H-indène.

A une solution de (Z) 1-[2-(5,6,7,8-tétrahydro-5,5,8,8-tétraméthyl)naphtalényl-mèthylène]2,3-dihydro-5-bromo-1*H*-indène (0,51 g, 1,29 mmol) dans 5 ml de DMF anhydre, on ajoute à température ambiante du cyanure de cuivre (0,16 g, 1,81 mmol). Le milieu réactionnel est porté au reflux pendant 30 h sous atmosphère d'argon. Après retour à température ambiante le milieu réactionnel est dilué avec de l'éther (100 ml) et filtre sur célite. On lave la phase organique avec une solution aqueuse saturée en NaHCO₃ (3 x 25 ml) puis sèche sur MgSO₄, filtre et évapore. On purifie le produit brut par chromatographie éclair sur silice (éluant éther : éther de pétrole 2 : 98). On obtient 0,16 g d'un solide blanc, le (Z) 1-[2-(5,6,7,8-tétrahydro-5,5,8,8-tétraméthyl)naphtalényl-méthylène)-2,3-dihydro-5-cyano-1*H*-indène (Rdt = 36 %) de formule :

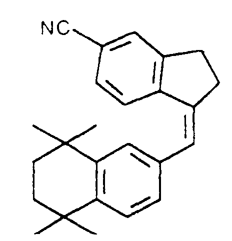

F(°C) = 132-135.

RMN¹H 200MHz (CDCl₃) : 1,22 et 1,29 (2s, 12H, 5,5,8,8-Me); 1,69 (s, 4H, 6,7-CH₂) ; 2,80-3,10 (m, 4H, -CH₂-indène); 6,70 (s, 1H, H vinylique); 7,03 (dd, 1H, ArH *J* 1,7Hz *J* 8,1Hz); 7,18 (dd, 1H, ArH *J* 1,2Hz *J* 8,2Hz); 7,27 (d, 1H, ArH *J* 8,2Hz) ; 7,30 (s, 1H, ArH); 7,38 (d, 1H, ArH *J* 8, 1Hz); 7,49 (s, 1H, ArH).

MS EI 70 eV (m/z, % intensité) : 341 (M⁺, 96%); 326 (100); 154 (64); 143 (26); 142 (35); 127 (36).

### f) Acide (Z) 1-[2-(5,6,7,8-tétrahydro-5,5,8,8-tétraméthyl)naphtalénylméthylène]-2,3-dihydro-1H-indène-5-carboxylique (CB27871).

A une suspension du (Z) 1-[2-(5,6,7,8-tétrahydro-5,5,8,8-tétraméthyl)naphtalénylméthylène]-2,3-dihydro-5-cyano-1*H*-indène (0,13 g, 0,38 mmol) dans une solution hydroéthanolique (H₂O 0,50 ml et EtOH 4,5 ml) est ajouté de la potasse (0,48 g, 8,57 mmol). On chauffe à reflux sous agitation magnétique pendant 20 h. Après refroidissement, on acidifie par HCl 1N (15 ml), extrait à l'éther (3 x 30 ml), sèche par MgSO₄, filtre et évapore. Le produit brut est purifié par HPLC préparative sur une colonne Waters HR C₁₈ (25 x 100 mm) avec comme éluant MeOH : H₂O = 90 : 10 + 0,1% de TFA. On obtient après évaporation puis séchage 0,07 g d'une poudre blanche, l'acide (Z) 1-[2-(5,6,7,8-tétrahydro-5,5,8,8-tétraméthyl)naphtalénylméthylène]-2,3-dihydro-2-1*H*-indène-5-carboxylique (CB27871) (Rdt = 52,5%) de formule:

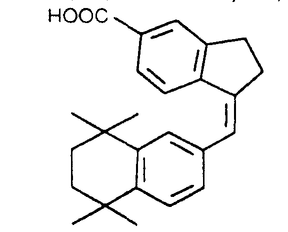

F (°C) = 232.

RMN¹H 200MHz (CDCl₃): 1,23 et 1,30 (2s, 12H, 5,5,8,8-Me); 1,69 (s, 4H, 6,7-CH₂) ; 2,97 (m, 4H, -CH₂-indène); 6,69 (s, 1H, H vinylique); 7,07 (dd, 1H, ArH *J* 1,3Hz J 8,1Hz); 7,28 (d, 1H, ArH J 8,1Hz) ; 7,34 (d, 1H, ArH *J* 1, 3Hz); 7,41 (d, 1H, ArH *J* = 8,3Hz); 7,67 (dd, 1H, ArH *J* 1, 3Hz *J* 8, 3Hz) ; 7,95 (s, 1H, ArH).

MS EI 70 eV (m/z, % intensité) : 360 (M⁺, 79 %); 345 (M⁺-CH₃, 100), 129 (46).

MSHR EI 70 eV : Mₜᵣ = 360,2085 pour C₂₅H₂₈O₂ Mₜₕ = 360,2090.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1 % de TFA, acide (CB27871) tr = 5,63 min 99,6%.

### g) (Z) 5-[1-[2-(5,6,7,8-Tétrahydro-5,5,8,8-tétraméthyl)naphtalénylméthylène]-2,3-dihydro-1H-indényl]-1H-tétrazole (CB94083).

A une solution du (Z) 1-[2-(5,6,7,8-tétrahydro-5,5,8,8-tétraméthyl)naphtalénylméthylène]-2,3-dihydro-5-cyano-1*H*-indène (0,14 g, 0,42 mmol) dans 0,9 ml de toluène anhydre, on additionne successivement de l'oxyde de dibutylétain (10 mg) et de l'azoture de triméthylsilyle (0,12 ml, 0,84 mmol). Le milieu réactionnel est chauffé 16 h à reflux (110°C) sous atmosphère d'argon et agitation magnétique. Après refroidissement, on évapore le toluène, reprend au dichlorométhane et incorpore sur silice. On purifie par chromatographie éclair sur silice (éluant CH₂Cl₂ puis MeOH : CH₂Cl₂ = 5 : 95) pour obtenir après évaporation, puis séchage à la pompe à palette, 0,08 g d'un solide blanc, le (Z) 5-[1-[2-(5,6,7,8-tétrahydro-5,5,8,8-tétraméthyl)naphtalénylméthylène]-2,3-dihydro-1*H*-indényl]-1*H*-tétrazole (CB94083) (Rdt = 49,5%).

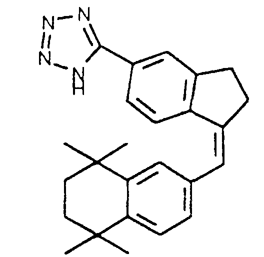

F (°C) = 237-239.

RMN¹H 200MHz (DMSO-D₆) : 1,23 et 1,25 (2s, 12H, 5,5,8,8-Me); 1,67 (s, 4H, 6,7-CH₂) ; 3,48 (s, 4H, -CH₂-indène); 6,40 (s, 1H, H vinylique); 7,05 (dd, 1H, ArH *J* 8,1Hz *J* 1, 7Hz) ; 7,24 (d, 1H, ArH *J* 8,1Hz); 7,35 (d, 1H, ArH *J* 1, 7Hz); 7,54 (d, 1H, ArH *J* 8, 3 Hz); 8,01 (d, 1H, ArH *J* 8,3Hz); 8,19 (s, 1H, ArH).

MS EI 70 eV (m/z, % intensité) : 384 (M⁺, 35%); 369 (18); 356 (100), 201 (92); 143 (42); 128 (47).

MSHR EI 70 eV : Mₜᵣ = 384,2309 pour C₂₅H₂₈N₄ = 384,2314.

HPLC : Colonne Waters HR C₁₈, 8 x 100 mm, 6 µ, détecteur UV Waters 486 à 260 nm, débit 3 ml/min, éluant MeOH : H₂O = 91 : 9 + 0,1% de TFA, tétrazole (CB94083) tr = 2,77 min 99,2%.

### Exemple 7 : Préparation des acides (E) et (Z) 1-[2-(5,6,7,8-tétrahydro-3,5,5,8,8-pentaméthyl)naphtalényl méthylène]-2,3-dihydro-1H-indène-5-carboxyliques (CB40341 et CB75403) et des (E) et (Z) 5-[1-[2-(5,6,7,8-tétrahydro-3,5,5,8,8-pentaméthyl)naphtalénylméthyléne]-2,3-dihydro-1H-indényl]-1H-tétrazoles (CB23804 et CB02981).

### a) (E) et (Z) 1-[2-(5,6,7,8-tétrahydro-3,5,5,8,8-pentaméthyl)naphtalénylméthylène]-2,3-dihydro-5-bromo-1H-indènes.

A une solution de bromure de (5,6,7,8-tétrahydro-3,5,5,8,8-pentaméthyl-2-naphtalényl)-6-méthyltriphénylphosphonium (7,00 g, 12,56 mmol) dans 28 ml de THF anhydre, sous atmosphère d'argon et agitation magnétique, on ajoute à - 70 °C une solution de *t*-BuOK 1M dans le THF (12,56 ml, 12,56 mmol). On poursuit l'agitation à - 70 °C pendant 1 h avant d'ajouter une solution de 5-bromo-1-indanone (1,33 g, 6,28 mmol) dans 10 ml de THF anhydre. Le milieu réactionnel est porté à température ambiante et on agite pendant 17 h. On verse alors le milieu réactionnel sur un mélange eau-glace (150 ml), puis extrait à l'éther (3 x 150 ml), sèche sur MgSO₄, filtre et évapore. Le produit brut est purifié par chromatographie éclair sur silice (éluant éther de pétrole pur). On obtient 0,57 g d'un solide blanc, le (Z) 1-[2-(5,6,7,8-tétrahydro-3,5,5,8,8-pentaméthyl) naphtalényl méthylène]-2,3-dihydro-5-bromo-1*H*-indène (Rdt = 25%) puis 1,09 g d'un solide blanc, le 1-[(E)-2-(5,6,7,8-tétrahydro-3,5,5,8,8-pentaméthyl)naphtalénylméthylène)-2,3-dihydro-5-bromo-1*H*-indène (Rdt = 42,5%).

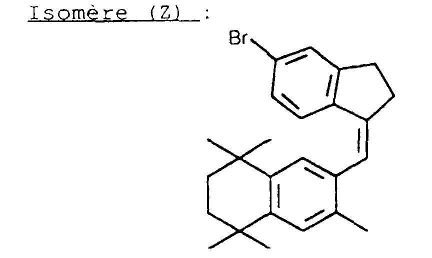

F (°C) = 112.

RMN¹H 200MHz (CDCl₃) : 1,20 et 1,31 (2s, 12H, 5,5,8,8-Me); 1,68 (s, 4H, 6,7-CH₂) ; 2,21 (s, 3H, Me vinylique); 2,96 (m, 4H, -CH₂- indène); 6,53 (s, 1H, H vinylique); 6,89 (d, 1H, ArH *J* 8, 3Hz); 7,00 (dd, 1H, ArH *J* 1,7Hz *J* 8,3Hz); 7,14 (s, 1H, ArH) ; 7,26 (s, 1H, ArH); 7,36 (d, 1H, ArH *J* 1,7Hz).

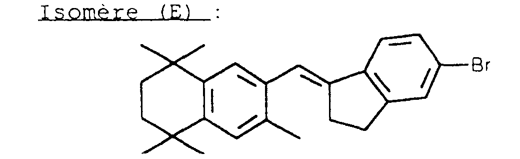

RMN¹H 200MHz (CDCl₃) : 1,29 et 1,30 (2s, 12H, 5,5,8,8-Me); 1,69 (s, 4H, 6,7-CH₂) ; 2,31 (s, 3H, Me); 3,02 (s, 4H, -CH₂- indène); 6,98 (s, 1H, H vinylique); 7,12 (s, 1H, ArH); 7,34 (dd, 1H, ArH *J* 1,5Hz *J* 8,2Hz); 7,38 (s, 1H, ArH); 7,41 (d, 1H, ArH *J* 1, 5Hz) ; 7,45 (d, 1H, ArH *J* 8,2Hz).

### b) (Z) 1-[2-(5,6,7,8-Tétrahydro-3,5,5,8,8-pentaméthyl)naphtalénylméthylène]-2,3-dihydro-5-cyano-1H-indène.

A une solution de (Z) 1-[2-(5,6,7,8-tétrahydro-3,5,5,8,8-pentaméthyl)naphtalénylméthylène]-2,3-dihydro-5-bromo-1*H*-indène (0,61 g, 1,49 mmol) dans 10 ml de DMF anhydre, on ajoute à température ambiante du cyanure de cuivre (0,19 g, 2,09 mmol). Le milieu réactionnel est porté au reflux pendant 16 h sous atmosphère d'argon. Après retour à température ambiante le milieu réactionnel est dilué avec de l'éther (50 ml) et filtré sur célite. On lave la phase organique avec une solution aqueuse saturée en NaHCO₃ (3 x 50 ml) puis sèche sur MgSO₄, filtre et évapore. On purifie le produit brut par chromatographie éclair sur silice (éluant éther : éther de pétrole 2 : 98). On obtient 0,34 g d'un solide blanc, le (Z) 1-[2-(5,6,7,8-tétrahydro-3,5,5,8,8-pentaméthyl)naphtalényl méthylène]-2,3-dihydro-5-cyano-1*H*-indène (Rdt = 64 %) :

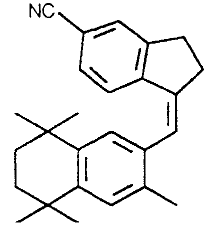

RMN¹H 200MHz (CDCl₃) : 1,17 et 1,29 (2s, 12H, 5,5,8,8-Me) ; 1,67 (s, 4H, 6,7-CH₂) ; 2,19 (s, 3H, Me) ; 2,98 (m, 4H, -CH₂- indène); 6,68 (s, 1H, H vinylique); 7,02-7,21 (m, 4H, ArH); 7,48 (s, 1H, ArH).

MS EI 70 eV (m/z, % intensité) : 355 (M⁺, 59); 340 (M⁺-CH₃, 100); 149 (19); 142 (20); 84 (28); 73 (17); 71 (19); 57 (62); 55 (38).

### c) Acide (Z) 1-[2-(5,6,7,8-tétrahydro-3,5,5,8,8-pentaméthyl)naphtalénylméthylène]-2,3-dihydro-1H-indène-5-carboxylique (CB75403).

A une suspension du (Z) 1-[2-(5,6,7,8-tétrahydro-3,5,5,8,8-pentaméthyl)naphtalénylméthylène]-2,3-dihydro-5-cyano-1*H*-indène (0,14 g, 0,39 mmol) dans une solution hydroéthanolique (H₂O 0,55 ml et EtOH 3,3 ml) est ajoutée de la potasse (0,22 g, 3,94 mmol). On chauffe à reflux sous agitation magnétique pendant 24 h. Après refroidissement, on acidifie par HCl 3N, extrait à l'éther (3 x 50 ml), sèche par MgSO₄, filtre et évapore. Le produit brut est purifié par HPLC préparative sur une colonne Waters HR C₁₈ (25 x 100 mm) avec comme éluant MeOH : H₂O = 90 : 10 + 0,1% de TFA. On obtient après évaporation puis séchage 0,10 g d'un solide blanc, l'acide (Z) 1-[2-(5,6,7,8-tétrahydro-3,5,5,8,8-pentaméthyl)-naphtalénylméthylène]-2,3-dihydro-2-1*H*-indène-5-carboxylique (CB75403) (Rdt = 70,5%) de formule:

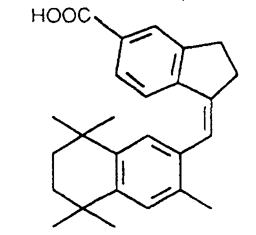

F (°C) = 242.

RMN¹H 200MHz (CDCl₃) : 1,18 et 1,30 (2s, 12H, 5,5,8,8-Me); 1,67 (s, 4H, 6,7-CH₂) ; 2,20 (s, 3H, Me) ; 3,01 (m, 4H, -CH₂- indène); 6,66 (s large, 1H, H vinylique) ; 7,10 (d, 1H, ArH *J* 8,2Hz); 7,15 (s, 1E, ArH); 7,26 (s, 1H, ArH); 7,62 (dd, 1H, ArH *J* 1, 5Hz *J* 8, 2Hz) ; 7,95 (d, 1H, ArH *J* 1,5Hz).

MS EI 70 eV (m/z, % intensité) :

MSHR EI 70 eV : Mₜᵣ = 360,2234 pour C₂₆H₃₀O₂ Mₜₕ = 374,2246.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB75403) tr = 6,36 min 98,0%.

### d) (Z) 5-[1-[2-(5,6,7,8-Tétrahydro-3,5,5,8,8-pentaméthyl)naphtalénylméthylènel-2,3-dihydro-1H-indényl]-1H-tétrazole (CB02981).

A une solution de (Z) 1-[2-(5,6,7,8-tétrahydro-3,5,5,8,8-pentaméthyl)naphtalényl-méthylène)-2,3-dihydro-5-cyano-1*H*-indène (0,18 g, 0,51 mmol) dans 1,1 ml de toluène anhydre, on additionne successivement de l'oxyde de dibutylétain (15 mg, 0,06 mmol) et de l'azoture de triméthylsilyle (0,13 ml, 1,01 mmol). Le milieu réactionnel est chauffé 15 h à reflux (110°C) sous atmosphère d'argon et agitation magnétique. Après refroidissement on évapore le toluène, le produit brut est purifié par HPLC préparative sur une colonne Waters HR C₁₈ (25 x 100 mm) avec comme éluant MeOH : H₂O = 88 : 12 + 0,1% de TFA. Après évaporation, puis séchage à la pompe à palette, on obtient 0,08 g d'un solide blanc, le (Z) 5-[1-[2-(5,6,7,8-tétrahydro-3,5,5,8,8-pentaméthyl) naphtalénylméthylène]-2,3-dihydro-1*H*-indényl]-1*H*-tétrazole (CB02981) (Rdt = 40%) de formule :

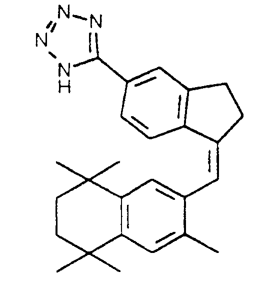

F (°C) = 235.

RMN¹H 200MHz (CDCl₃) : 1, 16 et 1,29 (2s, 12H, 5,5,8,8-Me); 1,65 (s, 4H, 6,7-CH₂) ; 2,21 (s, 3H, Me); 3,00 (m, 4H, -CH₂- indène); 6,64 (s large, 1H, H vinylique); 7,14 (s, 1H, ArH); 7,18 (d, 1H, ArH *J* 8,2Hz); 7,27 (s, 1H, ArH); 7,54 (d, 1H, ArH *J* 8, 1Hz) ; 7,93 (s, 1H, ArH).

MS EI 70 eV (m/z, % intensité) : 398 (M⁺, 72%); 371 (67); 370 (100), 355 (53); 205 (33); 157 (30); 83 (16) ; 69 (53); 60 (40); 57 (53); 55 (41).

MSHR EI 70 eV : Mₜᵣ = 398,2474 pour C₂₆H₃₀N₄ = 398,2470.

HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, tétrazole (CB02981) tr = 4,4 min 99,6%.

### e) (E) 1-[2-(5,6,7,8-Tétrahydro-3,5,5,8,8-pentaméthyl)naphtalénylméthylènel-2,3-dihydro-5-cyano-1H-indène.

A une solution de (E) 1-[2-(5,6,7,8-tétrahydro-3,5,5,8,8-pentaméthyl)naphtalénylméthylène]-2,3-dihydro-5-bromo-1*H*-indène (1,00 g, 2,44 mmol) dans 10 ml de DMF anhydre, on ajoute à température ambiante du cyanure de cuivre (0,33 g, 3,66 mmol). Le milieu réactionnel est porté au reflux pendant 16 h sous atmosphère d'argon. Après retour à température ambiante le milieu réactionnel est dilué avec de l'éther (50 ml) et filtré sur célite. On lave la phase organique avec une solution aqueuse saturée en NaHCO₃ (3 x 70 ml) puis sèche sur MgSO₄, filtre et évapore. On purifie le produit brut par chromatographie éclair sur silice (éluant éther : éther de pétrole 2 : 98). On obtient 0,54 g d'un solide jaunâtre, le (E) 1-[2-(5,6,7,8-tétrahydro-3,5,5,8,8-pentaméthyl)naphtalényl-méthylène]-2,3-dihydro-5-cyano-1*H*-indène (Rdt = 62 %) :

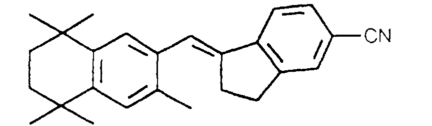

F (°C) = 158.

RMN¹H 200MHz (CDCl₃) : 1,28 et 1,29 (2s, 12H, 5,5,8,8-Me); 1,68 (s, 4H, 6,7-CH₂) ; 2,32 (s, 3H, Me); 3,05 (s, 4H, -CH₂- indène); 7,11 (s, 1H, ArH) ; 7,12 (s, 1H, H vinylique); 7,37 (s, 1H, ArH); 7,48 (dd, 1H, ArH *J* 1,3Hz *J* 8,0Hz); 7,53 (s, 1H, ArH); 7,63 (d, 1H, ArH *J* 8,0Hz).

MS EI 70 eV (m/z, % intensité) : 355 (M⁺, 87); 340 (M⁺-CH₃, 100); 157 (14); 154 (27); 142 (47).

### f) Acide (E) 1-[2-(5,6,7,8-tétrahydro-3,5,5,8,8-pentaméthyl)naphtalénylméthyléne]-2,3-dihydro-1H-indène-5-carboxylique (CB40341)

A une suspension du (E) 1-[2-(5,6,7,8-tétrahydro-3,5,5,8,8-pentaméthyl)naphtalénylméthylène)-2,3-dihydro-5-cyano-1*H*-indène (0,25 g, 0,70 mmol) dans une solution hydroéthanolique (H₂O 0,55 ml et EtOH 3,3 ml) est ajoutée de la potasse (0,39 g, 7,00 mmol). On chauffe à reflux sous agitation magnétique pendant 16 h. Après refroidissement, on acidifie par HCl 3N, extrait à l'éther (3 x 50 ml), sèche par MgSO₄, filtre et évapore. On obtient un solide que l'on lave dans un mélange pentane : éther = 90 :10. Après filtration puis séchage on obtient 0,17 g d'un solide brun, l'acide (E) 1-[2-(5,6,7,8-tétrahydro-3,5,5,8,8-pentaméthyl)naphtalényl méthylène}1-2,3-dihydro-2-1*H*-indène-5-carboxylique (CB40341) (Rdt = 65%) de formule:

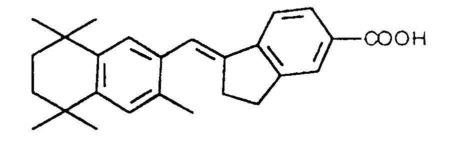

F (°C) = 206.

RMN¹H 200MHz (CDCl₃): 1,28 et 1,30 (2s, 12H, 5,5,8,8-Me); 1,69 (s, 4H, 6,7-CH₂) ; 2,34 (s, 3H, Me); 3,07 (s, 4H, -CH₂- indène); 7,13 (s, 1H, ArH); 7,14 (s, 1H, H vinylique); 7,41 (s, 1H, ArH); 7,65 (d, 1H, ArH *J* 8,1Hz) ; 7,93-8,10 (m, 3H, ArH).

MS EI 70 eV (m/z, % intensité) : 374 (M⁺, 90%) ; 359 (M⁺-CH₃, 100) ; 129 (140).

MSHR EI 70 eV : Mₜᵣ = 374,2237 pour C₂₆H₃₀O₂ Mₜₕ = 374,2246.

HPLC : Colonne Waters HR C₁₈, 8 x 100 mm, 6 µ, détecteur UV Waters 486 à 260 nm, débit 3 ml/min, éluant MeOH . H₂O = 90 : 10 + 0,1% de TFA, acide (CB40341) tr = 7,22 min 96,0%; impureté tr = 1,22 4,0%.

### g) (E) 5-[1-[2-(5,6,7,8-Tétrahydro-3,5,5,8,8-pentaméthyl)naphtalénylméthylène]-2,3-dihydro-1H-indényl]-1H-tétrazole (CB23804).

A une solution du (E) 1-[2-(5,6,7,8-tétrahydro-3,5,5,8,8-pentaméthyl)naphtalényl-méthylène]-2,3-dihydro-5-cyano-1*H*-indène (0,27 g, 0,76 mmol) dans 1,5 ml de toluène anhydre, on additionne successivement de l'oxyde de dibutylétain (19 mg) et de l'azoture de triméthylsilyle (0,20 ml, 1,52 mmol). Le milieu réactionnel est chauffé 16 h à reflux (110°C) sous atmosphère d'argon et agitation magnétique. Après refroidissement on évapore le toluène, le produit brut est purifié par chromatographie éclair sur silice (éluant CH₂Cl₂ puis CH₂Cl₂ : MeOH = 95 : 5). Après évaporation, le produit est lavé dans un minimum d'éther, filtré puis séché à la pompe à palette. On obtient 0,14 g d'un solide blanc, le (E) 5-[1-[2-(5,6,7,8-tétrahydro-3,5,5,8,8-pentaméthyl)naphtalénylméthylène)-2,3-dihydro-1*H*-indényl]-1*H*-tétrazole (CB23804) (Rdt = 46%) :

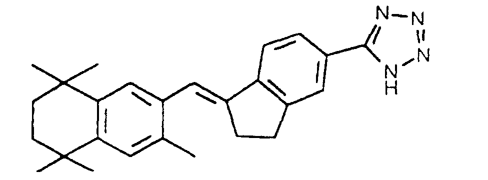

F (°C) = 269.

RMN¹H 200MHz (CDCl₃) : 1,20 et 1,22 (2s, 12H, 5,5,8,8-Me) ; 1,61 (s, 4H, 6,7-CH₂) ; 2,26 (s, 3H, Me) ; 3,03 (s, 4H, -CH₂- indène) ; 7,03 (s, 2H, ArH et H vinylique); 7,32 (s, 1H, ArH) ; 7,67 (d, 1H, ArH *J* 8,2Hz); 7,87 (d, 1H, ArH *J* 8,2Hz) ; 7,92 (s, 1H, ArH).

MS EI 70 eV (m/z, % intensité) : 398 (M⁺, 72%); 370 (100); 355 (73), 340 (30); 294 (41); 215 (33); 195 (82).

MSHR EI 70 eV : Mₜᵣ = 398,2476 pour C₂₆H₃₀N₄ = 398,2470.

HPLC : Colonne Waters HR C₁₈, 8 x 100 mm, 6 µ, détecteur UV Waters 486 à 280 nm, débit 3 ml/min, éluant MeOH : H₂O = 90 : 10 + 0,1% de TFA, tétrazole (CB23804) tr = 4,93 min 97,9%; impureté tr = 4,13 1,3%.

### Exemple 8 : Acide (E) 1-[2-(5,6,7,8-tétrahydro-3,5,5,8,8-pentaméthyl)naphtalénylméthyléne]-2,3-dihydro-1H-indényl-5-phosphonique (CB69179).

On porte à reflux pendant 24 h, sous atmosphère d'argon et agitation magnétique, une suspension de (E) 1-[2-(5,6,7,8-tétrahydro-3,5,5,8,8-pentaméthyl)naphtalényl-méthylène]-2,3-dihydro-5-bromo-1*H*-indène (0,69 g, 1,68 mmol), de diéthylphosphite (0,46 g, 3,37 mmol), de triéthylamine (0,17 g, 3,37 mmol), de tétrakis(triphénylphosphine)palladium (0,20 g, 0,17 mmol) dans 3 ml de THF anhydre. Après refroidissement du milieu réactionnel, on reprend à l'acétate d'éthyle (100 ml) et lave par une solution de HCl 1N puis par une solution saturée de NaCl. Après évaporation du solvant, le produit brut est purifié par chromatographie éclair sur silice (éluant éther : éther de pétrole = 10 : 90 puis 30 : 70). On obtient après évaporation 0,17 g d'un solide blanc (Rdt = 22%), le (E) 1-[2-(5,6,7,8-tétrahydro-3,5,5,8,8-pentaméthyl)naphtalénylméthylène]-2,3-dihydro-5-bromo-1*H*-indènyl-5-phosphonate de diéthyle. On additionne à la seringue du bromotriméthylsilane (0,31 ml, 2,31 mmol) sur une suspension du phosphonate de diéthyle (0,17 g, 0,36 mmol) dans 2,3 ml d'acétonitrile. On porte à reflux pendant 1,5 h, sous agitation magnétique et atmosphère d'argon, le milieu réactionnel. Après refroidissement, on évapore à sec et purifie le produit brut obtenu par HPLC préparative sur une colonne Waters HR C₁₈ (25 x 100 mm) avec comme éluant MeOH : H₂O = 85 : 15 + 0,1% de TFA. On obtient après évaporation des solvants puis séchage 0,08 g d'un solide blanc (Rdt = 54% à partir du phosphonate de diéthyle), l'acide (E) 1-[2-(5,6,7,8-tétrahydro-3,5,5,8,8-pentaméthyl)naphtalénylméthylène]-2,3-dihydro-1*H-*indényl-5-phosphonique (CB69179) de formule :

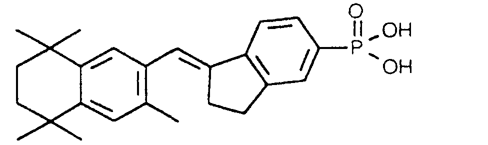

F (°C) = 218-219 (déc.).

RMN¹H 200MHz (CDCl₃): 1,14 et 1,23 (2s, 12H, 5,5,8,8-Me); 1,63 (s, 4H, 6,7-CH₂) ; 2,18 (s, 3H, Me); 3,35 (s, 2H, -CH₂- indène); 3,79 (s, 2H, -CH₂- indène); 6,06 (s, 1H, H vinylique); 7,05 (s, 2H, ArH); 7,41 (dd, 1H, ArH J 3,3Hz J 7,8Hz); 7,68 (dd, 1H, ArH *J* 3,3Hz *J* 13,2Hz) ; 7,84 (d, 1H, ArH J 13,2Hz).

MS FAB MMA (m/z, % intensité) : 411 (M⁺+1, 100%); 395 (37); 215 (40), 154 (84); 137 (55); 136 (62).

HPLC : Colonne Waters HR C₁₈, 8 x 100 mm, 6 µ, détecteur UV Waters 486 à 280 nm, débit 3 ml/min, éluant MeOH : H₂O = 85 : 15 + 0,1% de TFA, acide (CB69179) tr = 3,72 min 99,5%.

## Revendications

1. Composé tétracyclique aromatique de type rétinoïde de formule générale : 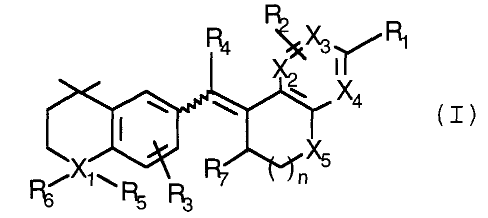 dans laquelle :
- R₁ est choisi parmi un atome d'hydrogène, un radical alkyle de 1 à 6 atomes de carbone linéaires ou ramifiés, un groupe de formule -PO₃H₂, -CH₂OH, -OH, -COR₈,-CH₂OCOR₉, -SH, -S-alkyl, -NH₂, NHCOOR₁₀, p-hydroxyphénylaminocarbonyl, tétrazol-5-yl-aminocarbonyl, tétrazol-5-yl, 5-trifluorométhyl-tétrazoyl et quand cela est possible leurs sels avec des acides tolérés physiologiquement, où R₁₀ est un groupe alkyle de 1 à 6 atomes de carbone linéaires ou ramifiés ou aralkyle, et R₈ et R₉ sont choisis parmi :
. un atome d'hydrogène, un groupe -OH, un radical alkyle de 1 à 6 atomes de carbone linéaires ou ramifiés ou un groupe de formule -OR₁₁, où R₁₁ représente un radical alkyle ramifié ou non ayant de 1 à 20 atomes de carbone, un radical alkényle ramifié ou non ayant de 2 à 20 atomes de carbone, un radical aryle ou aralkyle, ou
. un groupe aminé de formule : 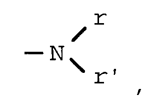 dans laquelle r et r', identiques ou différents, représentent un atome d'hydrogène, un radical alkyle de 1 à 6 atomes de carbone linéaires ou ramifiés, un radical aryle ou aralkyle, un reste d'α-aminoacide, un reste de sucre ou un hétérocycle dans lequel r et r' pris ensemble forment un hétérocycle.
- R₂ est choisi parmi un atome d'hydrogène, un atome d'halogène et plus particulièrement un atome de fluor, un radical alkyle de 1 à 6 atomes de carbone linéaires ou ramifiés, un groupe de formule -COOH, -OR₁₁, -SR₁₁,-(CF₂)ₙCF₃ où n est un nombre entier compris entre 0 et 10, ou un groupe -OCOR₁₂, et quand cela est possible leurs sels avec des acides tolérés physiologiquement, ou un groupe aminé de formule : 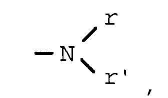 dans laquelle r et r' ont la même signification aue précédemment, et R₁₂ représente un atome d'hydrogène, un radical alkyle de 1 à 6 atomes de carbone linéaires ou ramifiés, un radical fluoroalkyle ayant de 1 à 6 atomes de carbone et de 3 à 7 atomes de fluor, un radical aryle ou un radical aralkyle.
- R₃ est choisi parmi un atome d'hydrogène, un radical alkyle de 1 à 6 atomes de carbone linéaires ou ramifiés, un atome d'halogène, un radical fluoroalkyle ayant de 1 à 6 atomes de carbone et de 3 à 7 atomes de fluor, ou un groupe de formule -OR₁₃ où R₁₃ représente un atome d'hydrogène, un radical alkyle de 1 à 6 atomes de carbone linéaires ou ramifiés, un radical aryle ou un radical aralkyle ou un groupement trifluorométhyle.
- X₁ est choisi parmi un atome de carbone, un atome d'oxygène ou un atome de soufre, et alors,
- R₅ et R₆ sont :
. des radicaux méthyles ou éthyles, dans le cas où X₁ est un atome de carbone,
. rien dans le cas où X₁ est un atome de soufre ou un atome d'oxygène,
. un ou deux atomes d'oxygène dans le cas où X₁ est un atome de soufre (cas d'un sulfoxyde -SO- ou d'une sulfone -SO₂-).
- R₄ est choisi parmi un atome d'hydrogène, un atome d'halogène et plus particulièrement un atome de fluor, un radical trifluorométhyle, un radical aryle, un radical aralkyle, ou un radical alkyle de 1 à 6 atomes de carbone linéaires ou ramifiés éventuellement substitué par un hydroxyle, un ou plusieurs atomes de fluor, un alcoxy de 1 à 6 atomes de carbone linéaires ou ramifiés ou par un groupe de formule -(C=O)R₁₄ dans laquelle R₁₄ représente un atome d'hydrogène, un radical de 1 à 6 atomes de carbone linéaires ou ramifiés, un radical hydroxyle, un radical alcoxy de 1 à 6 atomes de carbone linéaires ou ramifiés ou un groupe aminé de formule: 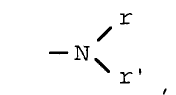 dans laquelle r et r' ont la même signification que précédemment.
- X₂ et X₃, identiques ou différents, représentent un atome de carbone ou un atome d'azote, ou encore X₂-X₃ sont un seul atome de soufre, d'oxygène, ou d'azote. Ainsi le noyau porteur de X₂ et X₃ peut être benzénique, pyridinique, thiophénique, furanique, pyrrolique.
- R7 est choisi parmi un atome d'hydrogène, un radical trifluorométhyle, un radical alkyle de 1 à 6 atomes de carbone linéaires ou ramifiés éventuellement substitué par un ou plusieurs atomes de fluor, un groupe -OR₁₅ où R₁₅ représente un atome d'hydrogène ou un radical alkyle de 1 à 6 atomes de carbone linéaires ou ramifiés
- X4 représente un atome de carbone ou d'azote.
- X₅ est choisi parmi un atome de carbone, d'oxygène, de soufre ou d'azote, un sulfure de formule -S-, un sulfoxyde de formule -SO-, une sulfone de formule -SO₂-, une amine de formule -NR₁₆- où R₁₆ représente un atome d'hydrogène ou un radical alkyle de 1 à 6 atomes de carbone linéaires ou ramifiés, un groupe de formule -COR₁₇- ou
- CO₂R₁₇- où R₁₇ est un radical alkyle de 1 à 6 atomes de carbone linéaires ou ramifiés ou benzyle.
- n est 0 ou 1.

2. Composé selon la revendication, **caractérisé en ce que** dans la formule (I) R₂ représente un atome d'hydrogène et R₁ est choisi parmi un groupe tétrazoyle, un groupe -COOH, un groupe -PO₃H₂, un groupe -CONH₂.

3. Les composés suivants :
L'acide (E) 3-[2-(5,6,7,8-tétrahydro-5,5,8,8-tétraméthyl)naphtalényllméthylène]-dihydro-benzo[b]furane-5-carboxylique,
L'acide (Z) 1-[2-(5,6,7,8-tétrahydro-5,5,8,8-tétraméthyl)naphtalénylméthylène]-1,2,3,4-tétrahydro-naphtalène-6-carboxylique,
L'acide (E) 1-[2-(5,6,7,8-tétrahydro-5,5,8,8-tétraméthyl)naphtalénylméthylène]-1,2,3,4-tétrahydronaphtalène-6-carboxylique,
Le (E) 5-[1-[2-(5,6,7,8-tétrahydro-5,5,8,8-tétraméthyl)-2-naphtalénylméthylène]-1,2,3,4-tétrahydronaphtalènyl] -1*H*-tétrazole,
L'acide (Z) 1-[2-(5,6,7,8-tétrahydro-3,5,5,8,8-pentaméthyl)naphtalénylméthylène]-1,2,3,4-tétrahydronaphtalène-6-carboxylique,
L'acide (E) 1-[2-(5,6,7,8-tétrahydro-3,5,5,8,8-pentaméthyl)naphtalénylméthylène]-1,2,3,4-tétrahydronaphtalène-6-carboxylique,
Le (Z) 5-[1-[2-(5,6,7,8-tétrahydro-3,5,5,8,8-pentamethyl)naphtalénylméthylène]-1,2,3,4-tétrahydronaphtalènyl]-1*H*-tétrazole,
Le (E) 5-[1-[2-(5,6,7,8-tétrahydro-3,5,5,8,8-pentaméthyl)naphtalénylméthylène]-1,2,3,4-tétrahydronaphtalènyl]-*1H*-tétrazole,
L'acide (E) 1-[2-(5,6,7,8-tétrahydro-5,5,8,8-tétraméthyl)naphtalénylméthylène]-2,3-dihydro-1*H*-indène-5-carboxylique,
Le (E) 5-[1-[2-(5,6,7,8-tétrahydro-5,5,8,8-tétraméthyl)naphtalénylméthylène]-2,3-dihydro-1*H*-indényl]-1*H*-tétrazole,
L'acide (Z) 1-[2-(5,6,7,8-tétrahydro-5,5,8,8-tétraméthyl)naphtalénylméthylène)-2,3-dihydro-1*H*-indène-5-carboxylique,
Le (Z) 5-[1-[2-(5,6,7,8-tétrahydro-5,5,8,8-tétraméthyl)naphtalénylméthylène]-2,3-dihydro-1*H*-indényl)-1*H*-tétrazole,
L'acide (Z) 1-[2-(5,6,7,8-tétrahydro-3,5,5,8,8-pentaméthyl)naphtalénylméthylène]-2,3-dihydro-1*H*-indéne-5-carboxylique,
Le (Z) 5-[1-[2-(5,6,7,8-tétrahydro-3,5,5,8,8-pentaméthyl)naphtalénylméthylène)-2,3-dihydro-1*H*-indényl]-1*H*-tétrazole,
L'acide (E) 1-[2-(5,6,7,8-tétrahydro-3,5,5,8,8-pentaméthyl)naphtalénylméthylène]-2,3-dihydro-1*H*-indène-5-carboxylique,
Le (E) 5-[1-[2-(5,6,7,8-tétrahydro-3,5,5,8,8-pentaméthyl)naphtalénylméthylène]-2,3-dihydro-1*H*-indényl]-1*H*-tétrazole,
L'acide (E) 1-[2-(5,6,7,8-tétrahydro-3,5,5,8,8-pentaméthyl)naphtalénylméthylène]-2,3-dihydro-1*H*-indényl-5-phosphonique,
l'acide 1-[(E)-2-(5,6,7,8-tétrahydro-5,5,8,8-tétraméthyl)naphtalènylméthylène]-2,3-dihydro-2-méthyl-1*H*-indène-5-carboxylique,
Le (Z) 1-[2-(5,6,7,8-tétrahydro-5,5,8,8-tétraméthyl)naphtalénylméthylène]-2,3-dihydro-2-méthyl-1*H*-indène-5-amide,
L'acide (Z) 1-[2-(5,6,7,8-tétrahydro-5,5,8,8-tétraméthyl)naphtalénylméthylène)-2,3-dihydro-2-méthyl-1*H*-indène-5-carboxylique,
Le (E) 5-[1-[2-(5,6,7,8-tétrahydro-5,5,8,8-tétraméthyl)naphtalénylméthylène]-2,3-dihydro-2-méthyl-1*H*-indényl]-1*H*-tétrazole.

4. Composition thérapeutique, dermatologique ou cosmétique renfermant au moins un dérivé de formule I défini dans l'une quelconque des revendications 1 à 3, sous forme libre ou sous forme d'un sel ou d'un ester pharmaceutiquement acceptable, en association avec un véhicule ou diluant traditionnel.

## Patentansprüche

1. Tetrazyklische aromatische Verbindung des Retinoid-Typs mit der allgemeinen Formel: 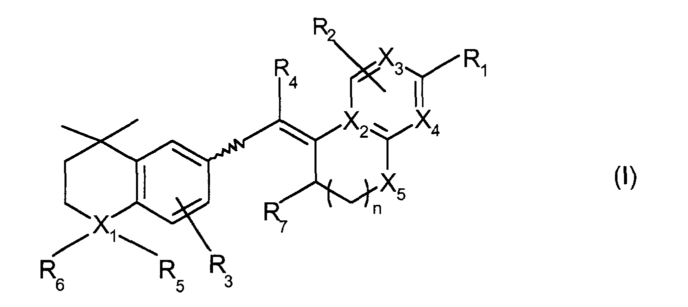 in der:
- R₁ gewählt wird unter einem Wasserstoffatom, einem Alkylrest mit 1 bis 6 Kohlenstoffatomen in linearer oder verzweigter Anordnung, einer Gruppe der Formel -PO₃H₂, -CH₂OH, OH, -COR₈, -CH₂OCOR₉, -SH, -S-Alkyl, -NH₂, -NHCOOR₁₀, p-Hydroxyphenylaminocarbonyl, Tetrazol-5-yl-aminocarbonyl, Tetrazol-5-yl, 5-Trifluoromethyl-tetrazoyl, und, soweit möglich, deren Salze mit Säuren, die physiologisch verträglich sind, wobei R₁₀ ein Alkylrest mit 1 bis 6 Kohlenstoffatomen in linearer oder verzweigter Anordnung oder ein Aralkylrest ist, und R₈ und R₉ gewählt werden unter:
. einem Wasserstoffatom, einer OH-Gruppe, einem Alkylrest mit 1 bis 6 Kohlenstoffatomen in linearer oder verzweigter Anordnung oder einer Gruppe der Formel -OR₁₁, dabei stellt R₁₁ einen verzweigten oder unverzweigten Alkylrest mit 1 bis 20 Kohlenstoffatomen, einen verzweigten oder unverzweigten Alkenrest mit 2 bis 20 Kohlenstoffatomen, einen Aryl- oder Aralkylrest oder eine Aminogruppe der Formel : 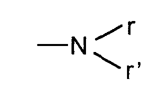 bei der r und r' gleich oder verschieden sein können und ein Wasserstoffatom, einen Alkylrest mit 1 bis 6 Kohlenstoffatomen in linearer oder verzweigter Anordnung, einen Aryl- oder Aralkylrest, einen α-Aminosäurerest, einen Zuckerrest oder einen Heterozyklus bei dem r und r' gemeinsam den Heterozyklus bilden bedeuten können dar.
- R₂ gewählt wird unter einem Wasserstoffatom, einem Halogenatom, insbesondere einem Fluoratom, einem Alkylrest mit 1 bis 6 Kohlenstoffatomen in linearer oder verzweigter Anordnung, einer Gruppe der Formel -COOH, -OR₁₁, -SR₁₁, -(CF₂)ₙCF₃ wobei n eine ganze Zahl zwischen 0 und 10 ist, oder einer Gruppe -OCOR₁₂, und, soweit möglich, deren Salze mit Säuren die physiologisch verträglich sind, oder einer Aminogruppe der Formel: 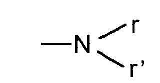 bei der r und r' die gleiche Bedeutung wie oben haben, und R₁₂ ein Wasserstoffatom, einen Alkylrest mit 1 bis 6 Kohlenstoffatomen in linearer oder verzweigter Anordnung, einen Fluoralkylrest mit 1 bis 6 Kohlenstoffatomen und 3 bis 7 Fluoratomen, einen Aryl- oder Aralkylrest darstellt.
- R₃ gewählt wird unter einem Wasserstoffatom, einem Alkylrest mit 1 bis 6 Kohlenstoffatomen in linearer oder verzweigter Anordnung, einem Fluoralkylrest mit 1 bis 6 Kohlenstoffatomen und 3 bis 7 Fluoratomen, oder einer Gruppe der Formel -OR₁₃, wobei R₁₃ ein Wasserstoffatom, einen Alkylrest mit 1 bis 6 Kohlenstoffatomen in linearer oder verzweigter Anordnung, einen Aryl- oder Aralkylrest oder eine Trifluoromethylgruppe darstellt.
- X₁ wird gewählt unter einem Kohlenstoffatom, einem Sauerstoffatom oder einem Schwefelatom und
- R₅ und R₆ sind:
. Methyl- oder Ethylreste, wenn X₁ ein Kohlenstoffatom ist,
. gar nichts, wenn X₁ ein Schwefel- oder Sauerstoffatom ist,
. Ein oder zwei Sauerstoffatome im Fall daß X₁ ein Schwefelatom ist (Fall einer Sulfoxid -SO- oder Sulfon-SO₂-Gruppe)
- R₄ wird gewählt unter einem Wasserstoffatom, einem Halogenatom, insbesondere einem Fluoratom, einem Trifluoromethylrest, einem Arylrest, einem Aralkylrest, oder einem Alkylrest mit 1 bis 6 Kohlenstoffatomen in linearer oder verzweigter Anordnung, der eventuell substituiert ist mit einer Hydroxylgruppe, einem oder mehreren Fluoratomen, einer Alkoxygruppe mit 1 bis 6 Kohlenstoffatomen in linearer oder verzweigter Anordnung oder mit einer Gruppe der Formel -(C=O)R₁₄ in der R₁₄ ein Wasserstoffatom, einen Alkylrest mit 1 bis 6 Kohlenstoffatomen in linearer oder verzweigter Anordnung, eine Hydroxylgruppe, eine Alkoxygruppe mit 1 bis 6 Kohlenstoffatomen in linearer oder verzweigter Anordnung, oder eine Aminogruppe der Formel: 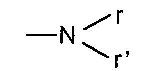 bei der r und r' die gleiche Bedeutung wie oben haben darstellt.
- X₂ und X₃ können gleich oder verschieden sein und stellen ein Kohlenstoffatom oder ein Stickstoffatom dar, oder aber X₂ und X₃ zusammen sind ein einziges Schwefel-, Sauerstoff- oder Stickstoffatom. Der Ring, der X₂ und X₃ enthält kann also ein Benzolring, ein Pyridinring, ein Thiophenring, ein Furanring oder ein Pyrrolring sein.
- R₇ ist gewählt unter einem Wasserstoffatom, einem Trifluoromethylrest, einem Alkylrest mit 1 bis 6 Kohlenstoffatomen in linearer oder verzweigter Anordnung, der eventuell substituiert ist mit einem oder mehreren Fluoratomen, und einer Gruppe -OR₁₅, wobei R₁₅ ein Wasserstoffatom oder einen Alkylrest mit 1 bis 6 Kohlenstoffatomen in linearer oder verzweigter Anordnung darstellt
- X₄ stellt ein Kohlenstoff- oder Stickstoffatom dar
- X₅ ist gewählt unter einem Kohlenstoffatom, einem Sauerstoffatom, einem Schwefelatom oder einem Stickstoffatom, einem Sulfidrest -S-, einem Sulfoxidrest -SO-, einem Sulfonrest -SO₂-, einem Amin der Formel -NR₁₆-wobei R₁₆ ein Wasserstoffatom oder einen Alkylrest mit 1 bis 6 Kohlenstoffatomen in linearer oder verzweigter Anordnung darstellt, einer Gruppe der Formel -COR₁₇- oder -CO₂R₁₇- wobei R₁₇ ein Alkylrest mit 1 bis 6 Kohlenstoffatomen in linearer oder verzweigter Anordnung oder ein Benzylrest ist.
- n ist 0 oder 1.

2. Verbindung nach Anspruch 1, **dadurch gekennzeichnet**, daß in der Formel (1) R₂ ein Wasserstoffatom darstellt und R₁ gewählt wird unter einer Tetrazolylgruppe, einer -COOH-Gruppe, einer -PO₃H₂-Gruppe, einer -CONH₂-Gruppe.

3. Die folgenden Verbindungen:
(E) 3-[2-(5,6,7,8-Tetrahydro-5,5,8,8-tetramethyl) naphtalenylmethylen]-dihydro-benzo[b]furan-5-carbonsäure,
(Z) 1-[2-(5,6,7,8-Tetrahydro-5,5,8,8-tetramethyl) naphtalenylmethylen]-1,2,3,4-tetrahydronaphtalen-6-carbonsäure,
(E) 1-[2-(5,6,7,8-Tetrahydro-5,5,8,8-tetramethyl) naphtalenylmethylen]-1,2,3,4-tetrahydronaphtalen-6-carbonsäure,
(E) 5[1-[2-(5,6,7,8-Tetrahydro-5,5,8,8-tetramethyl)-2-naphtalenmethylen]-1,2,3,4-tetrahydronaphtalenyl]-1H-tetrazol,
(Z) 1-[2-(5,6,7,8-Tetrahydro-3,5,5,8,8-pentamethyl) naphtalenylmethylen]-1,2,3,4-tetrahydronaphtalen-6-carbonsäure,
(E) 1-[2-(5,6,7,8-Tetrahydro-3,5,5,8,8-pentamethyl) naphtalenylmethylen]-1,2,3,4-tetrahydronaphtalen-6-carbonsäure,
(Z) 5-[1-[2-(5,6,7,8-Tetrahydro-3,5,5,8,8-pentamethyl)naphtalenylmethylen]-1,2,3,4-tetrahydro-naphtalenyl]-1H-tetrazol,
(E) 5-[1-[2-(5,6,7,8-Tetrahydro-3,5,5,8,8-pentamethyl)naphtalenylmethylen]-1,2,3,4-tetrahydro-naphtalenyl]-1H-tetrazol,
(E) 1-[2-(5,6,7,8-Tetrahydro-5,5,8,8-tetramethyl) naphtalenylmethylen]-2,3-dihydro-1H-inden-5-carbonsäure,
(E) 5-[1-[2-(5,6,7,8-Tetrahydro-5,5,8,8-tetramethyl) naphtalenylmethylen]-2,3-dihydro-1H-indenyl]-1H-tetrazol,
(Z) 1-[2-(5,6,7,8-Tetrahydro-5,5,8,8-tetramethyl) naphtalenylmethylen]-2,3-dihydro-1H-inden-5-carbonsäure,
(Z) 5-[1-[2-(5,6,7,8-Tetrahydro-5,5,8,8-tetramethyl) naphtalenylmethylen]-2,3-dihydro-1H-indenyl]-1H-tetrazol,
(Z) 1-[2-(5,6,7,8-Tetrahydro-3,5,5,8,8-pentamethyl) naphtalenylmethylen]-2,3-dihydro-1H-inden-5-carbonsäure,
(Z) 5-[1-[2-(5,6,7,8-Tetrahydro-3,5,5,8,8-pentamethyl) naphtalenylmethylen]-2,3-dihydro-1H-indenyl]-1H-tetrazol,
(E) 1-[2-(5,6,7,8-Tetrahydro-3,5,5,8,8-pentamethyl) naphtalenylmethylen]-2,3-dihydro-1H-inden-5-carbonsäure,
(E) 5-[1-[2-(5,6,7,8-Tetrahydro-3,5,5,8,8-pentamethyl) naphtalenylmethylen]2,3-dihydro-1H-indenyl]-1H-tetrazol,
(E) 1-[2-(5,6,7,8-Tetrahydro-3,5,5,8,8-pentamethyl) naphtalenylmethylen]-2,3-dihydro-1H-indenyl-5-phosphonsäure,
1-[(E)-2-(5,6,7,8-Tetrahydro-5,5,8,8-tetramethyl) naphtalenylmethylen]-2,3-dihydro-2-methyl-1H-inden-5-carbonsäure,
(Z)-1-[2-(5,6,7,8-Tetrahydro-5,5,8,8-tetramethyl) naphtalenylmethylen]-2,3-dihydro-2-methyl-1H-inden-5-amid,
(Z)-1-[2-(5,6,7,8-Tetrahydro-5,5,8,8-tetramethyl) naphtalenylmethylen]-2,3-dihydro-2-methyl-1H-inden-5-carbonsäure,
(E) 5-[1-[2-(5,6,7,8-Tetrahydro-5,5,8,8-tetramethyl) naphtalenylmethylen]-2,3-dihydro-2-methyl-1H-indenyl]-1H-tetrazol.

4. Pharmazeutische, dermatologische oder kosmetische Zubereitung die mindestens ein Derivat der Formel (I), das in irgendeinem der Ansprüche 1 bis 3 definiert wird, frei oder in Form eines pharmazeutisch akzeptablen Salzes oder Esters enthält, in Verbindung mit einem traditionellen Trägermaterial oder Lösungsmittel.

## Claims

1. Aromatic tetracyclic compound of the retinol type of general formula: 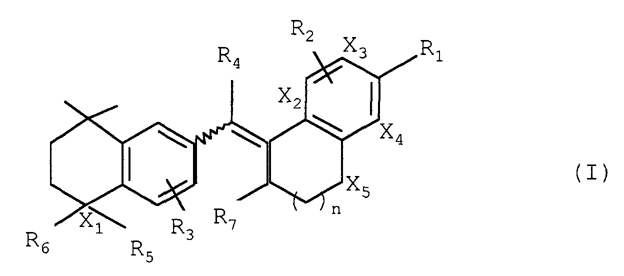 in which:
- R₁ is chosen from amongst a hydrogen atom, an alkyl radical of 1 to 6 linear or branched carbon atoms, a group of formula -PO₃H₂, -CH₂OH, -OH, -COR₈, -CH₂OCOR₉,-SH, -S-alkyl, -NH₂, NHCOOR₁₀, p-hydroxyphenylamino-carbonyl, tetrazol-5-yl-aminocarbonyl, tetrazol-5-yl, 5-trifluormethyl-tetrazoyl and when possible their salts with physiologically tolerated acids, where R₁₀ is an alkyl group of 1 to 6 linear or branched carbon atoms or aralkyl, and R₈ and R₉ are chosen among:
. a hydrogen atom, an -OH group, an alkyl radical of 1 to 6 linear or branched carbon atoms or a group of formula -OR₁₁, where OR₁₁ represents an alkyl radical, branched or not, having 1 to 20 carbon atoms, an alkenyl radical, branched or not, having 2 to 20 carbon atoms, an aryl or aralkyl radical, or
. an amine group of formula: 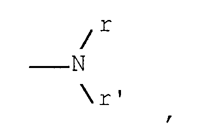 in which r and r', identical or different, represent a hydrogen atom, an alkyl radical of 1 to 6 linear or branched carbon atoms, an aryl or aralkyl radical, a residual of α-aminoacid, a residual of sugar or a heterocycle in which r and r' taken together form a heterocycle.
- R₂ is chosen from among a hydrogen atom, a halogen atom and more particularly a fluorine atom, an alkyl radical of 1 to 6 linear or branched carbon atoms, a group of formula -COOH, -OR₁₁, -SR₁₁, - (CF₂)ₙCF₃ where n is a whole number comprised between 0 and 10, or a group -OCOR₁₂, and when possible their salts with physiologically tolerated acids, or an amine group of formula: 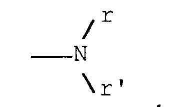 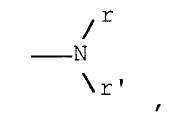 in which r and r' have the same meaning as above, and R₁₂ represents a hydrogen atom, an alkyl radical of 1 to 6 linear or branched carbon atoms, a fluoroalkyl radical having 1 to 6 linear or branched carbon atoms and from 3 to 7 fluorine atoms, an aryl radical or an aralkyl radical.
- R₃ is chosen from among a hydrogen atom, an alkyl radical of 1 to 6 linear or branched carbon atoms, a halogen atom, a fluoroalkyl radical having 1 to 6 carbon atoms and 3 to 7 fluorine atoms, or a group of formula -OR₁₃ where R₁₃ represents a hydrogen atom, an alkyl radical of 1 to 6 linear or branched carbon atoms, an aryl radical or an aralkyl radical or a trifluoromethyl grouping.
- X₁ is chosen from amongst a carbon atom, an oxygen atom or a sulphur atom, and where,
- R₅ and R₆ are:
. methyl or ethyl radicals, in the case where X₁ is an atom of carbon,
. nothing in the case where X₁ is an atom of sulphur or an atom of oxygen,
. one or two oxygen atoms in the case where X₁ is an atom of sulphur (the case of a sulphoxide -SO- or a sulphone -SO₂).
- R₄ is chosen from among an atom of hydrogen, an atom of halogen and more particularly an atom of fluorine, a trifluoromethyl radical, an aryl radical, an aralkyl radical, or an alkyl radical of 1 to 6 linear or branched carbon atoms possibly substituted by a hydroxyl, one or several atoms of fluorine, an alkoxy of 1 to 6 linear or branched carbon atoms or by a group of formula -(C=O)R₁₄ in which R₁₄ represents an atom of hydrogen, a radical of 1 to 6 linear or branched carbon atoms, a hydroxyl radical, an alkoxy radical of 1 to 6 linear or branched carbon atoms or an amine group of formula: 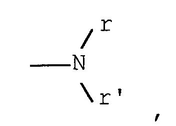 in which r and r' have the same meaning as above.
- X₂ and X₃, identical or different, represent an atom of carbon or an atom of nitrogen, or yet again X₂-X₃ are a single atom of sulphur, oxygen or nitrogen. Thus the carrier nucleus of X₂ and X₃ can be benzenic, pyridinic, thiophenic, furanic, pyrrolic.
- R₇ is chosen from among an atom of hydrogen, a trifluoromethyl radical, an alkyl radical of 1 to 6 linear or branched carbon atoms, possibly substituted by one or several fluorine atoms, a group -OR₁₅ where R₁₅ represents an atom of hydrogen or an alkyl radical of 1 to 6 linear or branched carbon atoms.
- X₄ represents an atom of carbon or nitrogen.
- X₅ is chosen from among an atom of carbon, oxygen, sulphur or nitrogen, a sulphide of formula -S-, a sulfoxide of formula -SO-, a sulphone of formula -SO₂-, an amine of formula -NR₁₆- where R₁₆ represents an atom of hydrogen or an alkyl radical of 1 to 6 linear or branched carbon atoms, a group of formula -COR₁₇- or -CO₂R₁₇- where R₁₇ is an alkyl radical of 1 to 6 linear or branched carbon atoms or benzyl.
- n is 0 or 1.

2. Compound according to the claim, **characterised in that** in the formula (I), R₂ represents an atom of hydrogen and R₁ is chosen among a tetrazoyl group, a -COOH group, a -PO₃H₂ group, a -CONH₂ group.

3. The following compounds:
the (E) 3-[2-(5,6,7,8-tetrahydro-5,5,8,8-tetramethyl) napthtalenylmethylene]-dihydro-benzo [b] furane-5-carboxylic acid,
the (Z) 1-[2-(5,6,7,8-tetrahydro-5,5,8,8-tetramethyl) naphtalenylmethylene]-1,2,3,4-tetrahydronaphtalene-6-carboxylic acid,
the (E) 1-[2-(5,6,7,8-tetrahydro-5,5,8,8-tetramethyl) naphtalenylmethylene]-1,2,3,4-tetrahydro-naphtalene-6-carboxylic acid,
the (E) 5-{1-[2-(5,6,7,8-tetrahydro-5,5,8,8-tetramethyl)-2-naphtalenylmethylene]-1,2,3,4-tetrahydronaphtalenyl}-1 *H*-tetrazol,
the (Z) 1-[2-(5,6,7,8 tetrahydro-3,5,5,8,8-pentamethyl) naphtalenylmethylene]-1,2,3,4-tetrahydronaphtalene-6-carboxylic acid,
the (E) 1-[2-(5,6,7,8-tetrahydro-3,5,5,8,8-pentamethyl) naphtalenylmethylene]-1,2,3,4-tetrahydronaphtalene-6-carboxylic acid,
the (Z) 5-{1-[2-(5,6,7,8-tetrahydro-3,5,5,8,8-pentamethyl)naphtalenylmethylene]-1,2,3,4-tetrahydronaphtalenyl}-1*H*-tetrazol,
the (E) 5-{1-[2-(5,6,7,8-tetrahydro-3,5,5,8,8-pentamethyl)naphtalenylmethylene]-1,2,3,4-tetrahydronaphtalenyl}-1*H*-tetrazol,
the (E) 1-[2-(5,6,7,8-tetrahydro-5,5,8,8-tetramethyl) naphtalenylmethylene]-2,3-dihydro-1*H*-indene-5-carboxylic acid,
The (E) 5-{1-[2-(5,6,7,8-tetrahydro-5,5,8,8-tetramethyl) naphtalenylmethylene]-2,3-dihydro-1*H*-indenyl}-1*H*-tetrazol,
the (Z) 1-[2-(5,6,7,8-tetrahydro-5,5,8,8-tetramethyl) naphtalenylmethylene]-2,3-dihydro-1*H*-indene-5-carboxylic acid,
the (Z) 5-{1-[2-(5,6,7,8-tetrahydro-5,5,8,8-tetramethyl) naphtalenylmethylene]-2,3-dihydro-1*H*-indenyl}-1*H*-tetrazol,
the (Z) 1-[2-(5,6,7,8-tetrahydro-3,5,5,8,8-pentamethyl) naphtalenylmethylene]-2,3-dihydro-1*H*-indene-5-carboxylic acid,
The (Z) 5-{1-[2-(5,6,7,8-tetrahydro-3,5,5,8,8-pentamethyl)naphtalenylmethylene]-2,3-dihydro-1*H*-indenyl}-*1H*-tetrazol,
the (E) 1-[2-(5,6,7,8-tetrahydro-3,5,5,8,8-pentamethyl) naphtalenylmethylene]-2,3-dihydro-1*H*-indene-5-carboxylic acid,
the (E) 5-{1-[2-(5,6,7,8-tetrahydro-3,5,5,8,8-pentamethyl) naphtalenylmethylene]-2,3-dihydro-1*H*-indenyl}-1*H*-tetrazol,
the (E) 1-[2-(5,6,7,8-tetrahydro-3,5,5,8,8-pentamethyl) naphtalenylmethylene]-2,3-dihydro-1*H*-indenyl-5-phosphonic acid,
the (E) -1-[2-(5,6,7,8-tetrahydro-5,5,8,8-tetramethyl) naphtalenylmethylene]-2,3-dihydro-2-methyl-1*H*-indene-5-carboxylic acid,
the (Z) 1-[2-(5,6,7,8-tetrahydro-5,5,8,8-tetramethyl) naphtalenylmethylene]-2,3-dihydro-2-methyl-IH-indene-5-amide,
the (Z) 1-[2-(5,6,7,8-tetrahydro-5,5,8,8-tetramethyl) naphtalenylmethylene]-2,3-dihydro-2-methyl 1*H*-indene-5-carboxylic acid,
the (E) 5-{1-[2-(5,6,7,9-tetrahydro-5,5,8,8-tetramethyl) naphtalenylmethylene]-2,3-dihydro-2-methyl-1*H*-indenyl}-1*H*-tetrazol.

4. Therapeutic, dermatologic or cosmetic composition, comprising at least one derivative of formula I defined in any one of claims 1 to 3, in free form or under the form of a salt or an ester pharmaceutically acceptable, in association with a traditional carrier or diluent.
